# EUROPEAN PATENT APPLICATION

(11) **EP 4 043 443 A1**
(43) Date of publication of application: **17.08.2022**
(21) Application number: 20874145.4
(22) Date of filing: 30.09.2020
(51) Int. Cl.: C07D 257/06, C07D 405/12, C07D 285/08, C07D 413/12, C07D 401/12, A01N 43/713, A01N 43/836, A01N 43/80, A01P 13/00

(54) **ALKENE-CONTAINING AMIDE COMPOUND AND USE THEREOF**

(30) Priority: 08.10.2019 CN 201910950934; 13.03.2020 CN 202010174419
(71) Applicant: Shenyang Sinochem Agrochemicals R & D Co., Ltd., Shenyang, Liaoning 110021 (CN); Jiangsu Yangnong Chemical Co., Ltd., Yangzhou, Jiangsu 225009 (CN)
(72) Inventor: SUN, Bing, Shenyang, Liaoning 110021 (CN); YANG, Huibin, Shenyang, Liaoning 110021 (CN); MA, Hongjuan, Shenyang, Liaoning 110021 (CN); YING, Junwu, Shenyang, Liaoning 110021 (CN); CUI, Dongliang, Shenyang, Liaoning 110021 (CN); QIN, Bo, Shenyang, Liaoning 110021 (CN); LIANG, Shuang, Shenyang, Liaoning 110021 (CN); WANG, Gang, Shenyang, Liaoning 110021 (CN); LU, Zhengmao, Shenyang, Liaoning 110021 (CN); ZHANG, Fan, Shenyang, Liaoning 110021 (CN); CHEN, Lin, Shenyang, Liaoning 110021 (CN); PEI, Heying, Shenyang, Liaoning 110021 (CN); CHENG, Yan, Shenyang, Liaoning 110021 (CN); WANG, Mingxin, Shenyang, Liaoning 110021 (CN); LI, Bin, Shenyang, Liaoning 110021 (CN)
(74) Representative: Nederlandsch Octrooibureau
(86) International application number: PCT/CN2020/119134
(87) International publication number: WO 2021/068816

(57) **Abstract**

The present invention discloses an alkene-containing amide compound and an application of agriculturally acceptable salt as a herbicide. The compound is shown in formula (I):

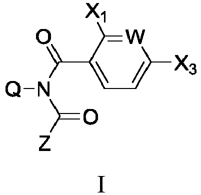

The definition of each substituent in the formula I is described in the description. The compound of the formula I of the present invention has excellent herbicidal activity and can be used for controlling agricultural weeds.

## Description

### Technical Field

The present invention belongs to the field of herbicides, and particularly relates to an alkene-containing amide compound and an application thereof as a herbicide.

### Background

Due to the succession and change of weed populations and the emergence and rapid development of resistance to chemical pesticides, people have continuously strengthened awareness on ecological environmental protection, and have paid more attention to the knowledge of chemical pesticide pollution and the influence of pesticides on non-target organisms and the end-result problem in the pesticide ecological environment. With the gradual decrease of the arable land area in the world, the continuous increase of the population and the increase of the demands for food, people are forced to rapidly develop agricultural production technologies, enhance and improve the farming system, and continuously invent novel and improved herbicidal compounds and compositions.

CN108290846A has reported that some benzamide compounds have herbicidal activity, such as compounds 13-30 (KC):

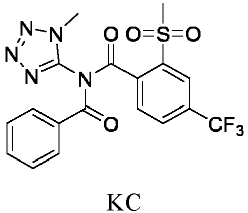

The alkene-containing amide compound shown in the present invention is not disclosed.

### Summary

The purpose of the present invention is to provide an alkene-containing amide compound with novel structure and safety for crops and an application thereof as a herbicide.

To achieve the above purpose, the present invention adopts the following technical solution:

An alkene-containing amide compound is shown in formula I:

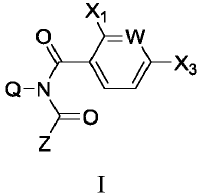

In the formula:
X₁ and X₃ are independently selected from hydrogen, cyano, nitro, halogen, C₁-C₆ alkylsulfonyl, C₁-C₆ haloalkylsulfonyl, C₁-C₆ alkylsulfinyl, C₁-C₆ haloalkylsulfinyl, C₁-C₆ alkylthio, C₁-C₆ haloalkylthio, C₁-C₆ alkyl, C₁-C₆ haloalkyl, C₁-C₆ alkoxy, C₁-C₆ haloalkoxy, C₁-C₆ alkoxy C₁-C₃ alkyl, C₁-C₆ alkoxy C₁-C₃ alkoxy, C₁-C₆ alkoxy C₁-C₃ alkoxy C₁-C₃ alkyl, C₃-C₆ cycloalkyl, C₃-C₆ cycloalkyl C₁-C₃ alkyl, C₃-C₆ cycloalkyl C₁-C₃ alkoxy, C₃-C₆ cycloalkyloxy, C₂-C₆ alkenyl, C₂-C₆ alkynyl, phenyl, C₂-C₆ alkenylsulfonyl, C₂-C₆ alkynylsulfonyl, phenylsulfonyl, C₂-C₆ alkenyloxy, C₂-C₆ alkynyloxy, phenyloxy, C₂-C₆ alkenylthio, C₂-C₆ alkynylthio, phenylthio, 5-7 membered aliphatic heterocycle containing 1-4 heteroatoms, 5-7 membered aromatic heterocycle containing 1-4 heteroatoms, 5-7 membered aliphatic heterocyclic C₁-C₆ alkyl containing 1-4 heteroatoms or 5-7 membered aromatic heterocyclic C₁-C₆ alkyl containing 1-4 heteroatoms; hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy or C₃-C₆ cycloalkyl;
W is selected from N or CX₂;
X₂ is selected from hydrogen, cyano, nitro, halogen, C₁-C₆ alkyl, C₁-C₆ haloalkyl, C₃-C₆ cycloalkyl, C₃-C₆ cycloalkyl C₁-C₆ alkyl, C₃-C₆ halocycloalkyl C₁-C₆ alkyl, Y₁ oxy, Y₁ thio, Y₁Y₂ amino, Y₁ sulfinyl, Y₁ sulfonyl, Y₁ oxy C₁-C₆ alkyl, Y₁ thio C₁-C₆ alkyl, Y₁Y₂ amino C₁-C₆ alkyl, Y₁ sulfinyl C₁-C₆ alkyl, Y₁ sulfonyl C₁-C₆ alkyl, C(O)Y₁, C(O)OY₁, OC(O)OY₁, N(Y₁)C(O)OY₂, C(O)N(Y₁)Y₂, N(Y₁)C(O)N(Y₁)Y₂, OC(O)N(Y₁)Y₂, C(O)N(Y₁)OY₂, N(Y₁)S(O)₂Y₂, N(Y₁)C(O)Y₂, OS(O)₂Y₁, CH=NOY₁, C₁-C₆ alkyl-CH=NOY₁, C₁-C₆ alkyl-O-N=C(Y₁)Y₂, phenyl, 5-7 membered aliphatic heterocycle containing 1-4 heteroatoms, 5-7 membered aromatic heterocycle containing 1-4 heteroatoms, 5-7 membered aliphatic heterocyclic C₁-C₆ alkyl containing 1-4 heteroatoms or 5-7 membered aromatic heterocyclic C₁-C₆ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy, C₃-C₆ cycloalkyl, C₃-C₆ cycloalkoxy, C₂-C₆ alkenyl, C₂-C₆ alkynyl, phenyl or halophenyl;
Y₁ and Y₂ are independently selected from C₁-C₆ alkyl, C₁-C₆ haloalkyl, C₃-C₆ cycloalkyl, C₃-C₆ cycloalkyl C₁-C₆ alkyl, C₁-C₆ alkoxy C₁-C₆ alkyl, phenyl, 5-7 membered aliphatic heterocycle containing 1-4 heteroatoms, 5-7 membered aromatic heterocycle containing 1-4 heteroatoms, 5-7 membered aliphatic heterocyclic C₁-C₆ alkyl containing 1-4 heteroatoms or 5-7 membered aromatic heterocyclic C₁-C₆ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy, C₃-C₆ cycloalkyl, C₃-C₆ cycloalkoxy, C₂-C₆ alkenyl, C₂-C₆ alkynyl, phenyl or halophenyl;
Z is selected from Z₁ or Z₂ group; 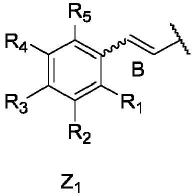
Z₂ is selected from C₃-C₈ cycloalkenyl; the hydrogen on the ring can be substituted by the following substituents; the following substituents are selected from nitro, halogen, C₁-C₆ alkyl, C₁-C₆ haloalkyl, C₁-C₆ alkoxy, C₁-C₆ haloalkoxy, C₁-C₆ alkenyl or C₃-C₆ cycloalkyl;
Q is selected from Q₁, Q₂, Q₃, Q₄, Q₅ or Q₆ group; 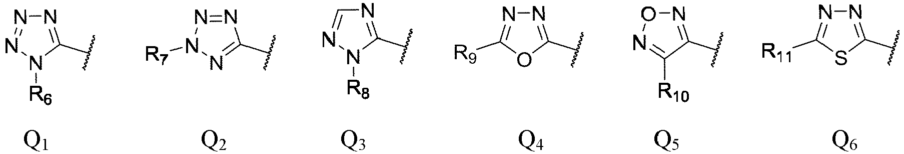
R₁ to R₅ are independently selected from hydrogen, hydroxyl, cyano, nitro, halogen, phenyl, C₁-C₆ alkyl, C₁-C₆ haloalkyl, C₂-C₆ alkenyl, C₂-C₆ haloalkenyl, C₂ -C₆ alkynyl, C₂-C₆ haloalkynyl, C₁-C₆ alkoxy, C₁-C₆ haloalkoxy, C₁-C₆ alkylthio or benzyloxy, wherein
R₁ and R₂ form a benzene ring, a 5-7 membered aliphatic heterocycle containing 1-4 heteroatoms or a 5-7 membered aromatic heterocycle containing 1-4 heteroatoms together with the carbon atoms on the connected benzene ring;
R₂ and R₃ can form a benzene ring, a 5-7 membered aliphatic heterocycle containing 1-4 heteroatoms or a 5-7 membered aromatic heterocycle containing 1-4 heteroatoms together with the carbon atoms on the connected benzene ring;
R₆, R₇ and R₈ are independently selected from hydrogen, C₁-C₆ alkyl, C₁-C₆ haloalkyl, C₃-C₆ cycloalkyl, C₃-C₆ cycloalkyl C₁-C₆ alkyl, C₂-C₆ alkenyl, C₂-C₆ alkynyl or phenyl;
R₉, R₁₀, and R₁₁ are independently selected from hydrogen, halogen, cyano, nitro, C₁-C₆ alkylsulfonyl, C₁-C₆ haloalkylsulfonyl, C₁-C₆ alkylsulfinyl, C₁-C₆ haloalkylsulfinyl, C₁-C₆ alkylthio, C₁-C₆ haloalkylthio, C₁-C₆ alkyl, C₁-C₆ haloalkyl, C₁-C₆ alkoxy, C₁-C₆ haloalkoxy, C₁-C₃ alkoxy C₁-C₃ alkyl, C₁-C₆ alkoxy C₁-C₃ alkoxy, C₁-C₆ alkoxy C₁-C₃ alkoxy C₁-C₃ alkyl, C₃-C₆ cycloalkyl, C₃-C₆ cycloalkyl C₁-C₃ alkyl, C₃-C₆ cycloalkyl C₁-C₃ alkoxy or C₃-C₆ cycloalkyloxy;
a stereoisomer of the compound of the above formula I; or, the compound of the formula I and agriculturally acceptable salt of the isomer.

Preferably, in the compound, wherein in the formula I:
X₁ and X₃ are independently selected from hydrogen, cyano, nitro, halogen, C₁-C₆ alkylsulfonyl, C₁-C₆ alkylsulfinyl, C₁-C₆ alkylthio, C₁-C₆ haloalkylthio, C₁-C₆ alkyl, C₁-C₆ haloalkyl, C₁-C₆ alkoxy, C₁-C₆ alkoxy C₁-C₃ alkyl, C₁-C₆ alkoxy C₁-C₃ alkoxy, C₁-C₆ alkoxy C₁-C₃ alkoxy C₁-C₃ alkyl, C₃-C₆ cycloalkyl, C₃-C₆ cycloalkyl C₁-C₃ alkyl, C₃-C₆ cycloalkyl, C₁-C₃ alkoxy, C₃-C₆ cycloalkyloxy, C₂-C₆ alkenyloxy, C₂-C₆ alkenyl, C₂-C₆ alkynyl, phenyl, phenylsulfonyl, phenyloxy, a 5-7 membered aliphatic heterocycle containing 1-4 heteroatoms and a 5-7 membered aromatic heterocycle containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy or C₃-C₆ cycloalkyl;
W is selected from N or CX₂;
X₂ is selected from hydrogen, cyano, nitro, halogen, C₁-C₆ alkyl, C₁-C₆ haloalkyl, C₃-C₆ cycloalkyl, C₃-C₆ cycloalkyl C₁-C₆ alkyl, C₃-C₆ halocycloalkyl C₁-C₆ alkyl, Y₁ oxy, Y₁ thio, Y₁Y₂ amino, Y₁ sulfinyl, Y₁ sulfonyl, Y₁ oxy C₁-C₆ alkyl, Y₁ thio C₁-C₆ alkyl, Y₁Y₂ amino C₁-C₆ alkyl, Y₁ sulfinyl C₁-C₆ alkyl, Y₁ sulfonyl C₁-C₆ alkyl, C(O)Y₁, C(O)OY₁, OC(O)OY₁, N(Y₁)C(O)OY₂ , C(O)N(Y₁)Y₂, N(Y₁)C(O)N(Y₁)Y₂, OC(O)N(Y₁)Y₂, C(O)N(Y₁)OY₂, N(Y₁)S(O)₂Y₂, N(Y₁)C(O)Y₂, OS(O)₂Y₁, CH=NOY₁, C₁-C₆ alkyl-CH=NOY₁, C₁-C₆ alkyl-O-N=C(Y₁)Y₂, phenyl, 5-7 membered aliphatic heterocycle containing 1-4 heteroatoms, 5-7 membered aromatic heterocycle containing 1-4 heteroatoms, 5-7 membered aliphatic heterocyclic C₁-C₆ alkyl containing 1-4 heteroatoms or 5-7 membered aromatic heterocyclic C₁-C₆ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy, C₃-C₆ cycloalkyl or C₃-C₆ cycloalkoxy;
Y₁ and Y₂ are independently selected from C₁-C₆ alkyl, C₁-C₆ haloalkyl, C₃-C₆ cycloalkyl, C₃-C₆ cycloalkyl C₁-C₆ alkyl, C₁-C₆ alkoxy C₁-C₆ alkyl, phenyl, a 5-7 membered aliphatic heterocycle containing 1-4 heteroatoms, a 5-7 membered aromatic heterocycle containing 1-4 heteroatoms, a 5-7 membered aliphatic heterocyclic C₁-C₆ alkyl containing 1-4 heteroatoms or a 5-7 membered aromatic heterocyclic C₁-C₆ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy, C₃-C₆ cycloalkyl or C₃-C₆ cycloalkoxy;
Z is selected from Z₁ or Z₂ group; 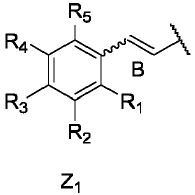
Z₂ is selected from C₃-C₈ cycloalkenyl; the hydrogen on the ring can be substituted by the following substituents; the following substituents are selected from nitro, halogen, C₁-C₆ alkyl, C₁-C₆ haloalkyl, C₁-C₆ alkoxy, C₁-C₆ haloalkoxy, C₁-C₆ alkenyl or C₃-C₆ cycloalkyl;
Q is selected from Q₁, Q₂, Q₃, Q₄ or Q₅ group; 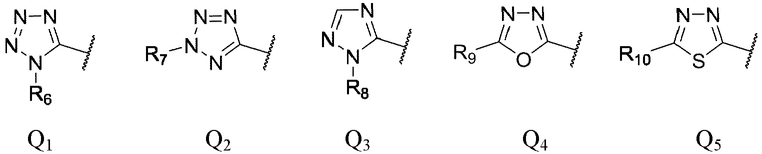
R₁ to R₅ are independently selected from hydrogen, hydroxyl, cyano, nitro, halogen, phenyl, C₁-C₆ alkyl, C₂-C₆ alkenyl, C₂-C₆ alkynyl, C₁-C₆ alkoxy, C₁-C₆ alkylthio or benzyloxy,
wherein R₁ and R₂ form a benzene ring, a 5-7 membered aliphatic heterocycle containing 1-4 heteroatoms or a 5-7 membered aromatic heterocycle containing 1-3 heteroatoms together with the carbon atoms on the connected benzene ring;
R₂ and R₃ can form a benzene ring, a 5-7 membered aliphatic heterocycle containing 1-4 heteroatoms or a 5-7 membered aromatic heterocycle containing 1-3 heteroatoms together with the carbon atoms on the connected benzene ring;
R₆, R₇ and R₈ are independently selected from hydrogen, C₁-C₆ alkyl, C₃-C₆ cycloalkyl, C₂-C₆ alkenyl, C₂-C₆ alkynyl or phenyl;
R₉ and R₁₀ are independently selected from hydrogen, halogen, C₁-C₆ alkylthio, C₁-C₆ alkyl, C₁-C₆ alkoxy, C₁-C₃ alkoxy C₁-C₃ alkyl, C₁-C₆ alkoxy C₁-C₃ alkoxy, C₁-C₆ alkoxy C₁-C₃ alkoxy C₁-C₃ alkyl or C₃-C₆ cycloalkyl;
the Z of the above formula I is selected from the stereoisomer of the compound shown by Z₁.

Further preferably, in the compound, wherein in the formula I:
X₁ and X₃ are independently selected from hydrogen, cyano, nitro, halogen, C₁-C₃ alkylsulfonyl, C₁-C₃ alkylsulfinyl, C₁-C₃ alkylthio, C₁-C₃ haloalkylthio, C₁-C₃ alkyl, C₁-C₃ haloalkyl, C₁-C₃ alkoxy, C₁-C₃ alkoxy C₁-C₃ alkyl, C₁-C₃ alkoxy C₁-C₃ alkoxy, C₁-C₃ alkoxy C₁-C₃ alkoxy C₁-C₃ alkyl, C₃-C₆ cycloalkyl or C₃-C₆ cycloalkyloxy;
W is selected from N or CX₂;
X₂ is selected from hydrogen, cyano, nitro, halogen, C₁-C₃ alkyl, C₁-C₃ haloalkyl, C₃-C₆ cycloalkyl, C₃-C₆ cycloalkyl C₁-C₃ alkyl, C₃-C₆ halocycloalkyl C₁-C₃ alkyl, Y₁ oxy, Y₁ thio, Y₁Y₂ amino, Y₁ sulfinyl, Y₁ sulfonyl, Y₁ oxy C₁-C₃ alkyl, Y₁ thio C₁-C₃ alkyl, Y₁Y₂ amino C₁-C₃ alkyl, Y₁ sulfinyl C₁-C₃ alkyl, Y₁ sulfonyl C₁-C₃ alkyl, C(O)Y₁, C(O)OY₁, OC(O)OY₁, N(Y₁)C(O)OY₂ , C(O)N(Y₁)Y₂, N(Y₁)C(O)N(Y₁)Y₂, OC(O)N(Y₁)Y₂, C(O)N(Y₁)OY₂, N(Y₁)S(O)₂Y₂, N(Y₁)C(O)Y₂, OS(O)₂Y₁, CH=NOY₁, C₁-C₆ alkyl-CH=NOY₁, C₁-C₆ alkyl-O-N=C(Y₁)Y₂, phenyl, 5-7 membered aliphatic heterocycle containing 1-4 heteroatoms, 5-7 membered aromatic heterocycle containing 1-4 heteroatoms, 5-7 membered aliphatic heterocyclic C₁-C₃ alkyl containing 1-4 heteroatoms or 5-7 membered aromatic heterocyclic C₁-C₃ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₃ alkyl, C₁-C₃ haloalkyl, C₁-C₃ alkoxy, C₃-C₆ cycloalkyl or C₃-C₆ cycloalkoxy;
Y₁ and Y₂ are independently selected from C₁-C₆ alkyl, C₁-C₆ haloalkyl, C₃-C₆ cycloalkyl, C₃-C₆ cycloalkyl C₁-C₆ alkyl, C₁-C₆ alkoxy C₁-C₆ alkyl, phenyl, a 5-7 membered aliphatic heterocycle containing 1-4 heteroatoms, a 5-7 membered aromatic heterocycle containing 1-4 heteroatoms, a 5-7 membered aliphatic heterocyclic C₁-C₆ alkyl containing 1-4 heteroatoms or a 5-7 membered aromatic heterocyclic C₁-C₆ alkyl containing 1-4 heteroatoms; the hydrogen on the phenyl, the aliphatic heterocycle, and the aromatic heterocycle may be substituted by one or more of the following substituents which are selected from nitro, halogen, C₁-C₆ alkyl, C₁-C₆ haloalkyl, C₁-C₆ alkoxy, C₁-C₆ haloalkoxy, C₃-C₆ cycloalkyl or C₃-C₆ cycloalkoxy;
Z is selected from Z₁ or Z₂ group; 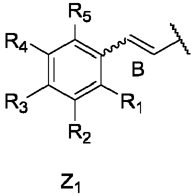
Z₂ is selected from C₅-C₆ cycloalkenyl; the hydrogen on the ring may be substituted by the following substituents which are selected from C₁-C₆ alkyl, C₁-C₆ alkoxy or C₁-C₆ alkenyl;
Q is selected from Q₁, Q₂, Q₃ or Q₄ group; 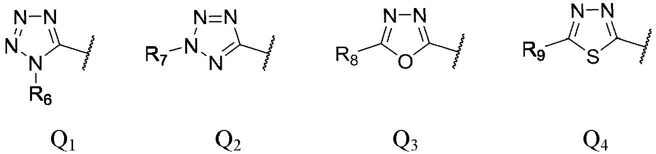
R₁ to R₅ are independently selected from hydrogen, hydroxyl, cyano, nitro, halogen, phenyl, C₁-C₆ alkyl, C₂-C₆ alkenyl, C₂-C₆ alkynyl, C₁-C₆ alkoxy or benzyloxy, wherein
R₁ and R₂ form a benzene ring or a 5-7 membered aliphatic heterocycle containing 1-4 heteroatoms together with the carbon atoms on the connected benzene ring;
R₂ and R₃ can form a benzene ring or a 5-7 membered aliphatic heterocycle containing 1-4 heteroatoms together with the carbon atoms on the connected benzene ring;
R₆ and R₇ are independently selected from hydrogen, C₁-C₆ alkyl, C₂-C₆ alkenyl or phenyl;
R₈ and R₉ are independently selected from hydrogen, halogen, C₁-C₆ alkyl, C₁-C₆ alkoxy, C₁-C₃ alkoxy C₁-C₃ alkyl, C₁-C₆ alkoxy C₁-C₃ alkoxy or C₃-C₆ cycloalkyl;
the Z of the above formula I is selected from the stereoisomer of the compound shown by Z₁.

More further preferably, in the compound, wherein in the formula I:
X₁ and X₃ are independently selected from hydrogen, cyano, nitro, halogen, C₁-C₃ alkylsulfonyl, C₁-C₃ alkyl and C₁-C₃ haloalkyl;
W is selected from N or CX₂;
X₂ is selected from hydrogen, halogen, C₁-C₃ alkyl, C₁-C₃ haloalkyl, C₃-C₆ cycloalkyl, C₃-C₆ cycloalkyl C₁-C₃ alkyl, C₃-C₆ halocycloalkyl C₁-C₃ alkyl, Y₁ oxy, Y₁ thio, Y₁Y₂ amino, Y₁ sulfonyl, Y₁ oxy C₁-C₃ alkyl, Y₁ thio C₁-C₃ alkyl, Y₁Y₂ amino C₁-C₃ alkyl, Y₁ sulfonyl C₁-C₃ alkyl, C(O)Y₁, C(O)OY₁, OC(O)OY₁, N(Y₁)C(O)OY₂ , C(O)N(Y₁)Y₂, N(Y₁)C(O)N(Y₁)Y₂, OC(O)N(Y₁)Y₂, C(O)N(Y₁)OY₂, N(Y₁)S(O)₂Y₂, N(Y₁)C(O)Y₂, OS(O)₂Y₁, CH=NOY₁, C₁-C₆ alkyl-CH=NOY₁, C₁-C₆ alkyl-O-N=C(Y₁)Y₂, phenyl, 5-7 membered aliphatic heterocycle containing 1-4 heteroatoms, 5-7 membered aromatic heterocycle containing 1-4 heteroatoms, 5-7 membered aliphatic heterocyclic C₁-C₃ alkyl containing 1-4 heteroatoms or 5-7 membered aromatic heterocyclic C₁-C₃ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₃ alkyl, C₁-C₃ haloalkyl, C₁-C₃ alkoxy, C₃-C₆ cycloalkyl or C₃-C₆ cycloalkoxy;
Y₁ and Y₂ are independently selected from C₁-C₆ alkyl, C₁-C₆ haloalkyl, C₃-C₆ cycloalkyl, C₃-C₆ cycloalkyl C₁-C₆ alkyl, C₁-C₆ alkoxy C₁-C₆ alkyl, phenyl, a 5-7 membered aliphatic heterocycle containing 1-4 heteroatoms, a 5-7 membered aromatic heterocycle containing 1-4 heteroatoms, a 5-7 membered aliphatic heterocyclic C₁-C₆ alkyl containing 1-4 heteroatoms or a 5-7 membered aromatic heterocyclic C₁-C₆ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy, C₃-C₆ cycloalkyl or C₃-C₆ cycloalkoxy;
Z is selected from Z₁ or Z₂ group; 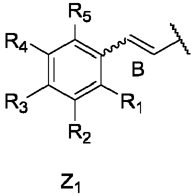
Z₂ is selected from G₁, G₂, G₃, G₄, G₅ or G₆ group; 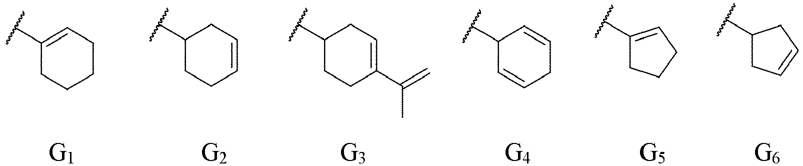
Q is selected from Q₁, Q₂, Q₃ or Q₄ group; 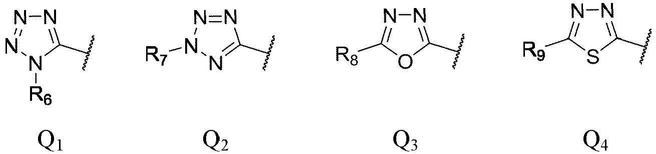
R₁ to R₅ are independently selected from hydrogen, hydroxyl, cyano, nitro, halogen, phenyl, methyl, ethyl, propyl, vinyl, propenyl, ethynyl, propynyl, methoxy, ethoxyl, benzyloxy, trifluoromethyl or trifluoromethoxy;
R₁ and R₂ can form a benzene ring together with the carbon atoms on the connected benzene ring;
R₂ and R₃ can form a benzene ring, 1,3-dioxane ring or 1,4-dioxane ring together with the carbon atoms on the connected benzene ring;
R₆ and R₇ are independently selected from hydrogen, methyl or ethyl;
R₈ and R₉ are independently selected from hydrogen, chlorine or methyl;
the Z of the above formula I is selected from a trans-stereoisomer of the compound shown by Z₁.

In the definitions of the compounds of the formula I provided above, the terms used in the collection are defined as follows:
Alkyl refers to linear or branched groups such as methyl, ethyl, n-propyl, isopropyl, n-butyl, isobutyl, sec-butyl, tert-butyl, n-pentyl, isopentyl,n-hexyl and so on. Cycloalkyl refers to groups in the form of cyclic chain, such as cyclopropyl, methylcyclopropyl, cyclopropylcyclopropyl, cyclobutyl, cyclopentyl and cyclohexyl and so on. Alkenyl refers to linear or branched alkenyl, such as vinyl, 1-propenyl, 2-propenyl, butenyl, pentenyl and hexenyl and so on. Alkynyl refers to linear or branched chain alkynyl, such as 1-propynyl, 2-propynyl, butynyl, pentynyl and hexynyl and so on. Alkoxy refers to a group having an oxygen atom at the end of the alkyl, such as methoxy, ethoxy, n-propoxy, isopropoxy and tert-butoxy and so on. The 5-7-membered heterocycle containing 1-4 heteroatoms refers to a 5-7-membered heterocyclic compound containing 1-4 heteroatoms without aromatic characteristics, such as ethylene oxide, tetrahydrofuran, imidazolinone, caprolactam, 1,3-dioxane ring and 1,4-dioxane ring and so on. The 5-7-membered aromatic heterocycle containing 1-4 heteroatoms refers to a 5-7-membered heterocyclic compound containing 1-4 heteroatoms having aromatic characteristics, such as furan, thiophene, pyrazole and pyridine and so on. Stereoisomers mean that hydrogen atoms on the carbon-carbon double bond B in the formula I are on the same side (cis) or on both sides (trans) of the bond B.

The compound I of the formula in the present invention can be prepared by the following method:
Method I:

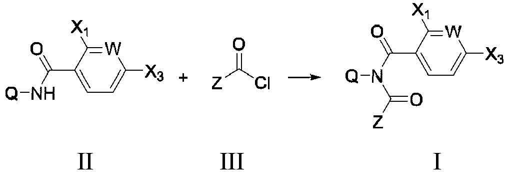

The compound of the formula II and the compound of the formula III react in a suitable solvent at temperature of -10°C to a boiling point of the suitable solvent for 0.5-48 hours to obtain a target compound I.

The suitable solvent is selected from dichloromethane, 1,2-dichloroethane, chloroform, carbon tetrachloride, hexane, benzene, toluene, ethyl acetate, acetonitrile, acetic acid, tetrahydrofuran, dioxane, N,N-dimethylformamide or dimethylsulfoxide and so on.

Addition of a suitable alkali substance in the reaction system can be beneficial to the reaction. The suitable alkali is selected from organic alkali such as triethylamine, N, N-dimethylaniline or pyridine and so on, or inorganic alkali such as sodium hydroxide, potassium hydroxide, sodium carbonate, sodium bicarbonate, potassium carbonate, sodium methoxide, sodium tert-butoxide or potassium tert-butoxide and so on.

The compound of the formula III can be prepared from the corresponding acid (commercially available) by reference to WO2009123714.

### Method II:

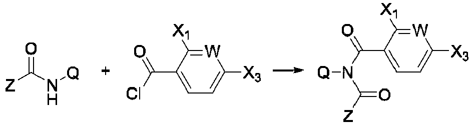

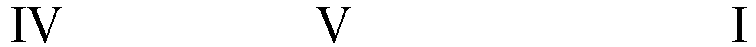

The compound of the formula IV and the compound of the formula V react in a suitable solvent at temperature of -10°C to a boiling point of the suitable solvent for 0.5-48 hours to obtain a target compound I.

The suitable solvent is selected from dichloromethane, 1,2-dichloroethane, chloroform, carbon tetrachloride, hexane, benzene, toluene, ethyl acetate, acetonitrile, acetic acid, tetrahydrofuran, dioxane, N,N-dimethylformamide or dimethylsulfoxide and so on.

Addition of a suitable alkali substance in the reaction system can be beneficial to the reaction. The suitable alkali is selected from organic alkali such as triethylamine, N, N-dimethylaniline or pyridine and so on, or inorganic alkali such as sodium hydroxide, potassium hydroxide, sodium carbonate, sodium bicarbonate, potassium carbonate, sodium methoxide, sodium tert-butoxide or potassium tert-butoxide and so on.

The compound of the formula V can be prepared from the corresponding acid (commercially available) by reference to WO2009123714.

The preparation method of the compound of the formula II is as follows:

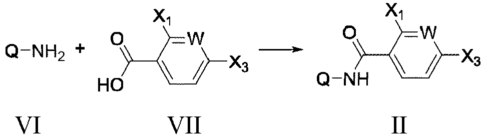

The compound of the formula VI (commercially available) and the compound of the formula VII (commercially available) react with a suitable activator in suitable alkali and solvent at temperature of -10°C to a boiling point of the suitable solvent for 0.5-48 hours to obtain the compound of the formula II. The suitable solvent is selected from petroleum ether, hexane, benzene, toluene, chlorobenzene, ethyl acetate, acetonitrile, tetrahydrofuran, N, N-dimethylformamide, dimethylsulfoxide, pyridine, 2-methylpyridine, 3-methylpyridine or 4-methylpyridine and so on. The suitable activator is selected from phosgene, triphosgene, CDI, DCC, dichlorosulfoxide, oxalyl chloride, phosphorus oxychloride, or phosphorus pentachloride and so on. The suitable alkali is selected from N-methylimidazole or DMAP and so on.

The preparation method of the compound of the formula IV can be referred to the preparation method of the compound of the formula II.

The compound of the formula I of the present invention and the stereoisomer thereof or the compound of the formula I and the agriculturally acceptable salt of the isomer have herbicidal activity and can be used for agriculturally controlling various weeds. Compared with the compound disclosed in the prior art, the alkene-containing amide compound of the present invention not only has excellent herbicidal activity, but also is safe for crops.

The present invention also comprises a herbicidal composition using the compound of the formula I and the stereoisomer thereof or the compound of the formula I and the agriculturally acceptable salt of the isomer as active ingredients. The weight percentage of the active ingredient in the herbicidal composition is 1-99%. The herbicidal composition also comprises an agriculturally acceptable carrier.

The herbicidal composition of the present invention can be applied in the forms of various formulations. The compound of the present invention is generally dissolved or dispersed in the carrier and prepared into the formulation for easier dispersion when used as a herbicide. For example, the chemical formulations can be prepared into wettable powder or missible oil. Therefore, in the compositions, at least one liquid or solid carrier is added, and generally a suitable surfactant needs to be added.

The present invention also provides an implementing method for controlling weeds. The method comprises applying an effective dose of the herbicidal composition of the present invention to the weed or a weed growing place or a surface of a growth medium thereof. A suitable effective dose is 1 to 1000 grams per hectare, and a preferred effective dose is 10 to 500 grams per hectare. For some applications, one or more other herbicides can be added to the herbicidal composition of the present invention, thereby generating additional advantages and effects.

The compound of the present invention can be used alone or in combination with other known pesticides, bactericides, plant growth regulators or fertilizers.

It should be clear that various changes and modifications can be made within the scope defined by the claims of the present invention.

The present invention has the following advantages:
Compared with the known benzamide compound, the compound of the formula in the present invention comprises a benzoyl and an alkene-containing acyl substitution, and has a novel structure. The alkene-containing amide compound in the present invention has unexpectedly high herbicidal activity, also has high herbicidal activity at a lower dosage, not only has high efficiency, but also reduces the use amount of pesticides, reduces the cost and reduces environmental pollution.

### Detailed Description

The following examples and biometric test results can be used to further illustrate the present invention, but are not intended to limit the present invention.

### Synthesis Example

### Embodiment 1 Synthesis of compound 1-1:

### (1) Synthesis of N-(1-methyl-tetrazole-5yl) -2-methanesulfonyl-4-trifluoromethylbenzamide

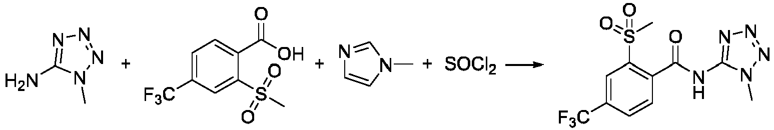

2-methanesulfonyl-4-trifluoromethylbenzoic acid (19.1 g, 71.2 mmol), 1-methyl-5-aminotetrazole (8.5 g, 85.4 mmol), 3-methylpyridine (100 ml) and N-methylimidazole (11.7 g, 142 mmol) were added to a reaction flask, stirred at room temperature for half an hour, and cooled to be below 10 °C in an ice-water bath; dichlorosulfoxide (13.6 g, 114.0 mmol) was slowly dripped; the mixture was stirred at room temperature for 2 hours, heated to 50 °C to preserve heat and react for 2 hours, and cooled to be below 10 °C in the ice-water bath; cold water was slowly dripped; solid precipitated out and was filtered; a filter cake was washed twice with 100 ml of water and dried to obtain 17.5 g of white solid, with a yield of 70%.

### (2) Synthesis of cinnamyl chloride

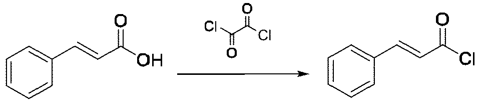

Cinnamic acid (10.0 g, 67.5 mmol), dichloromethane (300 ml) and DMF (3 drops) were added into the reaction flask; oxalyl chloride (42.8 g, 337.5 mmol) was slowly added; the mixture was stirred at room temperature for 2 hours; the solvent was evaporated under reduced pressure; toluene (150 ml) was added to the residue and stirred for 3 minutes; and then the solvent was evaporated under reduced pressure to obtain 11.3 g of yellow solid which was used directly in the next step.

### (3) Synthesis of compound 1-1

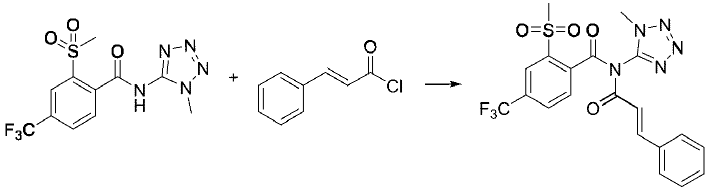

N-(1-methyl-tetrazole-5-yl) -2-methanesulfonyl-4-trifluoromethylbenzamide (1.2 g, 3.4 mmol), dichloromethane (20 ml) and triethylamine (0.7 g, 6.8 mmol) were added to the reaction flask, and the prepared dichloromethane solution of the cinnamyl chloride was dropwise added (the cinnamyl chloride (1.1g, 6.8 mmol) was dissolved in 15 ml of dichloromethane). The mixture was stirred at room temperature for 40 minutes; the solvent was evaporated under reduced pressure; ethyl acetate (50 ml) was added to the residue; water (50 ml) was used for separation and extraction; the organic phase was washed with saturated salt water (50 ml) and dried with anhydrous magnesium sulfate; the solvent was evaporated under reduced pressure; and the residue was separated by column chromatography to obtain 1.3 g of pale yellow solid, with a purity of 94% and a yield of 82%.

### Embodiment 2 Synthesis of compound 2-265:

### (1) Synthesis of 1-cyclohexenoyl chloride

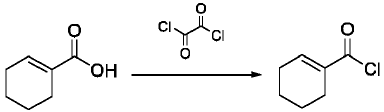

1-cyclohexenoic acid (0.36 g, 2.9 mmol), dichloromethane (30 ml) and DMF (1 drop) were added into the reaction flask; oxalyl chloride (1.82 g, 14.3 mmol) was slowly added; the mixture was stirred at room temperature for 1 hour; the solvent was evaporated under reduced pressure; toluene (15 ml) was added to the residue and stirred for 3 minutes; and then the solvent was evaporated under reduced pressure to obtain 0.42 g of pale yellow solid which was used directly in the next step.

### (2) Synthesis of compound 2-265

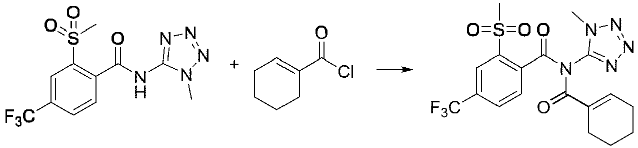

N-(1-methyl-tetrazole-5-yl) -2-methanesulfonyl-4-trifluoromethylbenzamide (0.5 g, 1.4 mmol, see step 3 of embodiment 1 for hte preparation), dichloromethane (20 ml) and triethylamine (0.29 g, 2.9 mmol) were added to the reaction flask, and the dichloromethane solution (15 ml) of 1-cyclohexenoyl chloride in the above step was added dropwise. The mixture was stirred at room temperature for 1 hour; the solvent is evaporated under reduced pressure; ethyl acetate (100 ml) was added to the residue; water (50 ml) was used for separation and extraction; the organic phase was sequentially washed with saturated salt water (50 ml) and dried with anhydrous magnesium sulfate; the solvent was evaporated under reduced pressure; and the residue was separated by column chromatography to obtain 0.55 g of off-white solid compound 2-265, with a purity of 97.6% and a yield of 82%.

### Embodiment 3 Synthesis of compound 1-24:

### (1) Synthesis of N-(1-methyl-tetrazole-5-yl)-2-chloro-3-methoxymethyl-4-methanesulfonyl benzamide

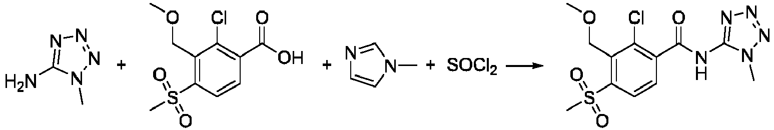

2-chloro-3-methoxymethyl-4-methanesulfonyl benzoic acid (5.0 g, 17.9 mmol), 1-methyl-5-aminotetrazole (2.1 g, 21.5 mmol), 3-methylpyridine (30 ml) and N-methylimidazole (3.0 g, 35.9 mmol) were added to the reaction flask, stirred at room temperature for half an hour, and cooled to be below 10 °C in an ice-water bath; dichlorosulfoxide (3.4 g, 28.7 mmol) was slowly dripped; the mixture was stirred at room temperature for 2 hours, heated to 50 °C to preserve heat and react for 2 hours, and cooled to be below 10 °C in the ice-water bath; cold water was slowly dripped; solid precipitated out and was filtered; a filter cake was washed twice with 30 ml of water and dried to obtain 3.29 g of off-white solid, with a yield of 51%.

### (2) Synthesis of compound 1-24

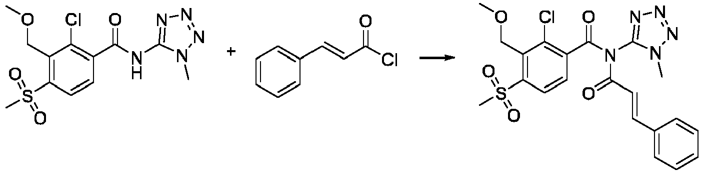

N-(1-methyl-tetrazole-5-yl)-2-chloro-3-methoxymethyl-4-methanesulfonyl benzamide (0.5 g, 1.4 mmol), dichloromethane (20 ml) and triethylamine (0.3 g, 2.8 mmol) were added to the reaction flask, and the prepared dichloromethane solution of the cinnamyl chloride was dropwise added (0.5 g of cinnamyl chloride was dissolved in 15 ml of dichloromethane). The mixture was stirred at room temperature for 40 minutes; the solvent was evaporated under reduced pressure; ethyl acetate (50 ml) was added to the residue; water (50 ml) was used for separation and extraction; the organic phase was washed with saturated salt water (50 ml) and dried with anhydrous magnesium sulfate; the solvent was evaporated under reduced pressure; and the residue was separated by column chromatography to obtain 0.4 g of pale yellow solid, with a purity of 95% and a yield of 56%.

### Embodiment 4 Synthesis of compound 1-41:

### (1) Synthesis of N-(1-methyl-tetrazole-5yl) cinnamamide

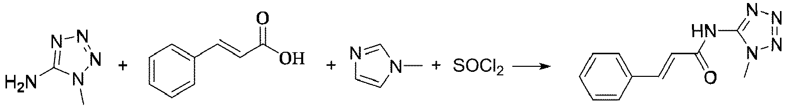

Cinnamic acid (5.0 g, 33.7 mmol), 1-methyl-5-aminotetrazole (3.7 g, 37.1 mmol), 3-methylpyridine (50 ml) and N-methylimidazole (5.5 g, 67.5 mmol) were added to the reaction flask, stirred at room temperature for half an hour, and cooled to be below 10 °C in an ice-water bath; dichlorosulfoxide (6.4 g, 54.0 mmol) was slowly dripped; the mixture was stirred at room temperature for 2 hours, heated to 50 °C to preserve heat and react for 2 hours, and cooled to be below 10 °C in the ice-water bath; cold water was slowly dripped; solid precipitated out and was filtered; a filter cake was washed twice with 50 ml of water and dried to obtain 3.3 g of yellow solid, with a yield of 42%.

### (2) Synthesis of 2-chloro-3-{[(tetrahydrofuran-2-yl)methoxy]methyl}-4-methanesulfonyl benzoyl chloride

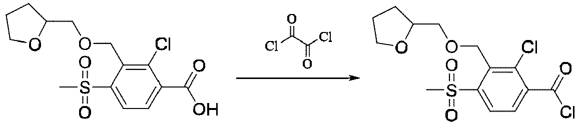

2-chloro-3-{[(tetrahydrofuran-2-yl)methoxy]methyl}-4-methanesulfonyl benzoic acid (1.3 g, 3.8 mmol), dichloromethane (20 ml) and DMF (1 drop) were added into the reaction flask; oxalyl chloride (2.4 g, 19.0 mmol) was slowly added; the mixture was stirred at room temperature for 2 hours; the solvent was evaporated under reduced pressure; toluene (10 ml) was added to the residue and stirred for 3 minutes; and then the solvent was evaporated under reduced pressure to obtain 1.3 g of yellow solid which was used directly in the next step.

### (3) Synthesis of compound 1-41

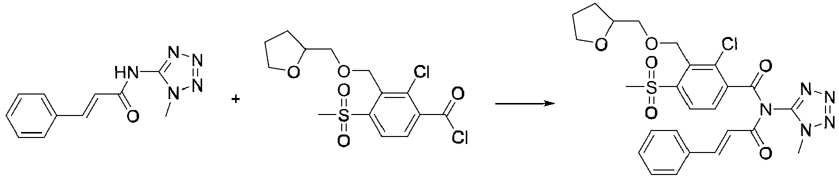

N-(1-methyl-tetrazole-5yl) cinnamamide (0.4 g, 1.9 mmol), dichloromethane (20 ml) and triethylamine (0.4 g, 3.8 mmol) were added to the reaction flask, and the dichloromethane solution of the prepared 2-chloro-3-{[(tetrahydrofuran-2-yl)methoxy]methyl}-4-methanesulfonyl benzoyl chloride was dropwise added (1.3 g of 2-chloro-3-{[(tetrahydrofuran-2-yl)methoxy]methyl}-4-methanesulfonyl benzoyl chloride was dissolved in 15 ml of dichloromethane). The mixture was stirred at room temperature for 40 minutes; the solvent was evaporated under reduced pressure; ethyl acetate (50 ml) was added to the residue; water (50 ml) was used for separation and extraction; the organic phase was washed with saturated salt water (50 ml) and dried with anhydrous magnesium sulfate; the solvent was evaporated under reduced pressure; and the residue was separated by column chromatography to obtain 0.3 g of yellow solid, with a purity of 88% and a yield of 25%.

The initial substances are replaced according to the above recorded method to obtain other compounds shown by the formula I. Part of the compounds of the formula I can be found in Table 1, Table 2, Table 3 and Table 4, wherein in Table 1 and Table 2, W is selected from CX₂ and the stereo configuration in Table 1 is trans; in Table 3 and Table 4, W is selected from N and the stereo configuration in Table 3 is trans.

In the compound of the formula I, W is CX₂ and the stereo configuration is trans.

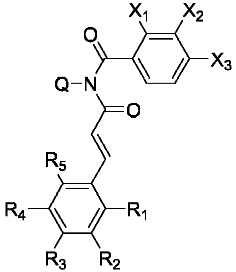

**Table 1 Structures and Physical Properties of Part of Compounds of Formula I**

| Compound | X₁ | X₂ | X₃ | Q | R₁ | R₂ | R₃ | R₄ | R₅ | Appearance (Melting Point °C) |
|---|---|---|---|---|---|---|---|---|---|---|
| 1-1 | SO₂CH₃ | H | CF₃ | 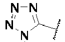 | H | H | H | H | H | pale yellow solid (145-146) |
| 1-2 | SO₂CH₃ | H | CF₃ | 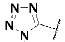 | Cl | H | Cl | H | H | |
| 1-3 | SO₂CH₃ | H | CF₃ | 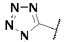 | H | H | OCH₃ | H | H | |
| 1-4 | SO₂CH₃ | H | CF₃ | 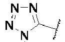 | H | H | NO₂ | H | H | |
| 1-5 | SO₂CH₃ | H | CF₃ | 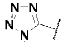 | H | H | CF₃ | H | H | |
| 1-6 | SO₂CH₃ | H | CF₃ | 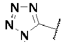 | H | OCF₃ | H | H | H | |
| 1-7 | SO₂CH₃ | H | CF₃ | 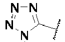 | H | H | 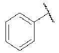 | H | H | |
| 1-8 | SO₂CH₃ | H | CF₃ | 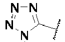 | H | 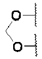 | | H | H | yellow solid (121-123) |
| 1-9 | SO₂CH₃ | H | CF₃ | 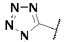 | H | 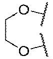 | | H | H | |
| 1-10 | SO₂CH₃ | H | CF₃ | 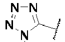 | H | 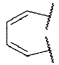 | | H | H | |
| 1-11 | SO₂CH₃ | H | CF₃ | 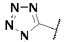 | 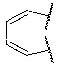 | | H | H | H | |
| 1-12 | NO₂ | H | SO₂CH₃ | 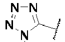 | H | H | H | H | H | pale yellow solid (189-191) |
| 1-13 | NO₂ | H | SO₂CH₃ | 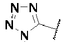 | H | 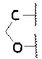 | | H | H | yellow solid (185-187) |
| 1-14 | NO₂ | H | Cl | 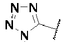 | H | H | H | H | H | white solid (157-159) |
| 1-15 | Cl | H | Cl | 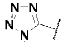 | H | H | H | H | H | yellow solid (148-150) |
| 1-16 | Cl | H | Cl | 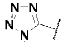 | H | 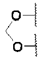 | | H | H | yellow solid (160-162) |
| 1-17 | Cl | H | SO₂CH₃ | 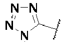 | H | H | H | H | H | white solid (190-192) |
| 1-18 | Cl | H | SO₂CH₃ | 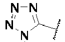 | H | 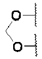 | | H | H | pale yellow solid (195-197) |
| 1-19 | Cl | CH₃ | SO₂CH₃ | 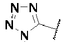 | H | H | H | H | H | white solid (185-186) |
| 1-20 | Cl | CH₃ | SO₂CH₃ | 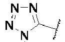 | H | 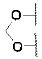 | | H | H | pale yellow solid (192-194) |
| 1-21 | Cl | CH₃ | SO₂CH₃ | 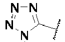 | H | H | OCH3 | H | H | pale yellow solid (200-202) |
| 1-22 | Cl | CH₃ | SO₂CH₃ | 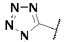 | H | H | 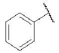 | H | H | pale yellow solid (175-177) |
| 1-23 | Cl | CH₂Br | SO₂CH₃ | 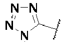 | H | H | H | H | H | |
| 1-24 | Cl | 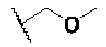 | SO₂CH₃ | 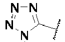 | H | H | H | H | H | pale yellow solid (180-182) |
| 1-25 | Cl | 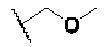 | SO₂CH₃ | 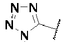 | Cl | H | Cl | H | H | |
| 1-26 | Cl | 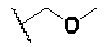 | SO₂CH₃ | 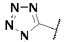 | H | H | OCH₃ | H | H | yellow solid (160-162) |
| 1-27 | Cl | 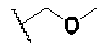 | SO₂CH₃ | 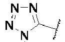 | H | H | NO₂ | H | H | |
| 1-28 | Cl | 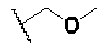 | SO₂CH₃ | 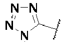 | H | H | CF₃ | H | H | white solid (164-165) |
| 1-29 | Cl | 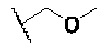 | SO₂CH₃ | 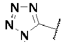 | H | OCF₃ | H | H | H | |
| 1-30 | Cl | 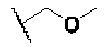 | SO₂CH₃ | 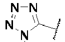 | H | H | 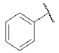 | H | H | pale yellow solid (120-123) |
| 1-31 | Cl | 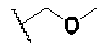 | SO₂CH₃ | 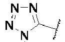 | H | 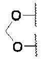 | | H | H | pale yellow solid (115-117) |
| 1-32 | Cl | 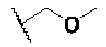 | SO₂CH₃ | 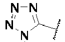 | H | 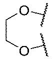 | | H | H | |
| 1-33 | Cl | 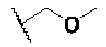 | SO₂CH₃ | 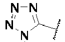 | H | 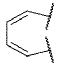 | | H | H | yellow solid (120-122) |
| 1-34 | Cl | 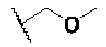 | SO₂CH₃ | 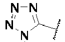 | 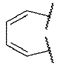 | | H | H | H | |
| 1-35 | Cl | 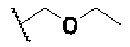 | SO₂CH₃ | 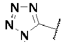 | H | H | H | H | H | |
| 1-36 | Cl | 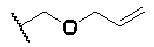 | SO₂CH₃ | 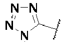 | H | H | H | H | H | |
| 1-37 | Cl | 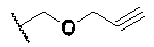 | SO₂CH₃ | 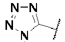 | H | H | H | H | H | |
| 1-38 | Cl | 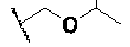 | SO₂CH₃ | 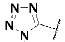 | H | H | H | H | H | |
| 1-39 | Cl | 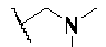 | SO₂CH₃ | 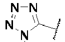 | H | H | H | H | H | |
| 1-40 | Cl | 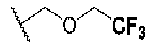 | SO₂CH₃ | 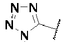 | H | H | H | H | H | |
| 1-41 | Cl | 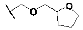 | SO₂CH₃ | 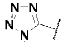 | H | H | H | H | H | yellow solid (98-100) |
| 1-42 | Cl | 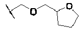 | SO₂CH₃ | 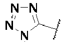 | Cl | H | Cl | H | H | |
| 1-43 | Cl | 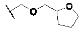 | SO₂CH₃ | 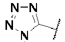 | H | H | OCH₃ | H | H | |
| 1-44 | Cl | 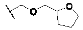 | SO₂CH₃ | 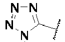 | H | H | NO₂ | H | H | |
| 1-45 | Cl | 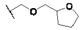 | SO₂CH₃ | 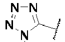 | H | H | CF₃ | H | H | |
| 1-46 | Cl | 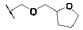 | SO₂CH₃ | 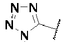 | H | OCF₃ | H | H | H | |
| 1-47 | Cl | 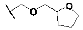 | SO₂CH₃ | 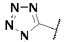 | H | H | 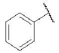 | H | H | |
| 1-48 | Cl | 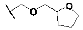 | SO₂CH₃ | 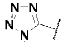 | H | 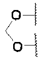 | | H | H | |
| 1-49 | Cl | 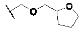 | SO₂CH₃ | 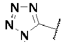 | H | 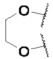 | | H | H | |
| 1-50 | Cl | 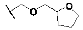 | SO₂CH₃ | 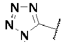 | H | 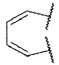 | | H | H | |
| 1-51 | Cl | 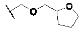 | SO₂CH₃ | 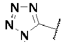 | 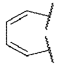 | | H | H | H | |
| 1-52 | Cl | 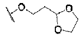 | SO₂CH₃ | 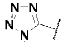 | H | H | H | H | H | |
| 1-53 | Cl | 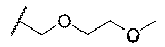 | SO₂CH₃ | 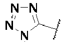 | H | H | H | H | H | pale yellow solid (85-87) |
| 1-54 | Cl | 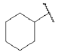 | SO₂CH₃ | 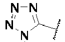 | H | H | H | H | H | |
| 1-55 | Cl | 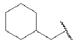 | SO₂CH₃ | 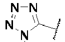 | H | H | H | H | H | |
| 1-56 | Cl | 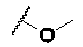 | SO₂CH₃ | 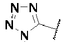 | H | H | H | H | H | white oil |
| 1-57 | Cl | 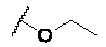 | SO₂CH₃ | 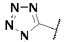 | H | H | H | H | H | white solid (200-201) |
| 1-58 | Cl | 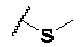 | SO₂CH₃ | 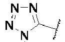 | H | H | H | H | H | |
| 1-59 | Cl | 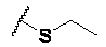 | SO₂CH₃ | 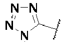 | H | H | H | H | H | |
| 1-60 | Cl | 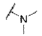 | SO₂CH₃ | 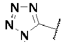 | H | H | H | H | H | |
| 1-61 | Cl | SO₂CH₃ | SO₂CH₃ | 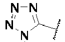 | H | H | H | H | H | |
| 1-62 | Cl | 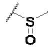 | SO₂CH₃ | 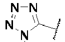 | H | H | H | H | H | |
| 1-63 | Cl | 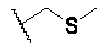 | SO₂CH₃ | 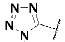 | H | H | H | H | H | |
| 1-64 | Cl | 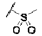 | SO₂CH₃ | 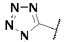 | H | H | H | H | H | |
| 1-65 | Cl | 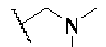 | SO₂CH₃ | 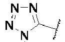 | H | H | H | H | H | |
| 1-66 | Cl | 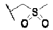 | SO₂CH₃ | 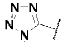 | H | H | H | H | H | |
| 1-67 | Cl | 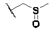 | SO₂CH₃ | 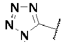 | H | H | H | H | H | |
| 1-68 | Cl | 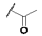 | SO₂CH₃ | 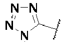 | H | H | H | H | H | |
| 1-69 | Cl | 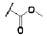 | SO₂CH₃ | 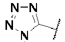 | H | H | H | H | H | |
| 1-70 | Cl | 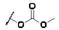 | SO₂CH₃ | 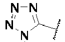 | H | H | H | H | H | |
| 1-71 | Cl | 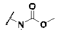 | SO₂CH₃ | 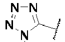 | H | H | H | H | H | |
| 1-72 | Cl | 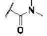 | SO₂CH₃ | 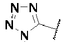 | H | H | H | H | H | |
| 1-73 | Cl | 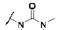 | SO₂CH₃ | 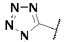 | H | H | H | H | H | |
| 1-74 | Cl | 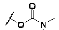 | SO₂CH₃ | 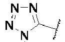 | H | H | H | H | H | |
| 1-75 | Cl | 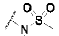 | SO₂CH₃ | 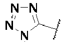 | H | H | H | H | H | |
| 1-76 | Cl | 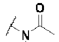 | SO₂CH₃ | 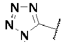 | H | H | H | H | H | |
| 1-77 | Cl | 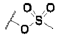 | SO₂CH₃ | 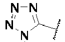 | H | H | H | H | H | |
| 1-78 | Cl | 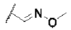 | SO₂CH₃ | 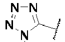 | H | H | H | H | H | |
| 1-79 | Cl | 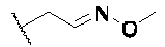 | SO₂CH₃ | 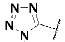 | H | H | H | H | H | |
| 1-80 | Cl | 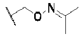 | SO₂CH₃ | 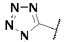 | H | H | H | H | H | |
| 1-81 | Cl | 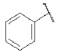 | SO₂CH₃ | 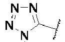 | H | H | H | H | H | |
| 1-82 | Cl | CN | SO₂CH₃ | 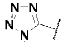 | H | H | H | H | H | |
| 1-83 | Cl | NO₂ | SO₂CH₃ | 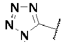 | H | H | H | H | H | |
| 1-84 | Cl | 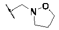 | SO₂CH₃ | 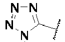 | H | H | H | H | H | |
| 1-85 | Cl | 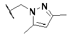 | SO₂CH₃ | 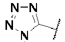 | H | H | H | H | H | |
| 1-86 | Cl | 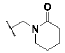 | SO₂CH₃ | 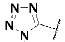 | H | H | H | H | H | |
| 1-87 | Cl | 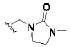 | SO₂CH₃ | 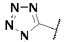 | H | H | H | H | H | |
| 1-88 | Cl | 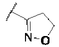 | SO₂CH₃ | 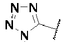 | H | H | H | H | H | |
| 1-89 | Cl | 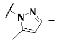 | SO₂CH₃ | 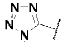 | H | H | H | H | H | |
| 1-90 | Cl | Cl | SO₂CH₃ | 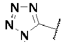 | H | H | H | H | H | yellow solid (198-200) |
| 1-91 | CH₃ | CH₃ | SO₂CH₃ | 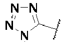 | H | H | H | H | H | white solid (202-204 ) |
| 1-92 | CH₃ | CH₂Br | SO₂CH₃ | 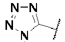 | H | H | H | H | H | |
| 1-93 | CH₃ | F | SO₂CH₃ | 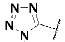 | H | H | H | H | H | |
| 1-94 | CH₃ | Br | SO₂CH₃ | 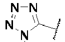 | H | H | H | H | H | |
| 1-95 | CH₃ | 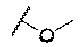 | SO₂CH₃ | 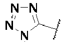 | H | H | H | H | H | white solid (206-208) |
| 1-96 | CH₃ | 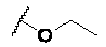 | SO₂CH₃ | 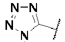 | H | H | H | H | H | off-white solid (205-207) |
| 1-97 | CH₃ | 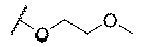 | SO₂CH₃ | 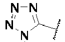 | H | H | H | H | H | white solid (171-173) |
| 1-98 | CH₃ | 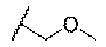 | SO₂CH₃ | 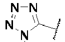 | H | H | H | H | H | white solid (202-204) |
| 1-99 | CH₃ | 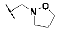 | SO₂CH₃ | 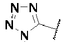 | H | H | H | H | H | |
| 1-100 | CH₃ | 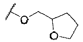 | SO₂CH₃ | 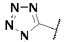 | H | H | H | H | H | white solid (185-187) |
| 1-101 | CH₃ | 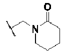 | SO₂CH₃ | 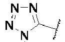 | H | H | H | H | H | |
| 1-102 | CH₃ | 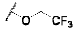 | SO₂CH₃ | 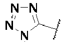 | H | H | H | H | H | yellow solid (216-218) |
| 1-103 | CH₃ | 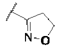 | SO₂CH₃ | 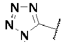 | H | H | H | H | H | |
| 1-104 | CN | 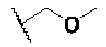 | SO₂CH₃ | 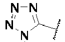 | H | H | H | H | H | |
| 1-105 | CF₃ | 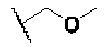 | SO₂CH₃ | 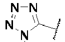 | H | H | H | H | H | |
| 1-106 | 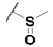 | 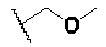 | SO₂CH₃ | 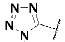 | H | H | H | H | H | |
| 1-107 | 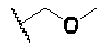 | 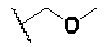 | SO₂CH₃ | 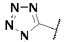 | H | H | H | H | H | |
| 1-108 | 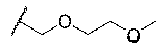 | 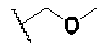 | SO₂CH₃ | 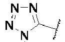 | H | H | H | H | H | |
| 1-109 | 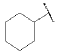 | 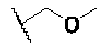 | SO₂CH₃ | 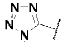 | H | H | H | H | H | |
| 1-110 | 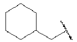 | 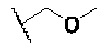 | SO₂CH₃ | 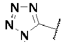 | H | H | H | H | H | |
| 1-111 | 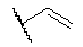 | 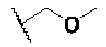 | SO₂CH₃ | 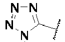 | H | H | H | H | H | |
| 1-112 | 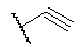 | 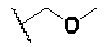 | SO₂CH₃ | 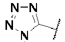 | H | H | H | H | H | |
| 1-113 | SO₂CH=CH₂ | 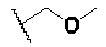 | SO₂CH₃ | 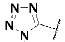 | H | H | H | H | H | |
| 1-114 | 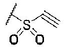 | 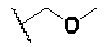 | SO₂CH₃ | 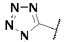 | H | H | H | H | H | |
| 1-115 | 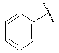 | 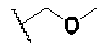 | SO₂CH₃ | 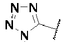 | H | H | H | H | H | |
| 1-116 | 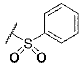 | 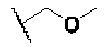 | SO₂CH₃ | 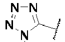 | H | H | H | H | H | |
| 1-117 | 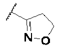 | 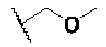 | SO₂CH₃ | 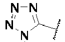 | H | H | H | H | H | |
| 1-118 | 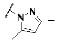 | 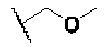 | SO₂CH₃ | 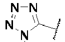 | H | H | H | H | H | |
| 1-119 | 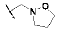 | 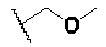 | SO₂CH₃ | 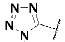 | H | H | H | H | H | |
| 1-120 | 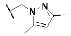 | 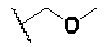 | SO₂CH₃ | 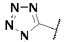 | H | H | H | H | H | |
| 1-121 | SO₂CH₃ | H | CF₃ | 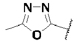 | H | H | H | H | H | |
| 1-122 | NO₂ | H | SO₂CH₃ | 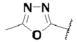 | H | H | H | H | H | |
| 1-123 | Cl | H | Cl | 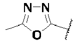 | H | H | H | H | H | |
| 1-124 | Cl | H | SO₂CH₃ | 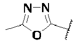 | H | H | H | H | H | |
| 1-125 | Cl | CH₃ | SO₂CH₃ | 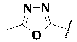 | H | H | H | H | H | |
| 1-126 | Cl | 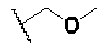 | SO₂CH₃ | 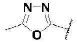 | H | H | H | H | H | |
| 1-127 | Cl | 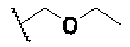 | SO₂CH₃ | 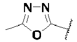 | H | H | H | H | H | |
| 1-128 | Cl | 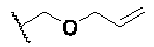 | SO₂CH₃ | 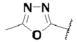 | H | H | H | H | H | |
| 1-129 | Cl | 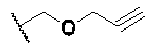 | SO₂CH₃ | 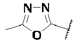 | H | H | H | H | H | |
| 1-130 | Cl | 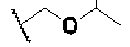 | SO₂CH₃ | 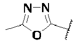 | H | H | H | H | H | |
| 1-131 | Cl | 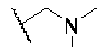 | SO₂CH₃ | 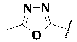 | H | H | H | H | H | |
| 1-132 | Cl | 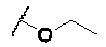 | SO₂CH₃ | 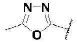 | H | H | H | H | H | |
| 1-133 | Cl | 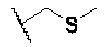 | SO₂CH₃ | 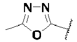 | H | H | H | H | H | |
| 1-134 | Cl | 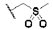 | SO₂CH₃ | 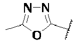 | H | H | H | H | H | |
| 1-135 | Cl | 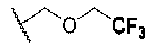 | SO₂CH₃ | 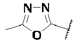 | H | H | H | H | H | |
| 1-136 | Cl | 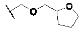 | SO₂CH₃ | 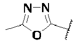 | H | H | H | H | H | |
| 1-137 | Cl | 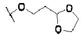 | SO₂CH₃ | 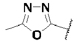 | H | H | H | H | H | |
| 1-138 | Cl | 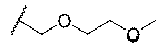 | SO₂CH₃ | 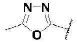 | H | H | H | H | H | |
| 1-139 | Cl | 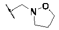 | SO₂CH₃ | 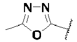 | H | H | H | H | H | |
| 1-140 | Cl | 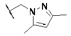 | SO₂CH₃ | 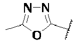 | H | H | H | H | H | |
| 1-141 | Cl | 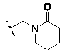 | SO₂CH₃ | 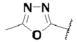 | H | H | H | H | H | |
| 1-142 | Cl | 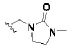 | SO₂CH₃ | 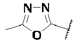 | H | H | H | H | H | |
| 1-143 | Cl | Cl | SO₂CH₃ | 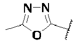 | H | H | H | H | H | |
| 1-144 | CH₃ | F | SO₂CH₃ | 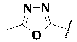 | H | H | H | H | H | |
| 1-145 | CH₃ | Br | SO₂CH₃ | 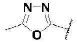 | H | H | H | H | H | |
| 1-146 | CH₃ | 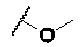 | SO₂CH₃ | 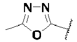 | H | H | H | H | H | - |
| 1-147 | CH₃ | 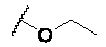 | SO₂CH₃ | 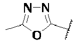 | H | H | H | H | H | |
| 1-148 | CH₃ | 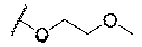 | SO₂CH₃ | 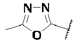 | H | H | H | H | H | |
| 1-149 | CH₃ | 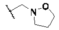 | SO₂CH₃ | 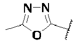 | H | H | H | H | H | |
| 1-150 | CH₃ | 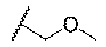 | SO₂CH₃ | 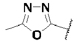 | H | H | H | H | H | |
| 1-151 | CH₃ | 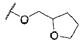 | SO₂CH₃ | 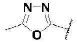 | H | H | H | H | H | |
| 1-152 | CH₃ | 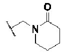 | SO₂CH₃ | 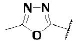 | H | H | H | H | H | |
| 1-153 | CH₃ | 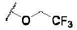 | SO₂CH₃ | 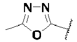 | H | H | H | H | H | |
| 1-154 | SO₂CH₃ | H | CF₃ | 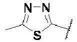 | H | H | H | H | H | yellow solid (180-182) |
| 1-155 | NO₂ | H | SO₂CH₃ | 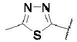 | H | H | H | H | H | |
| 1-156 | Cl | H | Cl | 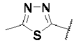 | H | H | H | H | H | |
| 1-157 | Cl | H | SO₂CH₃ | 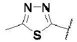 | H | H | H | H | H | |
| 1-158 | Cl | CH₃ | SO₂CH₃ | 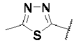 | H | H | H | H | H | |
| 1-159 | Cl | 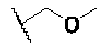 | SO₂CH₃ | 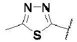 | H | H | H | H | H | |
| 1-160 | Cl | 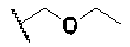 | SO₂CH₃ | 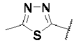 | H | H | H | H | H | |
| 1-161 | Cl | 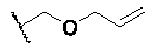 | SO₂CH₃ | 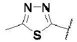 | H | H | H | H | H | |
| 1-162 | Cl | 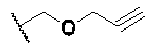 | SO₂CH₃ | 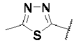 | H | H | H | H | H | |
| 1-163 | Cl | 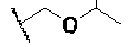 | SO₂CH₃ | 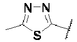 | H | H | H | H | H | |
| 1-164 | Cl | 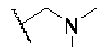 | SO₂CH₃ | 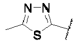 | H | H | H | H | H | |
| 1-165 | Cl | 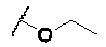 | SO₂CH₃ | 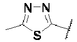 | H | H | H | H | H | |
| 1-166 | Cl | 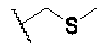 | SO₂CH₃ | 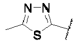 | H | H | H | H | H | |
| 1-167 | Cl | 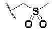 | SO₂CH₃ | 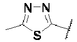 | H | H | H | H | H | |
| 1-168 | Cl | 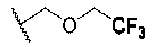 | SO₂CH₃ | 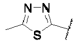 | H | H | H | H | H | |
| 1-169 | Cl | 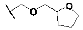 | SO₂CH₃ | 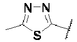 | H | H | H | H | H | |
| 1-170 | Cl | 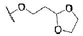 | SO₂CH₃ | 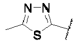 | H | H | H | H | H | |
| 1-171 | Cl | 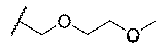 | SO₂CH₃ | 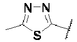 | H | H | H | H | H | |
| 1-172 | Cl | 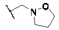 | SO₂CH₃ | 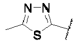 | H | H | H | H | H | |
| 1-173 | Cl | 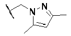 | SO₂CH₃ | 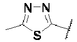 | H | H | H | H | H | |
| 1-174 | Cl | 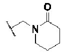 | SO₂CH₃ | 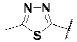 | H | H | H | H | H | |
| 1-175 | Cl | 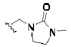 | SO₂CH₃ | 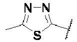 | H | H | H | H | H | |
| 1-176 | Cl | Cl | SO₂CH₃ | 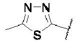 | H | H | H | H | H | |
| 1-177 | CH₃ | F | SO₂CH₃ | 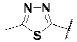 | H | H | H | H | H | |
| 1-178 | CH₃ | Br | SO₂CH₃ | 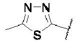 | H | H | H | H | H | |
| 1-179 | CH₃ | 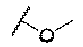 | SO₂CH₃ | 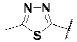 | H | H | H | H | H | |
| 1-180 | CH₃ | 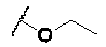 | SO₂CH₃ | 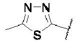 | H | H | H | H | H | |
| 1-181 | CH₃ | 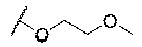 | SO₂CH₃ | 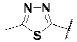 | H | H | H | H | H | |
| 1-182 | CH₃ | 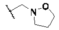 | SO₂CH₃ | 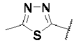 | H | H | H | H | H | |
| 1-183 | CH₃ | 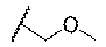 | SO₂CH₃ | 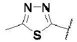 | H | H | H | H | H | |
| 1-184 | CH₃ | 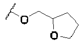 | SO₂CH₃ | 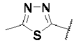 | H | H | H | H | H | |
| 1-185 | CH₃ | 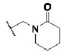 | SO₂CH₃ | 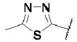 | H | H | H | H | H | |
| 1-186 | CH₃ | 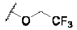 | SO₂CH₃ | 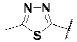 | H | H | H | H | H | |
| 1-187 | SO₂CH₃ | H | CF₃ | 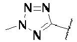 | H | H | H | H | H | off-white solid (100-102) |
| 1-188 | NO₂ | H | SO₂CH₃ | 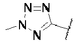 | H | H | H | H | H | |
| 1-189 | Cl | H | Cl | 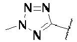 | H | H | H | H | H | |
| 1-190 | Cl | H | SO₂CH₃ | 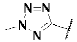 | H | H | H | H | H | |
| 1-191 | Cl | CH₃ | SO₂CH₃ | 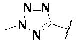 | H | H | H | H | H | |
| 1-192 | Cl | 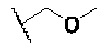 | SO₂CH₃ | 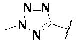 | H | H | H | H | H | |
| 1-193 | Cl | 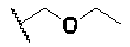 | SO₂CH₃ | 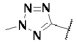 | H | H | H | H | H | |
| 1-194 | Cl | 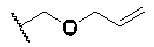 | SO₂CH₃ | 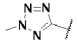 | H | H | H | H | H | |
| 1-195 | Cl | 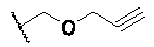 | SO₂CH₃ | 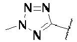 | H | H | H | H | H | |
| 1-196 | Cl | 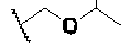 | SO₂CH₃ | 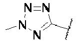 | H | H | H | H | H | |
| 1-197 | Cl | 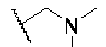 | SO₂CH₃ | 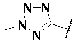 | H | H | H | H | H | |
| 1-198 | Cl | 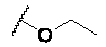 | SO₂CH₃ | 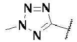 | H | H | H | H | H | |
| 1-199 | Cl | 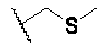 | SO₂CH₃ | 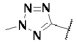 | H | H | H | H | H | |
| 1-200 | Cl | 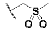 | SO₂CH₃ | 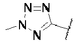 | H | H | H | H | H | |
| 1-201 | Cl | 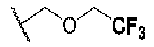 | SO₂CH₃ | 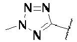 | H | H | H | H | H | |
| 1-202 | Cl | 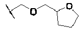 | SO₂CH₃ | 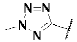 | H | H | H | H | H | |
| 1-203 | Cl | 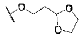 | SO₂CH₃ | 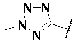 | H | H | H | H | H | |
| 1-204 | Cl | 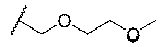 | SO₂CH₃ | 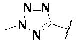 | H | H | H | H | H | |
| 1-205 | Cl | 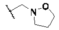 | SO₂CH₃ | 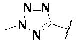 | H | H | H | H | H | |
| 1-206 | Cl | 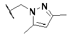 | SO₂CH₃ | 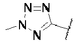 | H | H | H | H | H | |
| 1-207 | Cl | 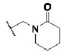 | SO₂CH₃ | 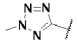 | H | H | H | H | H | |
| 1-208 | Cl | 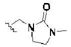 | SO₂CH₃ | 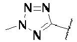 | H | H | H | H | H | |
| 1-209 | Cl | Cl | SO₂CH₃ | 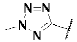 | H | H | H | H | H | |
| 1-210 | CH₃ | F | SO₂CH₃ | 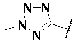 | H | H | H | H | H | |
| 1-211 | CH₃ | Br | SO₂CH₃ | 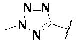 | H | H | H | H | H | |
| 1-212 | CH₃ | 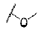 | SO₂CH₃ | 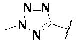 | H | H | H | H | H | |
| 1-213 | CH₃ | 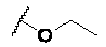 | SO₂CH₃ | 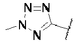 | H | H | H | H | H | |
| 1-214 | CH₃ | 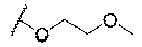 | SO₂CH₃ | 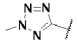 | H | H | H | H | H | |
| 1-215 | CH₃ | 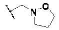 | SO₂CH₃ | 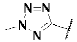 | H | H | H | H | H | |
| 1-216 | CH₃ | 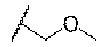 | SO₂CH₃ | 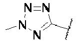 | H | H | H | H | H | |
| 1-217 | CH₃ | 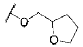 | SO₂CH₃ | 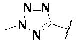 | H | H | H | H | H | |
| 1-218 | CH₃ | 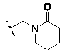 | SO₂CH₃ | 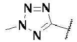 | H | H | H | H | H | |
| 1-219 | CH₃ | 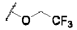 | SO₂CH₃ | 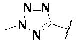 | H | H | H | H | H | |
| 1-220 | SO₂CH₃ | H | CF₃ | 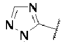 | H | H | H | H | H | |
| 1-221 | NO₂ | H | SO₂CH₃ | 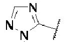 | H | H | H | H | H | |
| 1-222 | Cl | H | Cl | 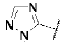 | H | H | H | H | H | |
| 1-223 | Cl | H | SO₂CH₃ | 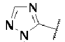 | H | H | H | H | H | |
| 1-224 | Cl | CH₃ | SO₂CH₃ | 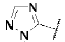 | H | H | H | H | H | |
| 1-225 | Cl | 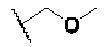 | SO₂CH₃ | 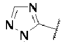 | H | H | H | H | H | |
| 1-226 | Cl | 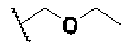 | SO₂CH₃ | 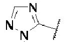 | H | H | H | H | H | |
| 1-227 | Cl | 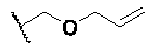 | SO₂CH₃ | 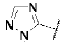 | H | H | H | H | H | |
| 1-228 | Cl | 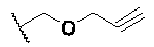 | SO₂CH₃ | 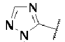 | H | H | H | H | H | |
| 1-229 | Cl | 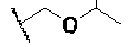 | SO₂CH₃ | 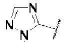 | H | H | H | H | H | |
| 1-230 | Cl | 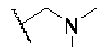 | SO₂CH₃ | 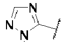 | H | H | H | H | H | |
| 1-231 | Cl | 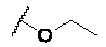 | SO₂CH₃ | 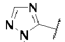 | H | H | H | H | H | |
| 1-232 | Cl | 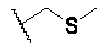 | SO₂CH₃ | 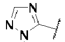 | H | H | H | H | H | |
| 1-233 | Cl | 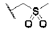 | SO₂CH₃ | 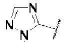 | H | H | H | H | H | |
| 1-234 | Cl | 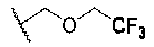 | SO₂CH₃ | 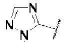 | H | H | H | H | H | |
| 1-235 | Cl | 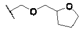 | SO₂CH₃ | 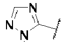 | H | H | H | H | H | |
| 1-236 | Cl | 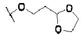 | SO₂CH₃ | 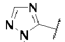 | H | H | H | H | H | |
| 1-237 | Cl | 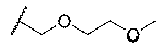 | SO₂CH₃ | 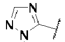 | H | H | H | H | H | |
| 1-238 | Cl | 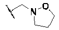 | SO₂CH₃ | 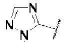 | H | H | H | H | H | |
| 1-239 | Cl | 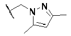 | SO₂CH₃ | 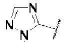 | H | H | H | H | H | |
| 1-240 | Cl | 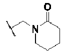 | SO₂CH₃ | 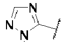 | H | H | H | H | H | |
| 1-241 | Cl | 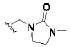 | SO₂CH₃ | 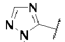 | H | H | H | H | H | |
| 1-242 | Cl | Cl | SO₂CH₃ | 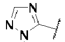 | H | H | H | H | H | |
| 1-243 | CH₃ | F | SO₂CH₃ | 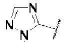 | H | H | H | H | H | |
| 1-244 | CH₃ | Br | SO₂CH₃ | 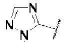 | H | H | H | H | H | |
| 1-245 | CH₃ | 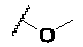 | SO₂CH₃ | 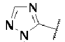 | H | H | H | H | H | |
| 1-246 | CH₃ | 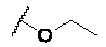 | SO₂CH₃ | 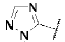 | H | H | H | H | H | |
| 1-247 | CH₃ | 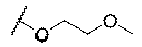 | SO₂CH₃ | 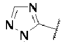 | H | H | H | H | H | |
| 1-248 | CH₃ | 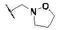 | SO₂CH₃ | 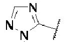 | H | H | H | H | H | |
| 1-249 | CH₃ | 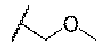 | SO₂CH₃ | 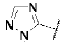 | H | H | H | H | H | |
| 1-250 | CH₃ | 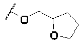 | SO₂CH₃ | 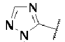 | H | H | H | H | H | |
| 1-251 | CH₃ | 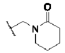 | SO₂CH₃ | 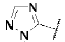 | H | H | H | H | H | |
| 1-252 | CH₃ | 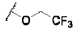 | SO₂CH₃ | 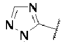 | H | H | H | H | H | |
| 1-253 | SO₂CH₃ | H | CF₃ | 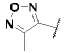 | H | H | H | H | H | |
| 1-254 | NO₂ | H | SO₂CH₃ | 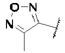 | H | H | H | H | H | |
| 1-255 | Cl | H | Cl | 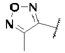 | H | H | H | H | H | |
| 1-256 | Cl | H | SO₂CH₃ | 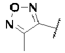 | H | H | H | H | H | |
| 1-257 | Cl | CH₃ | SO₂CH₃ | 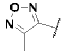 | H | H | H | H | H | |
| 1-258 | Cl | 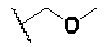 | SO₂CH₃ | 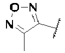 | H | H | H | H | H | |
| 1-259 | Cl | 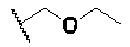 | SO₂CH₃ | 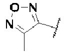 | H | H | H | H | H | |
| 1-260 | Cl | 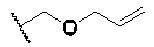 | SO₂CH₃ | 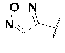 | H | H | H | H | H | |
| 1-261 | Cl | 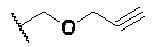 | SO₂CH₃ | 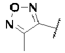 | H | H | H | H | H | |
| 1-262 | Cl | 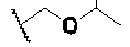 | SO₂CH₃ | 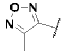 | H | H | H | H | H | |
| 1-263 | Cl | 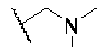 | SO₂CH₃ | 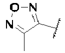 | H | H | H | H | H | |
| 1-264 | Cl | 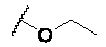 | SO₂CH₃ | 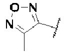 | H | H | H | H | H | |
| 1-265 | Cl | 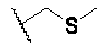 | SO₂CH₃ | 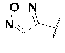 | H | H | H | H | H | |
| 1-266 | Cl | 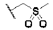 | SO₂CH₃ | 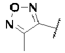 | H | H | H | H | H | |
| 1-267 | Cl | 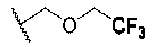 | SO₂CH₃ | 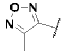 | H | H | H | H | H | |
| 1-268 | Cl | 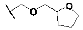 | SO₂CH₃ | 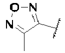 | H | H | H | H | H | |
| 1-269 | Cl | 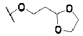 | SO₂CH₃ | 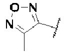 | H | H | H | H | H | |
| 1-270 | Cl | 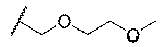 | SO₂CH₃ | 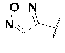 | H | H | H | H | H | |
| 1-271 | Cl | 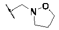 | SO₂CH₃ | 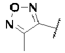 | H | H | H | H | H | |
| 1-272 | Cl | 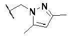 | SO₂CH₃ | 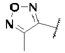 | H | H | H | H | H | |
| 1-273 | Cl | 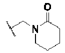 | SO₂CH₃ | 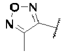 | H | H | H | H | H | |
| 1-274 | Cl | 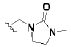 | SO₂CH₃ | 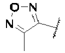 | H | H | H | H | H | |
| 1-275 | Cl | Cl | SO₂CH₃ | 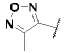 | H | H | H | H | H | |
| 1-276 | CH₃ | F | SO₂CH₃ | 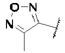 | H | H | H | H | H | |
| 1-277 | CH₃ | Br | SO₂CH₃ | 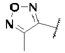 | H | H | H | H | H | |
| 1-278 | CH₃ | 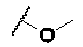 | SO₂CH₃ | 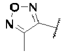 | H | H | H | H | H | |
| 1-279 | CH₃ | 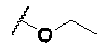 | SO₂CH₃ | 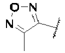 | H | H | H | H | H | |
| 1-280 | CH₃ | 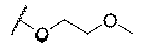 | SO₂CH₃ | 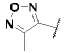 | H | H | H | H | H | |
| 1-281 | CH₃ | 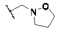 | SO₂CH₃ | 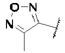 | H | H | H | H | H | |
| 1-282 | CH₃ | 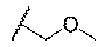 | SO₂CH₃ | 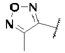 | H | H | H | H | H | |
| 1-283 | CH₃ | 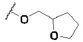 | SO₂CH₃ | 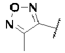 | H | H | H | H | H | |
| 1-284 | CH₃ | 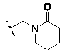 | SO₂CH₃ | 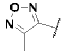 | H | H | H | H | H | |
| 1-285 | CH₃ | 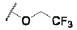 | SO₂CH₃ | 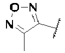 | H | H | H | H | H | |
| 1-286 | Cl | 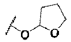 | SO₂CH₃ | 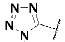 | H | H | H | H | H | |
| 1-287 | Cl | 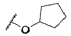 | SO₂CH₃ | 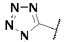 | H | H | H | H | H | |
| 1-288 | Cl | 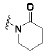 | SO₂CH₃ | 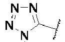 | H | H | H | H | H | |
| 1-289 | Cl | SO₂CH₃ | CF₃ | 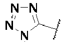 | H | H | H | H | H | |
| 1-290 | Cl | 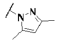 | SO₂CH₃ | 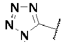 | H | H | H | H | H | |
| 1-291 | CH₃ | 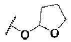 | SO₂CH₃ | 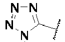 | H | H | H | H | H | |
| 1-292 | CH₃ | 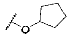 | SO₂CH₃ | 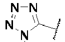 | H | H | H | H | H | |
| 1-293 | CH₃ | 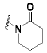 | SO₂CH₃ | 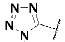 | H | H | H | H | H | |
| 1-294 | CH₃ | SO₂CH₃ | CF₃ | 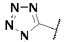 | H | H | H | H | H | |
| 1-295 | CH₃ | 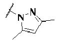 | SO₂CH₃ | 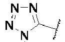 | H | H | H | H | H | |
| 1-296 | CH₃ | 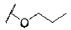 | SO₂CH₃ | 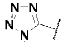 | H | H | H | H | H | orange solid (172-174) |
| 1-297 | CH₃ | 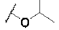 | SO₂CH₃ | 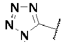 | H | H | H | H | H | yellow solid (190-192) |
| 1-298 | Cl | 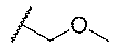 | SO₂CH₃ | 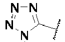 | H | H | CH₃ | H | H | off-white solid (191-193) |
| 1-299 | Cl | 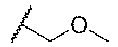 | SO₂CH₃ | 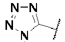 | H | H | F | H | H | off-white solid (188-190) |
| 1-300 | Cl | 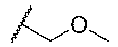 | SO₂CH₃ | 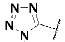 | CH₃ | H | H | H | H | off-white solid (150-152) |
| 1-301 | Cl | 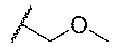 | SO₂CH₃ | 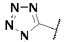 | OCH₃ | H | H | H | H | yellow solid (80-82) |
| 1-302 | CH₃ | 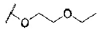 | SO₂CH₃ | 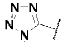 | H | H | H | H | H | pale yellow solid (115-117) |
| 1-303 | Cl | 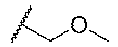 | SO₂CH₃ | 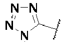 | F | H | H | H | H | off-white solid (157-161) |
| 1-304 | Cl | 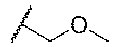 | SO₂CH₃ | 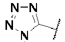 | CF₃ | H | H | H | H | off-white solid (178-182) |
| 1-305 | Cl | 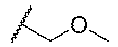 | SO₂CH₃ | 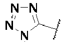 | Cl | H | H | H | H | off-white solid (165-169) |
| 1-306 | Cl | 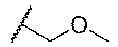 | SO₂CH₃ | 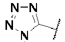 | Br | H | H | H | H | off-white solid (144-148) |
| 1-307 | Cl | 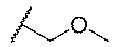 | SO₂CH₃ | 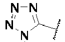 | H | CH₃ | H | H | H | off-white solid (96-100) |
| 1-308 | Cl | 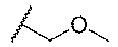 | SO₂CH₃ | 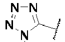 | H | Br | H | H | H | off-white solid (164-168) |
| 1-309 | Cl | 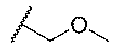 | SO₂CH₃ | 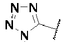 | H | Cl | H | H | H | off-white solid (186-190) |
| 1-310 | Cl | 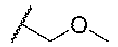 | SO₂CH₃ | 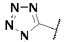 | H | CF₃ | H | H | H | off-white solid (179-182) |
| 1-311 | Cl | 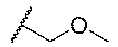 | SO₂CH₃ | 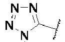 | H | F | H | H | H | off-white solid (207-212) |
| 1-312 | CH₃ | 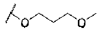 | SO₂CH₃ | 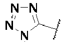 | H | H | H | H | H | white solid (169-170) |
| 1-313 | CH₃ | 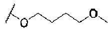 | SO₂CH₃ | 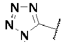 | H | H | H | H | H | white solid (176-177) |
| 1-314 | Cl | 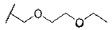 | SO₂CH₃ | 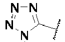 | H | H | H | H | H | yellow oil |
| 1-315 | Cl | 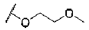 | SO₂CH₃ | 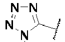 | H | H | H | H | H | white solid (210-212) |
| 1-316 | Cl | 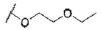 | SO₂CH₃ | 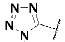 | H | H | H | H | H | yellow oil |
| 1-317 | Cl | 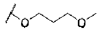 | SO₂CH₃ | 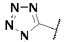 | H | H | H | H | H | yellow oil |
| 1-318 | Cl | 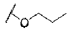 | SO₂CH₃ | 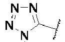 | H | H | H | H | H | pale yellow oil |
| 1-319 | Cl | 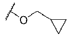 | SO₂CH₃ | 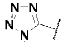 | H | H | H | H | H | pale yellow oil |

In the compound of the formula I, W is CX₂.

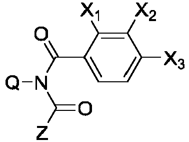

**Table 2 Structures and Physical Properties of Part of Compounds of Formula I**

| Compound | X₁ | X₂ | X₃ | Q | Z | Appearance (Melting Point °C) |
|---|---|---|---|---|---|---|
| 2-1 | CH₃ | 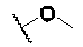 | SO₂CH₃ | 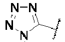 | 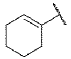 | |
| 2-2 | CH₃ | 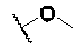 | SO₂CH₃ | 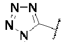 | 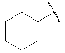 | |
| 2-3 | CH₃ | 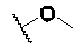 | SO₂CH₃ | 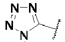 | 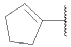 | |
| 2-4 | CH₃ | 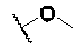 | SO₂CH₃ | 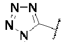 | 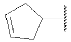 | |
| 2-5 | CH₃ | 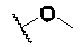 | SO₂CH₃ | 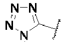 | 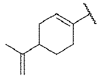 | |
| 2-6 | CH₃ | 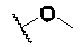 | SO₂CH₃ | 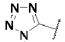 | 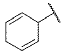 | |
| 2-7 | CH₃ | 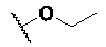 | SO₂CH₃ | 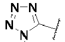 | 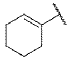 | orange solid (147-148) |
| 2-8 | CH₃ | 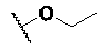 | SO₂CH₃ | 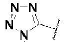 | 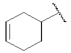 | |
| 2-9 | CH₃ | 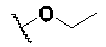 | SO₂CH₃ | 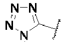 | 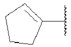 | white solid (177-178) |
| 2-10 | CH₃ | 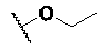 | SO₂CH₃ | 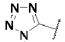 | 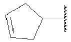 | |
| 2-11 | CH₃ | 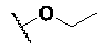 | SO₂CH₃ | 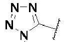 | 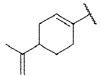 | |
| 2-12 | CH₃ | 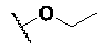 | SO₂CH₃ | 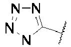 | 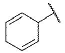 | |
| 2-13 | CH₃ | 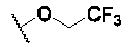 | SO₂CH₃ | 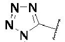 | 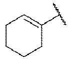 | white solid (142-143) |
| 2-14 | CH₃ | 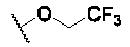 | SO₂CH₃ | 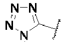 | 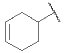 | |
| 2-15 | CH₃ | 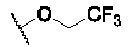 | SO₂CH₃ | 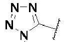 | 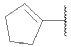 | pale yellow solid (130-131) |
| 2-16 | CH₃ | 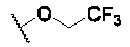 | SO₂CH₃ | 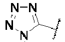 | 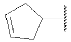 | |
| 2-17 | CH₃ | 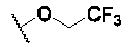 | SO₂CH₃ | 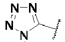 | 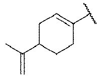 | |
| 2-18 | CH₃ | 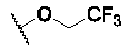 | SO₂CH₃ | 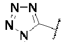 | 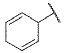 | |
| 2-19 | CH₃ | 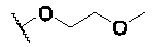 | SO₂CH₃ | 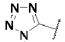 | 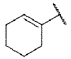 | yellow solid (60-62) |
| 2-20 | CH₃ | 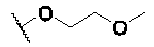 | SO₂CH₃ | 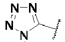 | 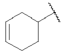 | |
| 2-21 | CH₃ | 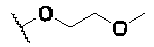 | SO₂CH₃ | 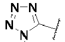 | 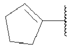 | yellow oil |
| 2-22 | CH₃ | 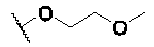 | SO₂CH₃ | 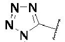 | 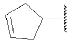 | |
| 2-23 | CH₃ | 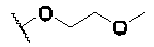 | SO₂CH₃ | 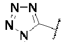 | 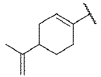 | |
| 2-24 | CH₃ | 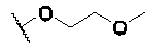 | SO₂CH₃ | 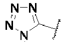 | 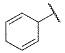 | |
| 2-25 | CH₃ | 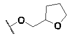 | SO₂CH₃ | 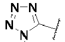 | 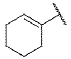 | |
| 2-26 | CH₃ | 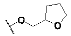 | SO₂CH₃ | 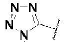 | 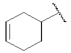 | |
| 2-27 | CH₃ | 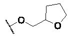 | SO₂CH₃ | 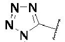 | 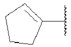 | |
| 2-28 | CH₃ | 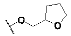 | SO₂CH₃ | 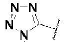 | 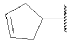 | |
| 2-29 | CH₃ | 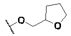 | SO₂CH₃ | 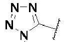 | 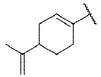 | |
| 2-30 | CH₃ | 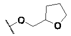 | SO₂CH₃ | 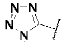 | 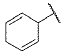 | |
| 2-31 | CH₃ | 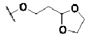 | SO₂CH₃ | 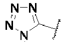 | 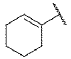 | |
| 2-32 | CH₃ | 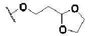 | SO₂CH₃ | 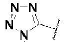 | 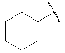 | |
| 2-33 | CH₃ | 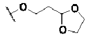 | SO₂CH₃ | 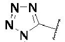 | 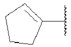 | |
| 2-34 | CH₃ | 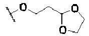 | SO₂CH₃ | 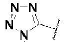 | 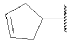 | |
| 2-35 | CH₃ | 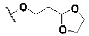 | SO₂CH₃ | 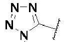 | 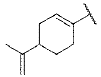 | |
| 2-36 | CH₃ | 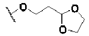 | SO₂CH₃ | 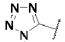 | 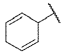 | |
| 2-37 | CH₃ | 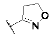 | SO₂CH₃ | 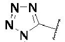 | 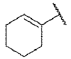 | yellow oil |
| 2-38 | CH₃ | 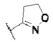 | SO₂CH₃ | 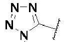 | 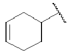 | |
| 2-39 | CH₃ | 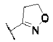 | SO₂CH₃ | 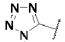 | 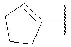 | |
| 2-40 | CH₃ | 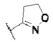 | SO₂CH₃ | 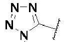 | 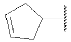 | |
| 2-41 | CH₃ | 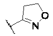 | SO₂CH₃ | 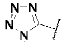 | 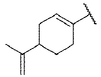 | |
| 2-42 | CH₃ | 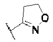 | SO₂CH₃ | 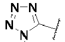 | 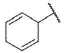 | |
| 2-43 | CH₃ | CH₃ | SO₂CH₃ | 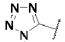 | 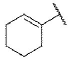 | white solid (91-93) |
| 2-44 | CH₃ | CH₃ | SO₂CH₃ | 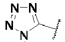 | 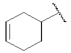 | |
| 2-45 | CH₃ | CH₃ | SO₂CH₃ | 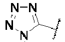 | 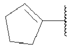 | |
| 2-46 | CH₃ | CH₃ | SO₂CH₃ | 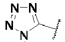 | 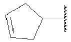 | |
| 2-47 | CH₃ | CH₃ | SO₂CH₃ | 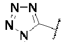 | 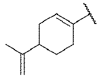 | |
| 2-48 | CH₃ | CH₃ | SO₂CH₃ | 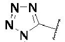 | 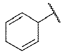 | |
| 2-49 | CH₃ | 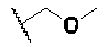 | SO₂CH₃ | 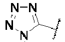 | 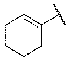 | |
| 2-50 | CH₃ | 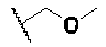 | SO₂CH₃ | 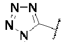 | 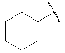 | |
| 2-51 | CH₃ | 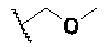 | SO₂CH₃ | 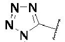 | 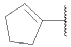 | |
| 2-52 | CH₃ | 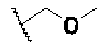 | SO₂CH₃ | 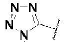 | 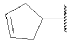 | |
| 2-53 | CH₃ | 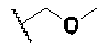 | SO₂CH₃ | 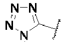 | 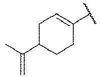 | |
| 2-54 | CH₃ | 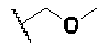 | SO₂CH₃ | 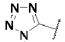 | 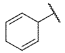 | |
| 2-55 | Cl | 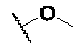 | SO₂CH₃ | 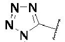 | 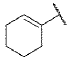 | pale yellow oil |
| 2-56 | Cl | 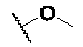 | SO₂CH₃ | 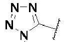 | 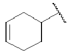 | |
| 2-57 | Cl | 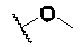 | SO₂CH₃ | 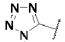 | 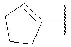 | |
| 2-58 | Cl | 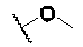 | SO₂CH₃ | 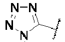 | 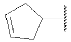 | |
| 2-59 | Cl | 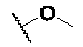 | SO₂CH₃ | 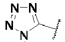 | 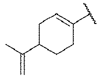 | |
| 2-60 | Cl | 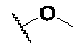 | SO₂CH₃ | 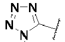 | 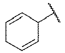 | |
| 2-61 | Cl | 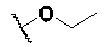 | SO₂CH₃ | 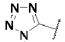 | 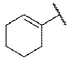 | pale yellow oil |
| 2-62 | Cl | 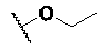 | SO₂CH₃ | 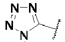 | 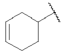 | |
| 2-63 | Cl | 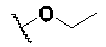 | SO₂CH₃ | 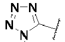 | 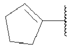 | |
| 2-64 | Cl | 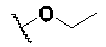 | SO₂CH₃ | 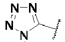 | 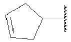 | |
| 2-65 | Cl | 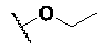 | SO₂CH₃ | 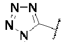 | 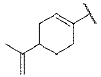 | |
| 2-66 | Cl | 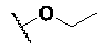 | SO₂CH₃ | 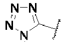 | 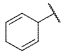 | |
| 2-67 | Cl | 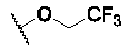 | SO₂CH₃ | 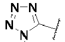 | 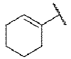 | |
| 2-68 | Cl | 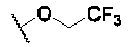 | SO₂CH₃ | 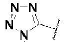 | 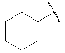 | |
| 2-69 | Cl | 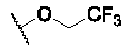 | SO₂CH₃ | 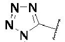 | 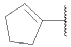 | |
| 2-70 | Cl | 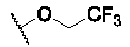 | SO₂CH₃ | 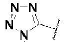 | 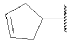 | |
| 2-71 | Cl | 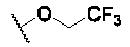 | SO₂CH₃ | 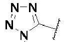 | 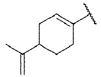 | |
| 2-72 | Cl | 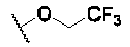 | SO₂CH₃ | 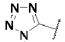 | 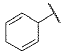 | |
| 2-73 | Cl | 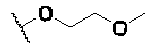 | SO₂CH₃ | 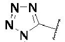 | 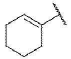 | |
| 2-74 | Cl | 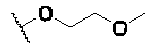 | SO₂CH₃ | 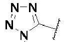 | 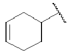 | |
| 2-75 | Cl | 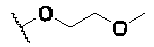 | SO₂CH₃ | 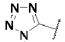 | 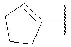 | |
| 2-76 | Cl | 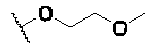 | SO₂CH₃ | 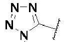 | 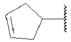 | |
| 2-77 | Cl | 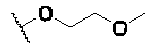 | SO₂CH₃ | 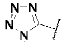 | 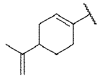 | |
| 2-78 | Cl | 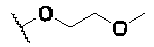 | SO₂CH₃ | 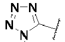 | 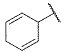 | |
| 2-79 | Cl | 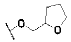 | SO₂CH₃ | 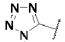 | 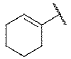 | |
| 2-80 | Cl | 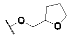 | SO₂CH₃ | 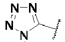 | 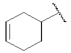 | |
| 2-81 | Cl | 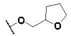 | SO₂CH₃ | 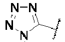 | 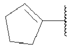 | |
| 2-82 | Cl | 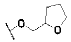 | SO₂CH₃ | 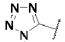 | 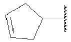 | |
| 2-83 | Cl | 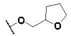 | SO₂CH₃ | 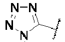 | 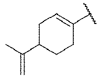 | |
| 2-84 | Cl | 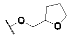 | SO₂CH₃ | 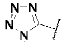 | 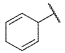 | |
| 2-85 | Cl | 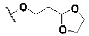 | SO₂CH₃ | 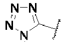 | 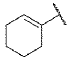 | |
| 2-86 | Cl | 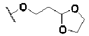 | SO₂CH₃ | 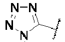 | 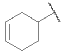 | |
| 2-87 | Cl | 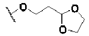 | SO₂CH₃ | 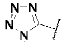 | 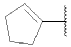 | |
| 2-88 | Cl | 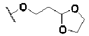 | SO₂CH₃ | 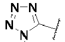 | 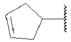 | |
| 2-89 | Cl | 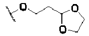 | SO₂CH₃ | 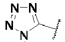 | 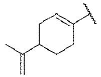 | |
| 2-90 | Cl | 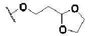 | SO₂CH₃ | 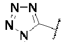 | 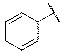 | |
| 2-91 | Cl | 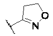 | SO₂CH₃ | 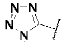 | 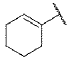 | |
| 2-92 | Cl | 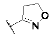 | SO₂CH₃ | 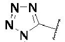 | 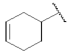 | |
| 2-93 | Cl | 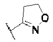 | SO₂CH₃ | 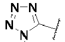 | 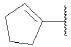 | |
| 2-94 | Cl | 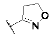 | SO₂CH₃ | 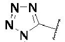 | 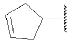 | |
| 2-95 | Cl | 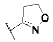 | SO₂CH₃ | 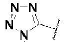 | 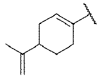 | |
| 2-96 | Cl | 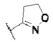 | SO₂CH₃ | 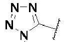 | 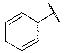 | |
| 2-97 | Cl | CH₃ | SO₂CH₃ | 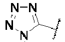 | 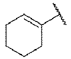 | pale yellow solid (90-92) |
| 2-98 | Cl | CH₃ | SO₂CH₃ | 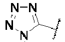 | 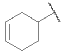 | |
| 2-99 | Cl | CH₃ | SO₂CH₃ | 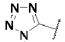 | 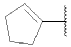 | |
| 2-100 | Cl | CH₃ | SO₂CH₃ | 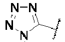 | 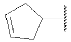 | |
| 2-101 | Cl | CH₃ | SO₂CH₃ | 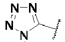 | 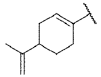 | |
| 2-102 | Cl | CH₃ | SO₂CH₃ | 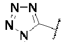 | 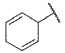 | |
| 2-103 | Cl | 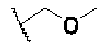 | SO₂CH₃ | 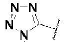 | 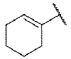 | pale yellow solid (155-157) |
| 2-104 | Cl | 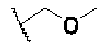 | SO₂CH₃ | 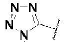 | 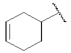 | off-white solid (125-127) |
| 2-105 | Cl | 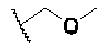 | SO₂CH₃ | 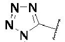 | 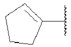 | pale yellow oil |
| 2-106 | Cl | 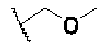 | SO₂CH₃ | 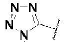 | 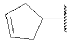 | brown solid (135-137) |
| 2-107 | Cl | 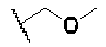 | SO₂CH₃ | 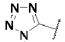 | 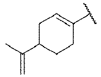 | |
| 2-108 | Cl | 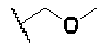 | SO₂CH₃ | 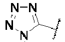 | 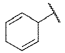 | |
| 2-109 | Cl | 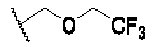 | SO₂CH₃ | 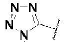 | 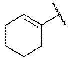 | |
| 2-110 | Cl | 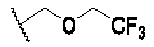 | SO₂CH₃ | 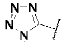 | 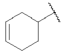 | |
| 2-111 | Cl | 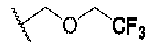 | SO₂CH₃ | 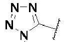 | 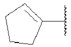 | |
| 2-112 | Cl | 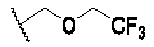 | SO₂CH₃ | 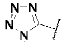 | 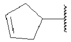 | |
| 2-113 | Cl | 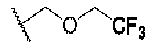 | SO₂CH₃ | 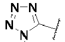 | 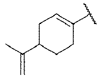 | |
| 2-114 | Cl | 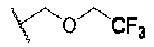 | SO₂CH₃ | 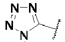 | 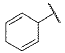 | |
| 2-115 | SO₂CH₃ | H | Cl | 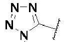 | 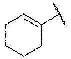 | |
| 2-116 | SO₂CH₃ | H | Cl | 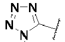 | 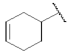 | |
| 2-117 | SO₂CH₃ | H | Cl | 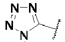 | 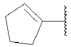 | |
| 2-118 | SO₂CH₃ | H | Cl | 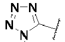 | 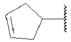 | |
| 2-119 | SO₂CH₃ | H | Cl | 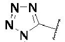 | 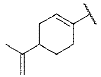 | |
| 2-120 | SO₂CH₃ | H | C1 | 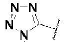 | 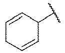 | |
| 2-121 | Cl | Cl | SO₂CH₃ | 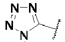 | 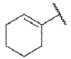 | |
| 2-122 | Cl | Cl | SO₂CH₃ | 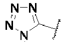 | 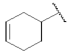 | |
| 2-123 | Cl | Cl | SO₂CH₃ | 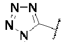 | 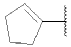 | |
| 2-124 | Cl | Cl | SO₂CH₃ | 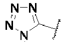 | 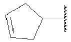 | |
| 2-125 | Cl | Cl | SO₂CH₃ | 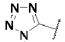 | 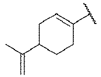 | |
| 2-126 | Cl | Cl | SO₂CH₃ | 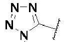 | 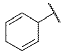 | |
| 2-127 | Cl | 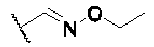 | SO₂CH₃ | 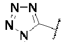 | 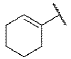 | |
| 2-128 | Cl | 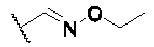 | SO₂CH₃ | 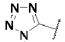 | 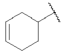 | |
| 2-129 | Cl | 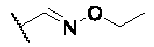 | SO₂CH₃ | 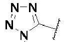 | 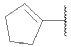 | |
| 2-130 | Cl | 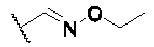 | SO₂CH₃ | 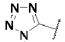 | 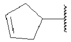 | |
| 2-131 | Cl | 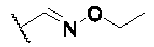 | SO₂CH₃ | 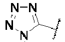 | 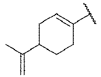 | |
| 2-132 | Cl | 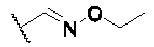 | SO₂CH₃ | 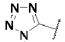 | 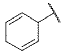 | |
| 2-133 | CH₃ | 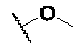 | SO₂CH₃ | 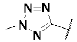 | 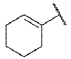 | |
| 2-134 | CH₃ | 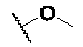 | SO₂CH₃ | 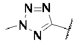 | 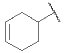 | |
| 2-135 | CH₃ | 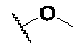 | SO₂CH₃ | 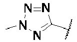 | 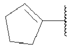 | |
| 2-136 | CH₃ | 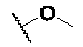 | SO₂CH₃ | 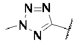 | 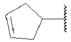 | |
| 2-137 | CH₃ | 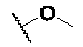 | SO₂CH₃ | 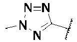 | 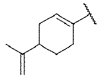 | |
| 2-138 | CH₃ | 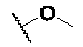 | SO₂CH₃ | 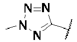 | 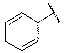 | |
| 2-139 | CH₃ | 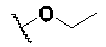 | SO₂CH₃ | 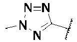 | 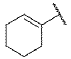 | |
| 2-140 | CH₃ | 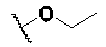 | SO₂CH₃ | 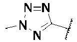 | 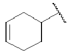 | |
| 2-141 | CH₃ | 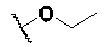 | SO₂CH₃ | 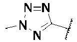 | 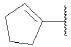 | |
| 2-142 | CH₃ | 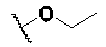 | SO₂CH₃ | 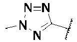 | 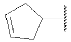 | |
| 2-143 | CH₃ | 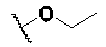 | SO₂CH₃ | 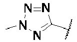 | 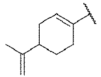 | |
| 2-144 | CH₃ | 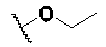 | SO₂CH₃ | 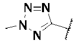 | 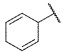 | |
| 2-145 | CH₃ | 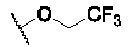 | SO₂CH₃ | 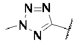 | 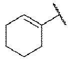 | |
| 2-146 | CH₃ | 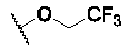 | SO₂CH₃ | 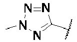 | 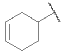 | - |
| 2-147 | CH₃ | 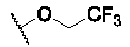 | SO₂CH₃ | 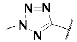 | 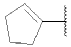 | |
| 2-148 | CH₃ | 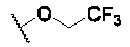 | SO₂CH₃ | 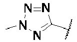 | 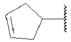 | |
| 2-149 | CH₃ | 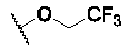 | SO₂CH₃ | 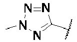 | 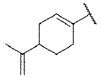 | |
| 2-150 | CH₃ | 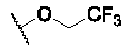 | SO₂CH₃ | 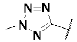 | 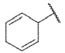 | |
| 2-151 | CH₃ | 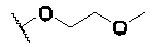 | SO₂CH₃ | 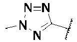 | 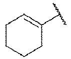 | |
| 2-152 | CH₃ | 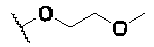 | SO₂CH₃ | 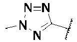 | 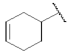 | |
| 2-153 | CH₃ | 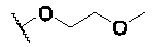 | SO₂CH₃ | 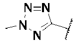 | 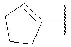 | |
| 2-154 | CH₃ | 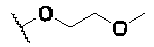 | SO₂CH₃ | 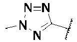 | 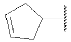 | |
| 2-155 | CH₃ | 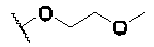 | SO₂CH₃ | 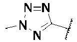 | 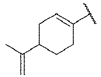 | |
| 2-156 | CH₃ | 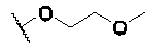 | SO₂CH₃ | 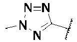 | 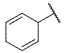 | |
| 2-157 | CH₃ | 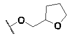 | SO₂CH₃ | 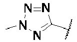 | 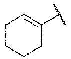 | |
| 2-158 | CH₃ | 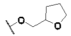 | SO₂CH₃ | 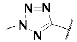 | 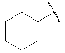 | |
| 2-159 | CH₃ | 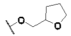 | SO₂CH₃ | 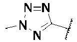 | 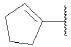 | |
| 2-160 | CH₃ | 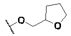 | SO₂CH₃ | 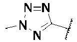 | 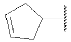 | |
| 2-161 | CH₃ | 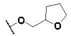 | SO₂CH₃ | 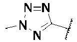 | 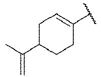 | |
| 2-162 | CH₃ | 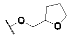 | SO₂CH₃ | 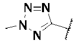 | 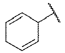 | |
| 2-163 | CH₃ | 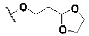 | SO₂CH₃ | 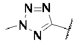 | 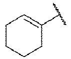 | |
| 2-164 | CH₃ | 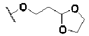 | SO₂CH₃ | 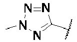 | 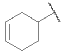 | |
| 2-165 | CH₃ | 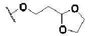 | SO₂CH₃ | 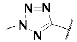 | 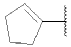 | |
| 2-166 | CH₃ | 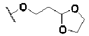 | SO₂CH₃ | 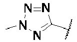 | 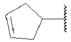 | |
| 2-167 | CH₃ | 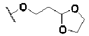 | SO₂CH₃ | 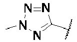 | 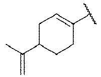 | |
| 2-168 | CH₃ | 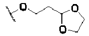 | SO₂CH₃ | 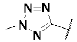 | 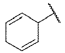 | |
| 2-169 | CH₃ | 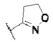 | SO₂CH₃ | 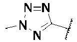 | 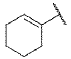 | |
| 2-170 | CH₃ | 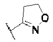 | SO₂CH₃ | 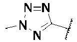 | 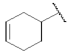 | |
| 2-171 | CH₃ | 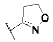 | SO₂CH₃ | 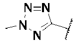 | 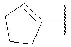 | |
| 2-172 | CH₃ | 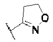 | SO₂CH₃ | 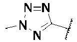 | 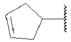 | |
| 2-173 | CH₃ | 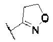 | SO₂CH₃ | 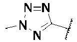 | 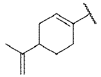 | |
| 2-174 | CH₃ | 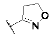 | SO₂CH₃ | 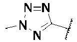 | 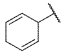 | |
| 2-175 | CH₃ | CH₃ | SO₂CH₃ | 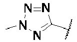 | 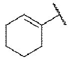 | |
| 2-176 | CH₃ | CH₃ | SO₂CH₃ | 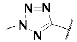 | 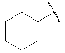 | |
| 2-177 | CH₃ | CH₃ | SO₂CH₃ | 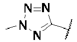 | 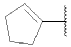 | |
| 2-178 | CH₃ | CH₃ | SO₂CH₃ | 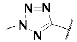 | 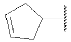 | |
| 2-179 | CH₃ | CH₃ | SO₂CH₃ | 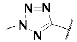 | 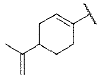 | |
| 2-180 | CH₃ | CH₃ | SO₂CH₃ | 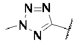 | 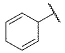 | |
| 2-181 | CH₃ | 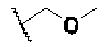 | SO₂CH₃ | 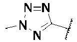 | 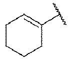 | |
| 2-182 | CH₃ | 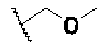 | SO₂CH₃ | 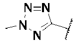 | 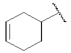 | |
| 2-183 | CH₃ | 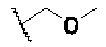 | SO₂CH₃ | 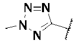 | 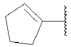 | |
| 2-184 | CH₃ | 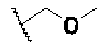 | SO₂CH₃ | 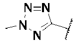 | 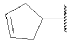 | |
| 2-185 | CH₃ | 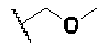 | SO₂CH₃ | 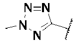 | 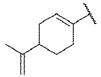 | |
| 2-186 | CH₃ | 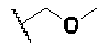 | SO₂CH₃ | 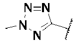 | 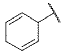 | |
| 2-187 | Cl | 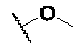 | SO₂CH₃ | 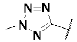 | 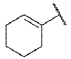 | |
| 2-188 | Cl | 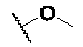 | SO₂CH₃ | 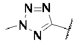 | 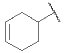 | |
| 2-189 | Cl | 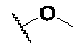 | SO₂CH₃ | 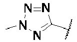 | 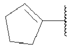 | |
| 2-190 | Cl | 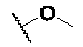 | SO₂CH₃ | 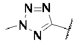 | 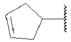 | |
| 2-191 | Cl | 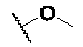 | SO₂CH₃ | 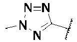 | 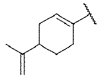 | |
| 2-192 | Cl | 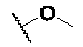 | SO₂CH₃ | 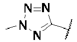 | 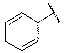 | |
| 2-193 | Cl | 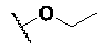 | SO₂CH₃ | 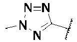 | 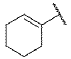 | |
| 2-194 | Cl | 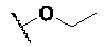 | SO₂CH₃ | 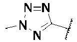 | 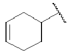 | |
| 2-195 | Cl | 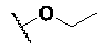 | SO₂CH₃ | 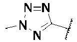 | 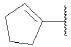 | |
| 2-196 | Cl | 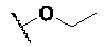 | SO₂CH₃ | 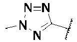 | 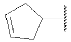 | |
| 2-197 | Cl | 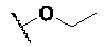 | SO₂CH₃ | 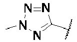 | 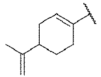 | |
| 2-198 | Cl | 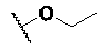 | SO₂CH₃ | 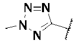 | 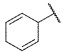 | |
| 2-199 | Cl | 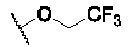 | SO₂CH₃ | 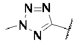 | 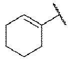 | |
| 2-200 | Cl | 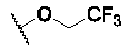 | SO₂CH₃ | 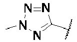 | 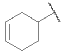 | |
| 2-201 | Cl | 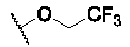 | SO₂CH₃ | 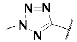 | 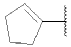 | |
| 2-202 | Cl | 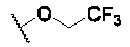 | SO₂CH₃ | 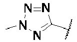 | 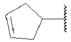 | |
| 2-203 | Cl | 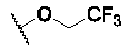 | SO₂CH₃ | 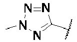 | 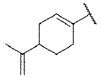 | |
| 2-204 | Cl | 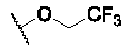 | SO₂CH₃ | 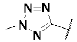 | 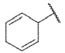 | |
| 2-205 | Cl | 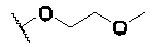 | SO₂CH₃ | 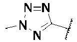 | 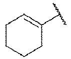 | |
| 2-206 | Cl | 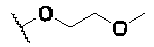 | SO₂CH₃ | 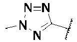 | 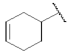 | |
| 2-207 | Cl | 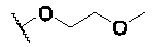 | SO₂CH₃ | 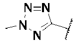 | 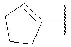 | |
| 2-208 | Cl | 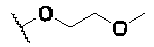 | SO₂CH₃ | 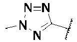 | 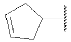 | |
| 2-209 | Cl | 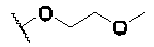 | SO₂CH₃ | 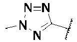 | 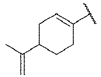 | |
| 2-210 | Cl | 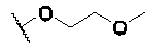 | SO₂CH₃ | 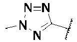 | 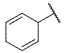 | |
| 2-211 | Cl | 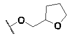 | SO₂CH₃ | 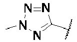 | 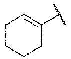 | |
| 2-212 | Cl | 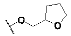 | SO₂CH₃ | 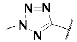 | 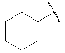 | |
| 2-213 | Cl | 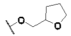 | SO₂CH₃ | 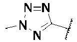 | 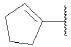 | |
| 2-214 | Cl | 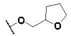 | SO₂CH₃ | 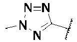 | 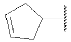 | |
| 2-215 | Cl | 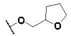 | SO₂CH₃ | 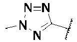 | 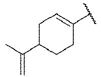 | |
| 2-216 | Cl | 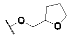 | SO₂CH₃ | 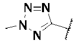 | 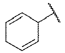 | |
| 2-217 | Cl | 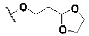 | SO₂CH₃ | 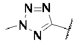 | 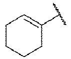 | |
| 2-218 | Cl | 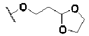 | SO₂CH₃ | 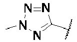 | 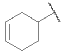 | |
| 2-219 | Cl | 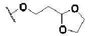 | SO₂CH₃ | 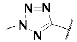 | 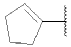 | |
| 2-220 | Cl | 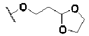 | SO₂CH₃ | 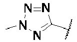 | 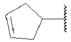 | |
| 2-221 | Cl | 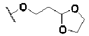 | SO₂CH₃ | 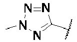 | 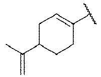 | |
| 2-222 | Cl | 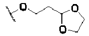 | SO₂CH₃ | 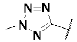 | 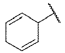 | |
| 2-223 | Cl | 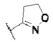 | SO₂CH₃ | 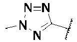 | 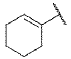 | |
| 2-224 | Cl | 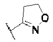 | SO₂CH₃ | 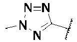 | 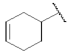 | |
| 2-225 | Cl | 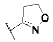 | SO₂CH₃ | 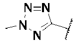 | 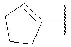 | |
| 2-226 | Cl | 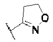 | SO₂CH₃ | 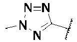 | 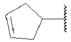 | |
| 2-227 | Cl | 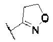 | SO₂CH₃ | 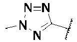 | 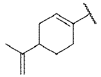 | |
| 2-228 | Cl | 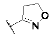 | SO₂CH₃ | 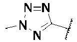 | 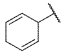 | |
| 2-229 | Cl | CH₃ | SO₂CH₃ | 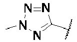 | 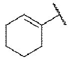 | |
| 2-230 | Cl | CH₃ | SO₂CH₃ | 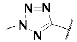 | 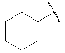 | |
| 2-231 | Cl | CH₃ | SO₂CH₃ | 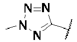 | 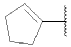 | |
| 2-232 | Cl | CH₃ | SO₂CH₃ | 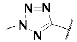 | 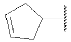 | |
| 2-233 | Cl | CH₃ | SO₂CH₃ | 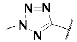 | 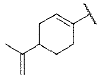 | |
| 2-234 | Cl | CH₃ | SO₂CH₃ | 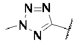 | 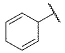 | |
| 2-235 | Cl | 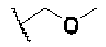 | SO₂CH₃ | 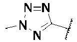 | 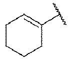 | yellow solid (82-84) |
| 2-236 | Cl | 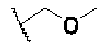 | SO₂CH₃ | 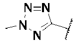 | 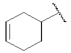 | |
| 2-237 | Cl | 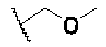 | SO₂CH₃ | 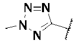 | 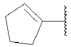 | yellow solid (128-130) |
| 2-238 | Cl | 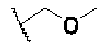 | SO₂CH₃ | 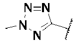 | 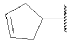 | |
| 2-239 | Cl | 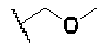 | SO₂CH₃ | 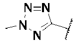 | 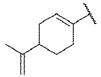 | |
| 2-240 | Cl | 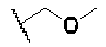 | SO₂CH₃ | 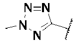 | 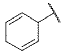 | |
| 2-241 | Cl | 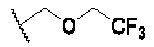 | SO₂CH₃ | 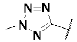 | 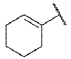 | |
| 2-242 | Cl | 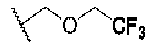 | SO₂CH₃ | 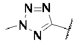 | 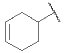 | |
| 2-243 | Cl | 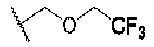 | SO₂CH₃ | 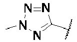 | 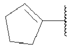 | |
| 2-244 | Cl | 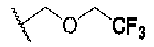 | SO₂CH₃ | 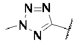 | 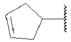 | |
| 2-245 | Cl | 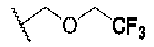 | SO₂CH₃ | 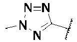 | 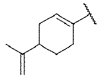 | |
| 2-246 | Cl | 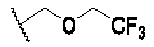 | SO₂CH₃ | 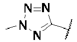 | 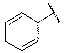 | |
| 2-247 | SO₂CH₃ | H | Cl | 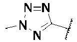 | 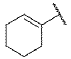 | |
| 2-248 | SO₂CH₃ | H | Cl | 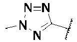 | 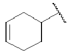 | |
| 2-249 | SO₂CH₃ | H | Cl | 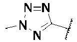 | 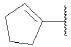 | |
| 2-250 | SO₂CH₃ | H | Cl | 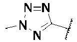 | 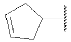 | |
| 2-251 | SO₂CH₃ | H | Cl | 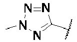 | 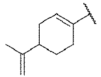 | |
| 2-252 | SO₂CH₃ | H | Cl | 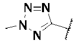 | 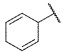 | |
| 2-253 | Cl | Cl | SO₂CH₃ | 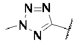 | 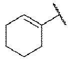 | |
| 2-254 | Cl | Cl | SO₂CH₃ | 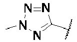 | 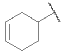 | |
| 2-255 | Cl | Cl | SO₂CH₃ | 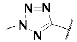 | 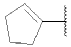 | |
| 2-256 | Cl | Cl | SO₂CH₃ | 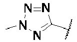 | 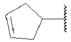 | |
| 2-257 | Cl | Cl | SO₂CH₃ | 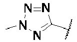 | 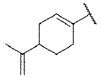 | |
| 2-258 | Cl | Cl | SO₂CH₃ | 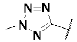 | 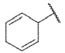 | |
| 2-259 | Cl | 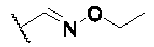 | SO₂CH₃ | 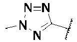 | 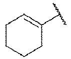 | |
| 2-260 | Cl | 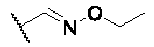 | SO₂CH₃ | 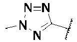 | 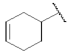 | |
| 2-261 | Cl | 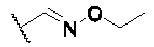 | SO₂CH₃ | 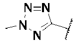 | 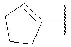 | |
| 2-262 | Cl | 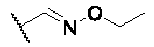 | SO₂CH₃ | 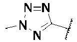 | 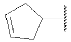 | |
| 2-263 | Cl | 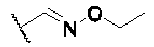 | SO₂CH₃ | 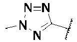 | 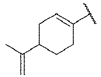 | |
| 2-264 | Cl | 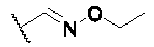 | SO₂CH₃ | 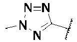 | 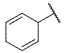 | |
| 2-265 | SO₂CH₃ | H | CF₃ | 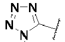 | 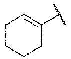 | off-white solid (131-133) |
| 2-266 | SO₂CH₃ | H | CF₃ | 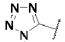 | 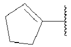 | off-white solid (107-109) |
| 2-267 | NO₂ | H | Cl | 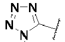 | 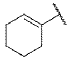 | yellow oil |
| 2-268 | Cl | H | Cl | 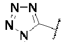 | 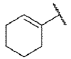 | pale yellow oil |
| 2-269 | Cl | H | SO₂CH₃ | 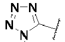 | 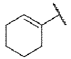 | pale yellow solid (126-128) |
| 2-270 | Cl | 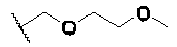 | SO₂CH₃ | 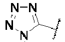 | 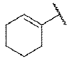 | pale yellow oil |
| 2-271 | Cl | H | NO₂ | 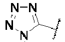 | 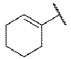 | yellow oil |
| 2-272 | Cl | CH₃SO₂CH₂ | SO₂CH₃ | 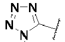 | 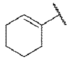 | |
| 2-273 | Cl | CH₃SO₂CH₂ | SO₂CH₃ | 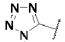 | 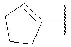 | |
| 2-274 | CH₃ | 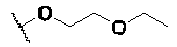 | SO₂CH₃ | 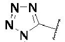 | 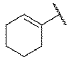 | yellow oil |
| 2-275 | CH₃ | 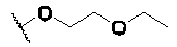 | SO₂CH₃ | 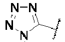 | 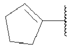 | yellow oil |
| 2-276 | SO₂CH₃ | H | Cl | 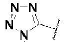 | 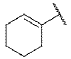 | yellow solid (175-176) |
| 2-277 | CH₃ | 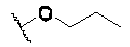 | SO₂CH₃ | 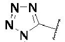 | 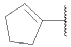 | brown solid (122-124) |
| 2-278 | CH₃ | 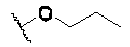 | SO₂CH₃ | 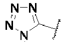 | 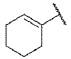 | orange yellow solid (139-141) |
| 2-279 | Cl | 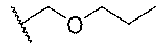 | SO₂CH₃ | 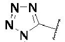 | 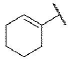 | orange oil |
| 2-280 | Cl | 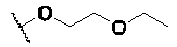 | SO₂CH₃ | 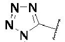 | 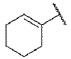 | yellow oil |
| 2-281 | Cl | 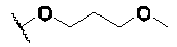 | SO₂CH₃ | 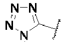 | 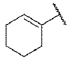 | yellow oil |
| 2-282 | Cl | 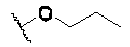 | SO₂CH₃ | 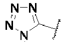 | 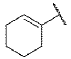 | pale yellow oil |
| 2-283 | Cl | 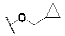 | SO₂CH₃ | 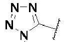 | 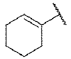 | pale yellow oil |

In the compound of the formula I, W is N and the stereo configuration is trans.

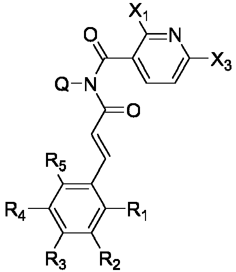

**Table 3 Structures and Physical Properties of Part of Compounds of Formula I**

| Compound | X₁ | X₃ | Q | R₁ | R₂ | R₃ | R₄ | R₅ | Appearance (Melting Point °C) |
|---|---|---|---|---|---|---|---|---|---|
| 3-1 | SO₂CH₃ | CF₃ | 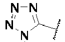 | H | H | H | H | H | |
| 3-2 | SO₂CH₃ | CF₃ | 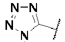 | Cl | H | Cl | H | H | |
| 3-3 | SO₂CH₃ | CF₃ | 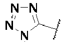 | H | H | OCH₃ | H | H | |
| 3-4 | SO₂CH₃ | CF₃ | 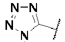 | H | H | NO₂ | H | H | |
| 3-5 | SO₂CH₃ | CF₃ | 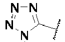 | H | H | CF₃ | H | H | |
| 3-6 | SO₂CH₃ | CF₃ | 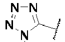 | H | OCF₃ | H | H | H | |
| 3-7 | SO₂CH₃ | CF₃ | 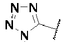 | H | H | 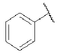 | H | H | |
| 3-8 | SO₂CH₃ | CF₃ | 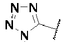 | H | 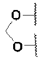 | | H | H | |
| 3-9 | SO₂CH₃ | CF₃ | 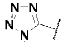 | H | 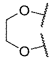 | | H | H | |
| 3-10 | SO₂CH₃ | CF₃ | 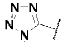 | H | 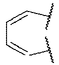 | | H | H | |
| 3-11 | SO₂CH₃ | CF₃ | 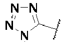 | 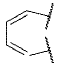 | | H | H | H | |
| 3-12 | NO₂ | SO₂CH₃ | 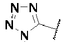 | H | H | H | H | H | |
| 3-13 | NO₂ | SO₂CH₃ | 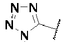 | H | 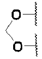 | | H | H | |
| 3-14 | NO₂ | Cl | 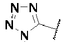 | H | H | H | H | H | |
| 3-15 | Cl | Cl | 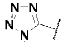 | H | H | H | H | H | |
| 3-16 | Cl | Cl | 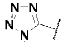 | H | 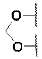 | | H | H | |
| 3-17 | Cl | SO₂CH₃ | 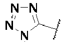 | H | H | H | H | H | |
| 3-18 | Cl | SO₂CH₃ | 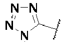 | H | 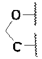 | | H | H | |
| 3-19 | CH₃ | SO₂CH₃ | 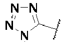 | H | H | H | H | H | |
| 3-20 | CN | SO₂CH₃ | 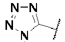 | H | H | H | H | H | |
| 3-21 | CF₃ | SO₂CH₃ | 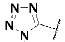 | H | H | H | H | H | |
| 3-22 | 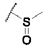 | SO₂CH₃ | 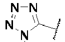 | H | H | H | H | H | |
| 3-23 | 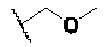 | SO₂CH₃ | 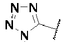 | H | H | H | H | H | |
| 3-24 | 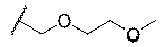 | SO₂CH₃ | 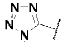 | H | H | H | H | H | |
| 3-25 | 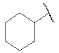 | SO₂CH₃ | 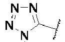 | H | H | H | H | H | |
| 3-26 | 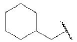 | SO₂CH₃ | 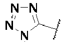 | H | H | H | H | H | |
| 3-27 | 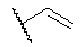 | SO₂CH₃ | 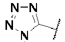 | H | H | H | H | H | |
| 3-28 | 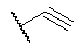 | SO₂CH₃ | 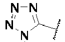 | H | H | H | H | H | |
| 3-29 | SO₂CH=CH₂ | SO₂CH₃ | 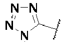 | H | H | H | H | H | |
| 3-30 | 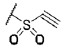 | SO₂CH₃ | 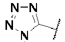 | H | H | H | H | H | |
| 3-31 | 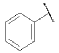 | SO₂CH₃ | 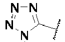 | H | H | H | H | H | |
| 3-32 | 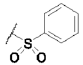 | SO₂CH₃ | 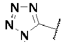 | H | H | H | H | H | |
| 3-33 | 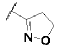 | SO₂CH₃ | 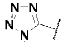 | H | H | H | H | H | |
| 3-34 | 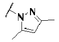 | SO₂CH₃ | 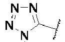 | H | H | H | H | H | |
| 3-35 | 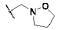 | SO₂CH₃ | 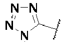 | H | H | H | H | H | |
| 3-36 | 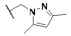 | SO₂CH₃ | 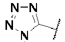 | H | H | H | H | H | |
| 3-37 | SO₂CH₃ | CF₃ | 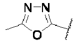 | H | H | H | H | H | |
| 3-38 | SO₂CH₃ | CF₃ | 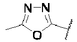 | Cl | H | Cl | H | H | |
| 3-39 | SO₂CH₃ | CF₃ | 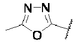 | H | H | OCH₃ | H | H | |
| 3-40 | SO₂CH₃ | CF₃ | 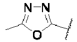 | H | H | NO₂ | H | H | |
| 3-41 | SO₂CH₃ | CF₃ | 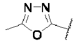 | H | H | CF₃ | H | H | |
| 3-42 | SO₂CH₃ | CF₃ | 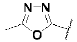 | H | OCF₃ | H | H | H | |
| 3-43 | SO₂CH₃ | CF₃ | 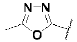 | H | H | 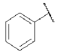 | H | H | |
| 3-44 | SO₂CH₃ | CF₃ | 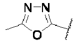 | H | 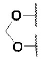 | | H | H | |
| 3-45 | SO₂CH₃ | CF₃ | 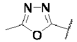 | H | 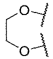 | | H | H | |
| 3-46 | SO₂CH₃ | CF₃ | 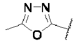 | H | 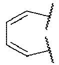 | | H | H | |
| 3-47 | SO₂CH₃ | CF₃ | 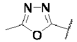 | 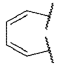 | | H | H | H | |
| 3-48 | NO₂ | SO₂CH₃ | 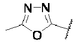 | H | H | H | H | H | |
| 3-49 | NO₂ | SO₂CH₃ | 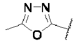 | H | 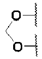 | | H | H | |
| 3-50 | NO₂ | Cl | 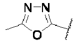 | H | H | H | H | H | |
| 3-51 | Cl | Cl | 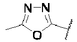 | H | H | H | H | H | |
| 3-52 | Cl | Cl | 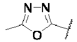 | H | 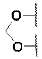 | | H | H | |
| 3-53 | Cl | SO₂CH₃ | 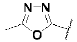 | H | H | H | H | H | |
| 3-54 | Cl | SO₂CH₃ | 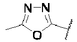 | H | 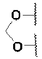 | | H | H | |
| 3-55 | CH₃ | SO₂CH₃ | 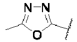 | H | H | H | H | H | |
| 3-56 | CN | SO₂CH₃ | 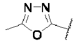 | H | H | H | H | H | |
| 3-57 | CF₃ | SO₂CH₃ | 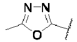 | H | H | H | H | H | |
| 3-58 | 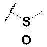 | SO₂CH₃ | 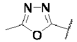 | H | H | H | H | H | |
| 3-59 | 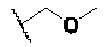 | SO₂CH₃ | 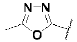 | H | H | H | H | H | |
| 3-60 | 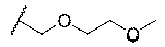 | SO₂CH₃ | 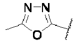 | H | H | H | H | H | |
| 3-61 | 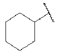 | SO₂CH₃ | 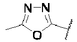 | H | H | H | H | H | |
| 3-62 | 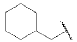 | SO₂CH₃ | 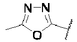 | H | H | H | H | H | |
| 3-63 | 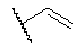 | SO₂CH₃ | 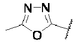 | H | H | H | H | H | |
| 3-64 | 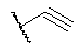 | SO₂CH₃ | 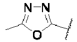 | H | H | H | H | H | |
| 3-65 | SO₂CH=CH₂ | SO₂CH₃ | 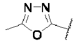 | H | H | H | H | H | |
| 3-66 | 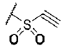 | SO₂CH₃ | 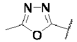 | H | H | H | H | H | |
| 3-67 | 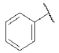 | SO₂CH₃ | 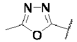 | H | H | H | H | H | |
| 3-68 | 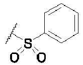 | SO₂CH₃ | 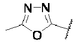 | H | H | H | H | H | |
| 3-69 | 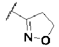 | SO₂CH₃ | 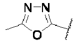 | H | H | H | H | H | |
| 3-70 | 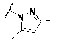 | SO₂CH₃ | 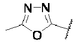 | H | H | H | H | H | |
| 3-71 | 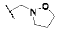 | SO₂CH₃ | 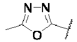 | H | H | H | H | H | |
| 3-72 | 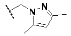 | SO₂CH₃ | 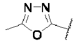 | H | H | H | H | H | |
| 3-73 | SO₂CH₃ | CF₃ | 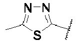 | H | H | H | H | H | |
| 3-74 | SO₂CH₃ | CF₃ | 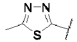 | Cl | H | Cl | H | H | |
| 3-75 | SO₂CH₃ | CF₃ | 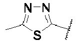 | H | H | OCH₃ | H | H | |
| 3-76 | SO₂CH₃ | CF₃ | 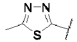 | H | H | NO₂ | H | H | |
| 3-77 | SO₂CH₃ | CF₃ | 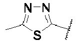 | H | H | CF₃ | H | H | |
| 3-78 | SO₂CH₃ | CF₃ | 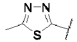 | H | OCF₃ | H | H | H | |
| 3-79 | SO₂CH₃ | CF₃ | 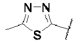 | H | H | 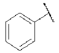 | H | H | |
| 3-80 | SO₂CH₃ | CF₃ | 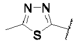 | H | 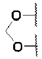 | | H | H | |
| 3-81 | SO₂CH₃ | CF₃ | 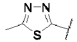 | H | 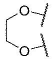 | | H | H | |
| 3-82 | SO₂CH₃ | CF₃ | 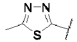 | H | 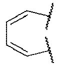 | | H | H | |
| 3-83 | SO₂CH₃ | CF₃ | 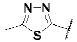 | 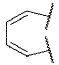 | | H | H | H | |
| 3-84 | NO₂ | SO₂CH₃ | 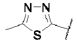 | H | H | H | H | H | |
| 3-85 | NO₂ | SO₂CH₃ | 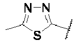 | H | 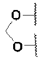 | | H | H | |
| 3-86 | NO₂ | Cl | 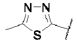 | H | H | H | H | H | |
| 3-87 | Cl | Cl | 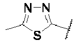 | H | H | H | H | H | |
| 3-88 | Cl | Cl | 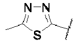 | H | 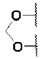 | | H | H | |
| 3-89 | Cl | SO₂CH₃ | 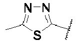 | H | H | H | H | H | |
| 3-90 | Cl | SO₂CH₃ | 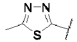 | H | 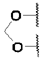 | | H | H | |
| 3-91 | CH₃ | SO₂CH₃ | 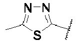 | H | H | H | H | H | |
| 3-92 | CN | SO₂CH₃ | 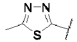 | H | H | H | H | H | |
| 3-93 | CF₃ | SO₂CH₃ | 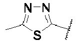 | H | H | H | H | H | |
| 3-94 | 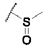 | SO₂CH₃ | 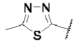 | H | H | H | H | H | |
| 3-95 | 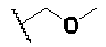 | SO₂CH₃ | 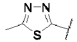 | H | H | H | H | H | |
| 3-96 | 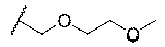 | SO₂CH₃ | 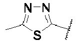 | H | H | H | H | H | |
| 3-97 | 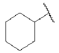 | SO₂CH₃ | 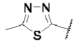 | H | H | H | H | H | |
| 3-98 | 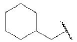 | SO₂CH₃ | 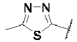 | H | H | H | H | H | |
| 3-99 | 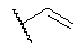 | SO₂CH₃ | 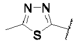 | H | H | H | H | H | |
| 3-100 | 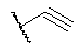 | SO₂CH₃ | 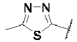 | H | H | H | H | H | |
| 3-101 | SO₂CH=CH₂ | SO₂CH₃ | 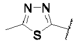 | H | H | H | H | H | |
| 3-102 | 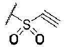 | SO₂CH₃ | 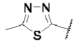 | H | H | H | H | H | |
| 3-103 | 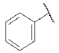 | SO₂CH₃ | 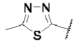 | H | H | H | H | H | |
| 3-104 | 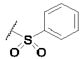 | SO₂CH₃ | 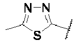 | H | H | H | H | H | |
| 3-105 | 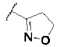 | SO₂CH₃ | 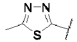 | H | H | H | H | H | |
| 3-106 | 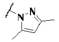 | SO₂CH₃ | 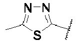 | H | H | H | H | H | |
| 3-107 | 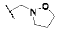 | SO₂CH₃ | 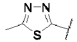 | H | H | H | H | H | |
| 3-108 | 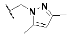 | SO₂CH₃ | 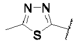 | H | H | H | H | H | |
| 3-109 | SO₂CH₃ | CF₃ | 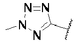 | H | H | H | H | H | |
| 3-110 | SO₂CH₃ | CF₃ | 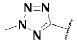 | Cl | H | Cl | H | H | |
| 3-111 | SO₂CH₃ | CF₃ | 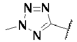 | H | H | OCH₃ | H | H | |
| 3-112 | SO₂CH₃ | CF₃ | 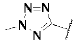 | H | H | NO₂ | H | H | |
| 3-113 | SO₂CH₃ | CF₃ | 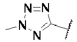 | H | H | CF₃ | H | H | |
| 3-114 | SO₂CH₃ | CF₃ | 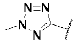 | H | OCF₃ | H | H | H | |
| 3-115 | SO₂CH₃ | CF₃ | 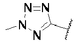 | H | H | 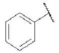 | H | H | |
| 3-116 | SO₂CH₃ | CF₃ | 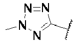 | H | 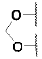 | | H | H | |
| 3-117 | SO₂CH₃ | CF₃ | 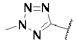 | H | 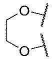 | | H | H | |
| 3-118 | SO₂CH₃ | CF₃ | 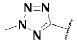 | H | 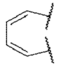 | | H | H | |
| 3-119 | SO₂CH₃ | CF₃ | 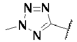 | 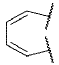 | | H | H | H | |
| 3-120 | NO₂ | SO₂CH₃ | 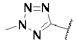 | H | H | H | H | H | |
| 3-121 | NO₂ | SO₂CH₃ | 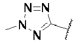 | H | 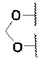 | | H | H | |
| 3-122 | NO₂ | Cl | 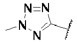 | H | H | H | H | H | |
| 3-123 | Cl | Cl | 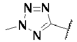 | H | H | H | H | H | |
| 3-124 | Cl | Cl | 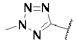 | H | 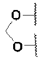 | | H | H | |
| 3-125 | Cl | SO₂CH₃ | 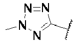 | H | H | H | H | H | |
| 3-126 | Cl | SO₂CH₃ | 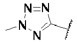 | H | 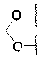 | | H | H | |
| 3-127 | CH₃ | SO₂CH₃ | 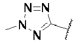 | H | H | H | H | H | |
| 3-128 | CN | SO₂CH₃ | 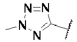 | H | H | H | H | H | |
| 3-129 | CF₃ | SO₂CH₃ | 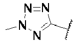 | H | H | H | H | H | |
| 3-130 | 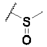 | SO₂CH₃ | 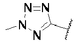 | H | H | H | H | H | |
| 3-131 | 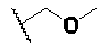 | SO₂CH₃ | 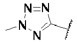 | H | H | H | H | H | |
| 3-132 | 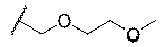 | SO₂CH₃ | 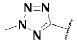 | H | H | H | H | H | |
| 3-133 | 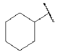 | SO₂CH₃ | 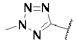 | H | H | H | H | H | |
| 3-134 | 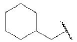 | SO₂CH₃ | 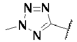 | H | H | H | H | H | |
| 3-135 | 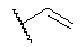 | SO₂CH₃ | 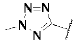 | H | H | H | H | H | |
| 3-136 | 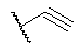 | SO₂CH₃ | 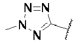 | H | H | H | H | H | |
| 3-137 | SO₂CH=CH₂ | SO₂CH₃ | 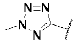 | H | H | H | H | H | |
| 3-138 | 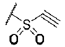 | SO₂CH₃ | 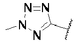 | H | H | H | H | H | |
| 3-139 | 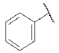 | SO₂CH₃ | 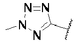 | H | H | H | H | H | |
| 3-140 | 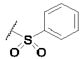 | SO₂CH₃ | 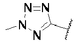 | H | H | H | H | H | |
| 3-141 | 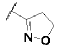 | SO₂CH₃ | 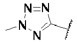 | H | H | H | H | H | |
| 3-142 | 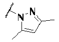 | SO₂CH₃ | 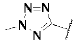 | H | H | H | H | H | |
| 3-143 | 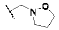 | SO₂CH₃ | 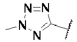 | H | H | H | H | H | |
| 3-144 | 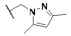 | SO₂CH₃ | 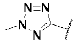 | H | H | H | H | H | |
| 3-145 | SO₂CH₃ | CF₃ | 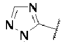 | H | H | H | H | H | |
| 3-146 | SO₂CH₃ | CF₃ | 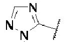 | Cl | H | Cl | H | H | |
| 3-147 | SO₂CH₃ | CF₃ | 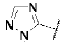 | H | H | OCH₃ | H | H | |
| 3-148 | SO₂CH₃ | CF₃ | 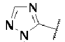 | H | H | NO₂ | H | H | |
| 3-149 | SO₂CH₃ | CF₃ | 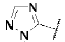 | H | H | CF₃ | H | H | |
| 3-150 | SO₂CH₃ | CF₃ | 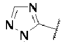 | H | OCF₃ | H | H | H | |
| 3-151 | SO₂CH₃ | CF₃ | 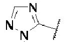 | H | H | 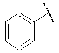 | H | H | |
| 3-152 | SO₂CH₃ | CF₃ | 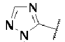 | H | 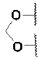 | | H | H | |
| 3-153 | SO₂CH₃ | CF₃ | 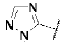 | H | 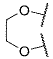 | | H | H | |
| 3-154 | SO₂CH₃ | CF₃ | 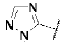 | H | 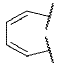 | | H | H | |
| 3-155 | SO₂CH₃ | CF₃ | 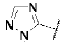 | 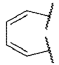 | | H | H | H | |
| 3-156 | NO₂ | SO₂CH₃ | 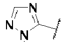 | H | H | H | H | H | |
| 3-157 | NO₂ | SO₂CH₃ | 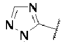 | H | 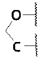 | | H | H | |
| 3-158 | NO₂ | Cl | 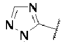 | H | H | H | H | H | |
| 3-159 | Cl | Cl | 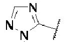 | H | H | H | H | H | |
| 3-160 | Cl | Cl | 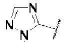 | H | 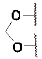 | | H | H | |
| 3-161 | Cl | SO₂CH₃ | 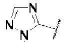 | H | H | H | H | H | |
| 3-162 | Cl | SO₂CH₃ | 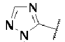 | H | 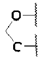 | | H | H | |
| 3-163 | CH₃ | SO₂CH₃ | 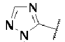 | H | H | H | H | H | |
| 3-164 | CN | SO₂CH₃ | 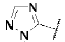 | H | H | H | H | H | |
| 3-165 | CF₃ | SO₂CH₃ | 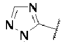 | H | H | H | H | H | |
| 3-166 | 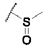 | SO₂CH₃ | 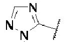 | H | H | H | H | H | |
| 3-167 | 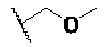 | SO₂CH₃ | 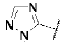 | H | H | H | H | H | |
| 3-168 | 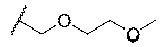 | SO₂CH₃ | 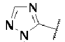 | H | H | H | H | H | |
| 3-169 | 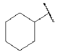 | SO₂CH₃ | 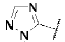 | H | H | H | H | H | |
| 3-170 | 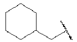 | SO₂CH₃ | 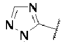 | H | H | H | H | H | |
| 3-171 | 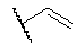 | SO₂CH₃ | 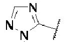 | H | H | H | H | H | |
| 3-172 | 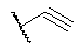 | SO₂CH₃ | 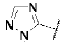 | H | H | H | H | H | |
| 3-173 | SO₂CH=CH₂ | SO₂CH₃ | 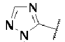 | H | H | H | H | H | |
| 3-174 | 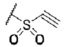 | SO₂CH₃ | 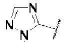 | H | H | H | H | H | |
| 3-175 | 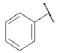 | SO₂CH₃ | 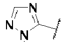 | H | H | H | H | H | |
| 3-176 | 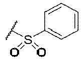 | SO₂CH₃ | 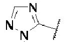 | H | H | H | H | H | |
| 3-177 | 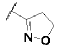 | SO₂CH₃ | 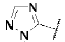 | H | H | H | H | H | |
| 3-178 | 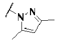 | SO₂CH₃ | 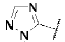 | H | H | H | H | H | |
| 3-179 | 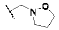 | SO₂CH₃ | 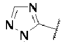 | H | H | H | H | H | |
| 3-180 | 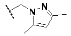 | SO₂CH₃ | 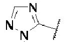 | H | H | H | H | H | |
| 3-181 | SO₂CH₃ | CF₃ | 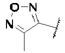 | H | H | H | H | H | |
| 3-182 | SO₂CH₃ | CF₃ | 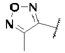 | Cl | H | Cl | H | H | |
| 3-183 | SO₂CH₃ | CF₃ | 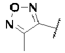 | H | H | OCH₃ | H | H | |
| 3-184 | SO₂CH₃ | CF₃ | 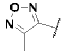 | H | H | NO₂ | H | H | |
| 3-185 | SO₂CH₃ | CF₃ | 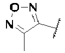 | H | H | CF₃ | H | H | |
| 3-186 | SO₂CH₃ | CF₃ | 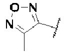 | H | OCF₃ | H | H | H | |
| 3-187 | SO₂CH₃ | CF₃ | 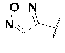 | H | H | 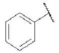 | H | H | |
| 3-188 | SO₂CH₃ | CF₃ | 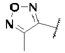 | H | 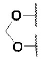 | | H | H | |
| 3-189 | SO₂CH₃ | CF₃ | 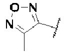 | H | 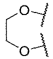 | | H | H | |
| 3-190 | SO₂CH₃ | CF₃ | 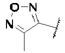 | H | 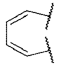 | | H | H | |
| 3-191 | SO₂CH₃ | CF₃ | 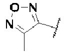 | 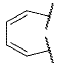 | | H | H | H | |
| 3-192 | NO₂ | SO₂CH₃ | 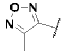 | H | H | H | H | H | |
| 3-193 | NO₂ | SO₂CH₃ | 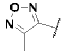 | H | 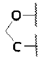 | | H | H | |
| 3-194 | NO₂ | Cl | 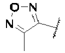 | H | H | H | H | H | |
| 3-195 | Cl | Cl | 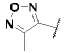 | H | H | H | H | H | |
| 3-196 | Cl | Cl | 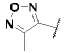 | H | 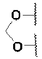 | | H | H | |
| 3-197 | Cl | SO₂CH₃ | 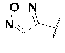 | H | H | H | H | H | |
| 3-198 | Cl | SO₂CH₃ | 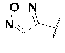 | H | 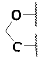 | | H | H | |
| 3-199 | CH₃ | SO₂CH₃ | 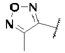 | H | H | H | H | H | |
| 3-200 | CN | SO₂CH₃ | 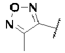 | H | H | H | H | H | |
| 3-201 | CF₃ | SO₂CH₃ | 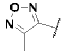 | H | H | H | H | H | |
| 3-202 | 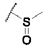 | SO₂CH₃ | 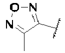 | H | H | H | H | H | |
| 3-203 | 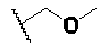 | SO₂CH₃ | 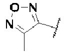 | H | H | H | H | H | |
| 3-204 | 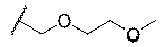 | SO₂CH₃ | 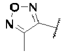 | H | H | H | H | H | |
| 3-205 | 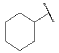 | SO₂CH₃ | 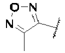 | H | H | H | H | H | |
| 3-206 | 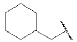 | SO₂CH₃ | 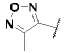 | H | H | H | H | H | |
| 3-207 | 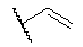 | SO₂CH₃ | 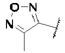 | H | H | H | H | H | |
| 3-208 | 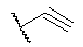 | SO₂CH₃ | 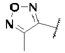 | H | H | H | H | H | |
| 3-209 | SO₂CH=CH₂ | SO₂CH₃ | 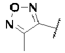 | H | H | H | H | H | |
| 3-210 | 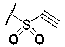 | SO₂CH₃ | 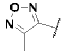 | H | H | H | H | H | |
| 3-211 | 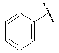 | SO₂CH₃ | 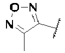 | H | H | H | H | H | |
| 3-212 | 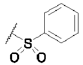 | SO₂CH₃ | 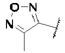 | H | H | H | H | H | |
| 3-213 | 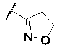 | SO₂CH₃ | 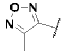 | H | H | H | H | H | |
| 3-214 | 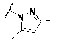 | SO₂CH₃ | 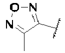 | H | H | H | H | H | |
| 3-215 | 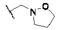 | SO₂CH₃ | 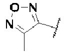 | H | H | H | H | H | |
| 3-216 | 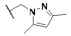 | SO₂CH₃ | 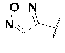 | H | H | H | H | H | |
| 3-217 | Cl | CF₃ | 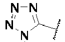 | H | H | H | H | H | white solid (158-164) |
| 3-218 | H | Cl | 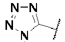 | H | H | H | H | H | white solid (171-175) |
| 3-219 | Cl | CH₃ | 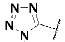 | H | H | H | H | H | white solid (124-130) |
| 3-220 | H | H | 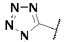 | H | H | H | H | H | white solid (110-116) |

In the compound of the formula I, W is N.

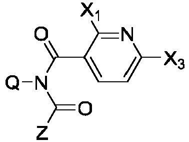

**Table 4 Structures and Physical Properties of Part of Compounds of Formula I**

| Compound | X₁ | X₃ | Q | Z | Appearance (Melting Point °C) |
|---|---|---|---|---|---|
| 4-1 | SO₂CH₃ | CF₃ | 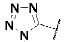 | 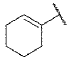 | |
| 4-2 | SO₂CH₃ | CF₃ | 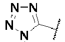 | 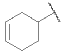 | |
| 4-3 | SO₂CH₃ | CF₃ | 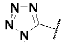 | 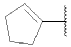 | |
| 4-4 | SO₂CH₃ | CF₃ | 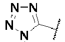 | 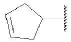 | |
| 4-5 | SO₂CH₃ | CF₃ | 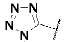 | 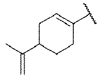 | |
| 4-6 | SO₂CH₃ | CF₃ | 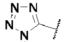 | 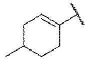 | |
| 4-7 | SO₂CH₃ | CF₃ | 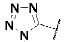 | 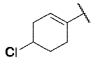 | |
| 4-8 | SO₂CH₃ | CF₃ | 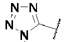 | 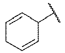 | |
| 4-9 | SO₂CH₃ | CF₃ | 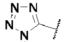 | 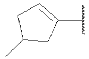 | |
| 4-10 | SO₂CH₃ | CF₃ | 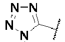 | 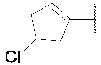 | |
| 4-11 | SO₂CH₃ | CF₃ | 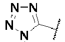 | 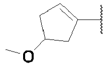 | |
| 4-12 | NO₂ | SO₂CH₃ | 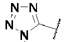 | 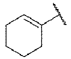 | |
| 4-13 | NO₂ | SO₂CH₃ | 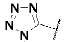 | 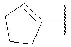 | |
| 4-14 | NO₂ | Cl | 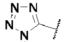 | 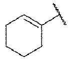 | |
| 4-15 | Cl | Cl | 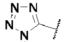 | 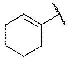 | |
| 4-16 | Cl | Cl | 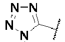 | 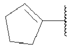 | |
| 4-17 | Cl | SO₂CH₃ | 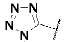 | 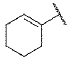 | |
| 4-18 | Cl | SO₂CH₃ | 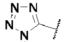 | 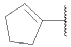 | |
| 4-19 | CH₃ | SO₂CH₃ | 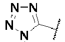 | 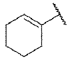 | |
| 4-20 | CN | SO₂CH₃ | 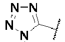 | 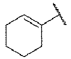 | |
| 4-21 | CF₃ | SO₂CH₃ | 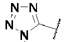 | 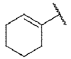 | |
| 4-22 | 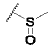 | SO₂CH₃ | 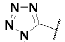 | 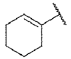 | |
| 4-23 | 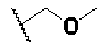 | SO₂CH₃ | 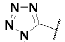 | 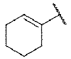 | |
| 4-24 | 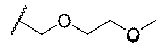 | SO₂CH₃ | 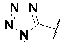 | 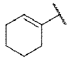 | |
| 4-25 | 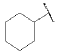 | SO₂CH₃ | 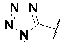 | 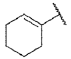 | |
| 4-26 | 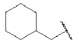 | SO₂CH₃ | 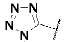 | 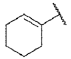 | |
| 4-27 | 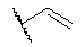 | SO₂CH₃ | 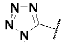 | 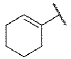 | |
| 4-28 | 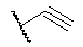 | SO₂CH₃ | 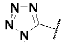 | 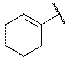 | |
| 4-29 | SO₂CH=CH₂ | SO₂CH₃ | 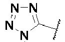 | 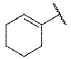 | |
| 4-30 | 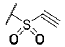 | SO₂CH₃ | 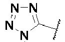 | 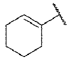 | |
| 4-31 | 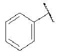 | SO₂CH₃ | 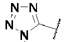 | 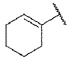 | |
| 4-32 | 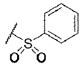 | SO₂CH₃ | 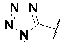 | 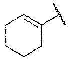 | |
| 4-33 | 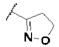 | SO₂CH₃ | 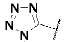 | 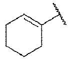 | |
| 4-34 | 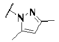 | SO₂CH₃ | 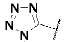 | 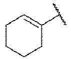 | |
| 4-35 | 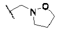 | SO₂CH₃ | 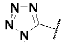 | 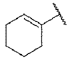 | |
| 4-36 | 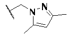 | SO₂CH₃ | 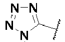 | 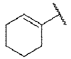 | |
| 4-37 | SO₂CH₃ | CF₃ | 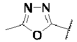 | 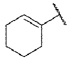 | |
| 4-38 | SO₂CH₃ | CF₃ | 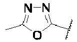 | 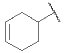 | |
| 4-39 | SO₂CH₃ | CF₃ | 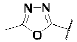 | 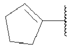 | |
| 4-40 | SO₂CH₃ | CF₃ | 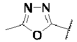 | 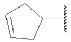 | |
| 4-41 | SO₂CH₃ | CF₃ | 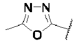 | 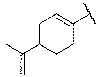 | |
| 4-42 | SO₂CH₃ | CF₃ | 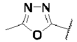 | 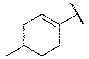 | |
| 4-43 | SO₂CH₃ | CF₃ | 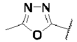 | 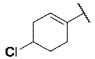 | |
| 4-44 | SO₂CH₃ | CF₃ | 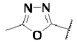 | 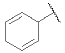 | |
| 4-45 | SO₂CH₃ | CF₃ | 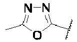 | 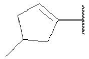 | |
| 4-46 | SO₂CH₃ | CF₃ | 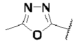 | 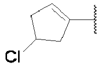 | |
| 4-47 | SO₂CH₃ | CF₃ | 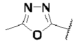 | 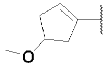 | |
| 4-48 | NO₂ | SO₂CH₃ | 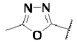 | 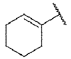 | |
| 4-49 | NO₂ | SO₂CH₃ | 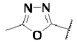 | 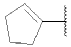 | |
| 4-50 | NO₂ | Cl | 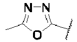 | 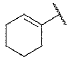 | |
| 4-51 | Cl | Cl | 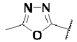 | 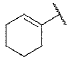 | |
| 4-52 | Cl | Cl | 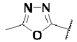 | 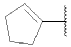 | |
| 4-53 | Cl | SO₂CH₃ | 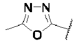 | 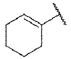 | |
| 4-54 | Cl | SO₂CH₃ | 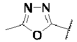 | 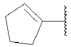 | |
| 4-55 | CH₃ | SO₂CH₃ | 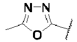 | 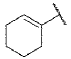 | |
| 4-56 | CN | SO₂CH₃ | 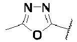 | 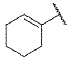 | |
| 4-57 | CF₃ | SO₂CH₃ | 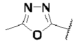 | 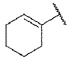 | |
| 4-58 | 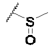 | SO₂CH₃ | 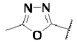 | 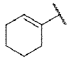 | |
| 4-59 | 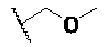 | SO₂CH₃ | 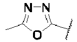 | 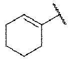 | |
| 4-60 | 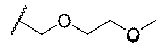 | SO₂CH₃ | 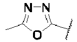 | 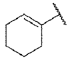 | |
| 4-61 | 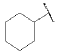 | SO₂CH₃ | 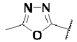 | 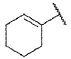 | |
| 4-62 | 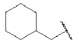 | SO₂CH₃ | 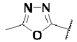 | 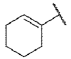 | |
| 4-63 | 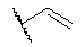 | SO₂CH₃ | 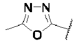 | 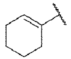 | |
| 4-64 | 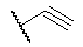 | SO₂CH₃ | 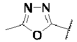 | 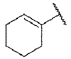 | |
| 4-65 | SO₂CH=CH₂ | SO₂CH₃ | 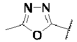 | 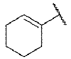 | |
| 4-66 | 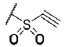 | SO₂CH₃ | 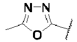 | 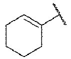 | |
| 4-67 | 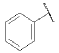 | SO₂CH₃ | 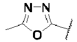 | 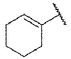 | |
| 4-68 | 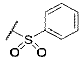 | SO₂CH₃ | 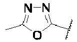 | 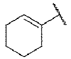 | |
| 4-69 | 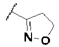 | SO₂CH₃ | 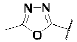 | 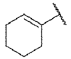 | |
| 4-70 | 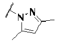 | SO₂CH₃ | 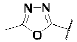 | 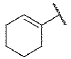 | |
| 4-71 | 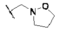 | SO₂CH₃ | 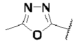 | 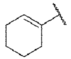 | |
| 4-72 | 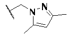 | SO₂CH₃ | 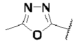 | 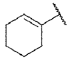 | |
| 4-73 | SO₂CH₃ | CF₃ | 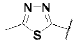 | 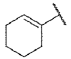 | |
| 4-74 | SO₂CH₃ | CF₃ | 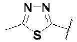 | 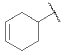 | |
| 4-75 | SO₂CH₃ | CF₃ | 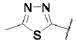 | 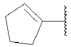 | |
| 4-76 | SO₂CH₃ | CF₃ | 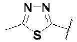 | 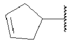 | |
| 4-77 | SO₂CH₃ | CF₃ | 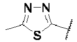 | 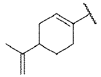 | |
| 4-78 | SO₂CH₃ | CF₃ | 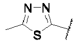 | 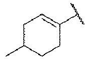 | |
| 4-79 | SO₂CH₃ | CF₃ | 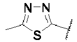 | 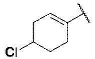 | |
| 4-80 | SO₂CH₃ | CF₃ | 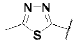 | 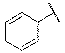 | |
| 4-81 | SO₂CH₃ | CF₃ | 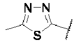 | 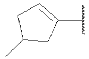 | |
| 4-82 | SO₂CH₃ | CF₃ | 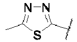 | 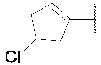 | |
| 4-83 | SO₂CH₃ | CF₃ | 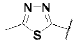 | 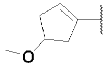 | |
| 4-84 | NO₂ | SO₂CH₃ | 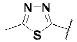 | 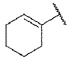 | |
| 4-85 | NO₂ | SO₂CH₃ | 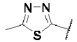 | 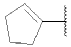 | |
| 4-86 | NO₂ | Cl | 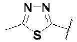 | 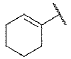 | |
| 4-87 | Cl | Cl | 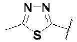 | 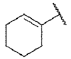 | |
| 4-88 | Cl | Cl | 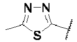 | 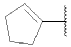 | |
| 4-89 | Cl | SO₂CH₃ | 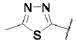 | 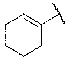 | |
| 4-90 | Cl | SO₂CH₃ | 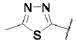 | 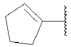 | |
| 4-91 | CH₃ | SO₂CH₃ | 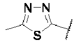 | 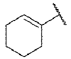 | |
| 4-92 | CN | SO₂CH₃ | 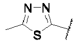 | 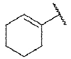 | |
| 4-93 | CF₃ | SO₂CH₃ | 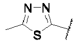 | 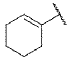 | |
| 4-94 | 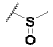 | SO₂CH₃ | 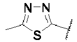 | 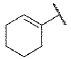 | |
| 4-95 | 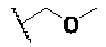 | SO₂CH₃ | 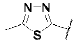 | 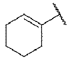 | |
| 4-96 | 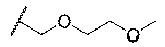 | SO₂CH₃ | 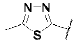 | 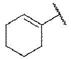 | |
| 4-97 | 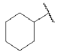 | SO₂CH₃ | 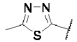 | 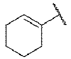 | |
| 4-98 | 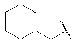 | SO₂CH₃ | 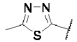 | 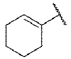 | |
| 4-99 | 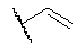 | SO₂CH₃ | 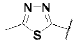 | 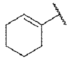 | |
| 4-100 | 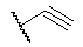 | SO₂CH₃ | 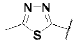 | 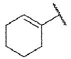 | |
| 4-101 | SO₂CH=CH₂ | SO₂CH₃ | 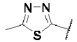 | 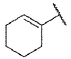 | |
| 4-102 | 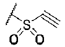 | SO₂CH₃ | 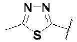 | 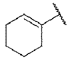 | |
| 4-103 | 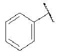 | SO₂CH₃ | 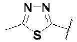 | 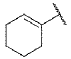 | |
| 4-104 | 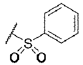 | SO₂CH₃ | 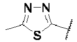 | 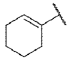 | |
| 4-105 | 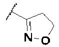 | SO₂CH₃ | 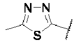 | 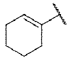 | |
| 4-106 | 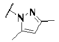 | SO₂CH₃ | 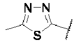 | 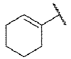 | |
| 4-107 | 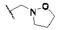 | SO₂CH₃ | 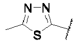 | 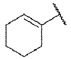 | |
| 4-108 | 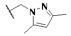 | SO₂CH₃ | 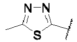 | 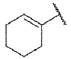 | |
| 4-109 | SO₂CH₃ | CF₃ | 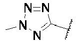 | 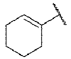 | |
| 4-110 | SO₂CH₃ | CF₃ | 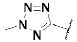 | 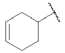 | |
| 4-111 | SO₂CH₃ | CF₃ | 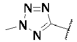 | 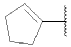 | |
| 4-112 | SO₂CH₃ | CF₃ | 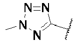 | 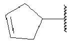 | |
| 4-113 | SO₂CH₃ | CF₃ | 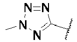 | 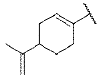 | |
| 4-114 | SO₂CH₃ | CF₃ | 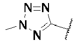 | 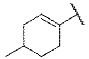 | |
| 4-115 | SO₂CH₃ | CF₃ | 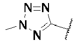 | 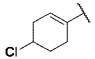 | |
| 4-116 | SO₂CH₃ | CF₃ | 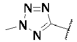 | 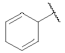 | |
| 4-117 | SO₂CH₃ | CF₃ | 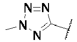 | 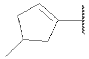 | |
| 4-118 | SO₂CH₃ | CF₃ | 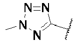 | 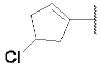 | |
| 4-119 | SO₂CH₃ | CF₃ | 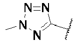 | 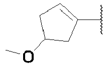 | |
| 4-120 | NO₂ | SO₂CH₃ | 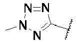 | 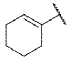 | |
| 4-121 | NO₂ | SO₂CH₃ | 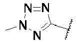 | 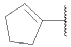 | |
| 4-122 | NO₂ | Cl | 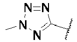 | 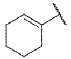 | |
| 4-123 | Cl | Cl | 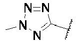 | 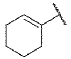 | |
| 4-124 | Cl | Cl | 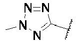 | 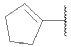 | |
| 4-125 | Cl | SO₂CH₃ | 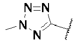 | 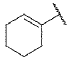 | |
| 4-126 | Cl | SO₂CH₃ | 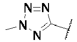 | 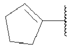 | |
| 4-127 | CH₃ | SO₂CH₃ | 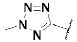 | 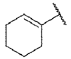 | |
| 4-128 | CN | SO₂CH₃ | 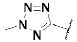 | 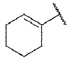 | |
| 4-129 | CF₃ | SO₂CH₃ | 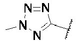 | 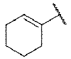 | |
| 4-130 | 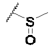 | SO₂CH₃ | 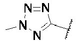 | 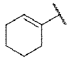 | |
| 4-131 | 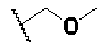 | SO₂CH₃ | 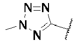 | 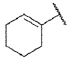 | |
| 4-132 | 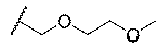 | SO₂CH₃ | 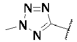 | 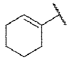 | |
| 4-133 | 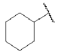 | SO₂CH₃ | 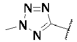 | 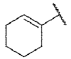 | |
| 4-134 | 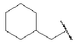 | SO₂CH₃ | 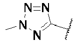 | 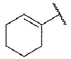 | |
| 4-135 | 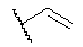 | SO₂CH₃ | 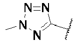 | 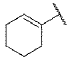 | |
| 4-136 | 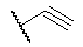 | SO₂CH₃ | 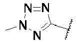 | 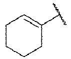 | |
| 4-137 | SO₂CH=CH₂ | SO₂CH₃ | 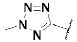 | 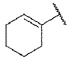 | |
| 4-138 | 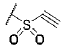 | SO₂CH₃ | 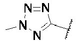 | 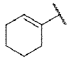 | |
| 4-139 | 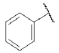 | SO₂CH₃ | 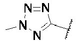 | 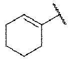 | |
| 4-140 | 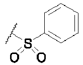 | SO₂CH₃ | 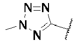 | 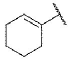 | |
| 4-141 | 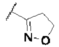 | SO₂CH₃ | 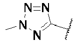 | 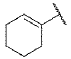 | |
| 4-142 | 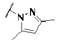 | SO₂CH₃ | 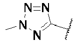 | 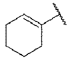 | |
| 4-143 | 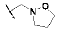 | SO₂CH₃ | 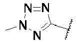 | 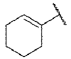 | |
| 4-144 | 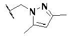 | SO₂CH₃ | 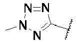 | 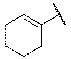 | |
| 4-145 | SO₂CH₃ | CF₃ | 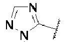 | 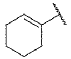 | |
| 4-146 | SO₂CH₃ | CF₃ | 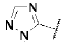 | 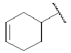 | |
| 4-147 | SO₂CH₃ | CF₃ | 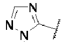 | 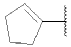 | |
| 4-148 | SO₂CH₃ | CF₃ | 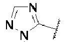 | 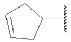 | |
| 4-149 | SO₂CH₃ | CF₃ | 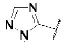 | 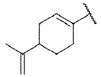 | |
| 4-150 | SO₂CH₃ | CF₃ | 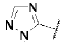 | 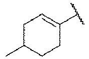 | |
| 4-151 | SO₂CH₃ | CF₃ | 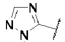 | 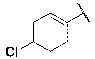 | |
| 4-152 | SO₂CH₃ | CF₃ | 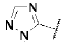 | 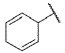 | |
| 4-153 | SO₂CH₃ | CF₃ | 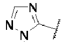 | 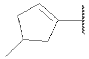 | |
| 4-154 | SO₂CH₃ | CF₃ | 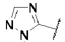 | 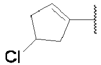 | |
| 4-155 | SO₂CH₃ | CF₃ | 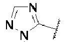 | 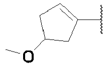 | |
| 4-156 | NO₂ | SO₂CH₃ | 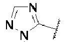 | 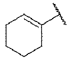 | |
| 4-157 | NO₂ | SO₂CH₃ | 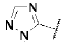 | 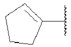 | |
| 4-158 | NO₂ | Cl | 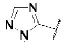 | 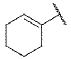 | |
| 4-159 | Cl | Cl | 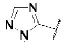 | 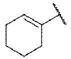 | |
| 4-160 | Cl | Cl | 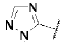 | 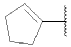 | |
| 4-161 | Cl | SO₂CH₃ | 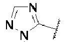 | 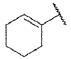 | |
| 4-162 | Cl | SO₂CH₃ | 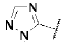 | 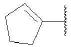 | |
| 4-163 | CH₃ | SO₂CH₃ | 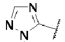 | 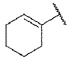 | |
| 4-164 | CN | SO₂CH₃ | 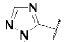 | 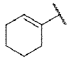 | |
| 4-165 | CF₃ | SO₂CH₃ | 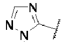 | 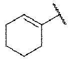 | |
| 4-166 | 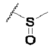 | SO₂CH₃ | 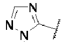 | 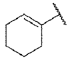 | |
| 4-167 | 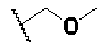 | SO₂CH₃ | 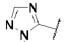 | 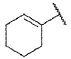 | |
| 4-168 | 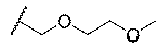 | SO₂CH₃ | 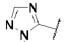 | 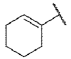 | |
| 4-169 | 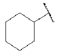 | SO₂CH₃ | 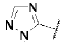 | 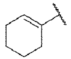 | |
| 4-170 | 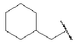 | SO₂CH₃ | 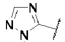 | 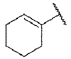 | |
| 4-171 | 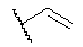 | SO₂CH₃ | 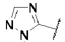 | 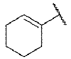 | |
| 4-172 | 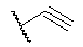 | SO₂CH₃ | 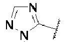 | 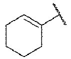 | |
| 4-173 | SO₂CH=CH₂ | SO₂CH₃ | 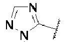 | 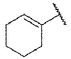 | |
| 4-174 | 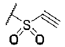 | SO₂CH₃ | 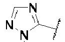 | 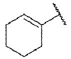 | |
| 4-175 | 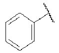 | SO₂CH₃ | 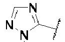 | 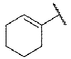 | |
| 4-176 | 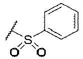 | SO₂CH₃ | 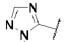 | 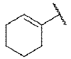 | |
| 4-177 | 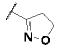 | SO₂CH₃ | 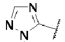 | 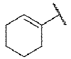 | |
| 4-178 | 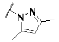 | SO₂CH₃ | 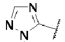 | 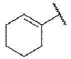 | |
| 4-179 | 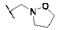 | SO₂CH₃ | 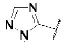 | 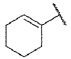 | |
| 4-180 | 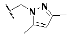 | SO₂CH₃ | 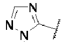 | 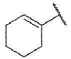 | |
| 4-181 | SO₂CH₃ | CF₃ | 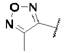 | 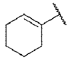 | |
| 4-182 | SO₂CH₃ | CF₃ | 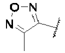 | 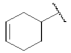 | |
| 4-183 | SO₂CH₃ | CF₃ | 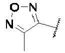 | 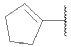 | |
| 4-184 | SO₂CH₃ | CF₃ | 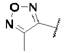 | 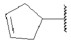 | |
| 4-185 | SO₂CH₃ | CF₃ | 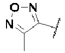 | 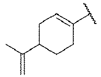 | |
| 4-186 | SO₂CH₃ | CF₃ | 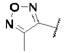 | 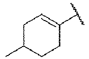 | |
| 4-187 | SO₂CH₃ | CF₃ | 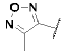 | 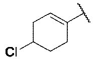 | |
| 4-188 | SO₂CH₃ | CF₃ | 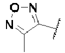 | 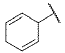 | |
| 4-189 | SO₂CH₃ | CF₃ | 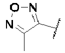 | 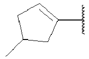 | |
| 4-190 | SO₂CH₃ | CF₃ | 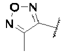 | 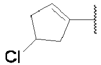 | |
| 4-191 | SO₂CH₃ | CF₃ | 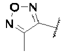 | 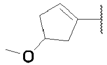 | |
| 4-192 | NO₂ | SO₂CH₃ | 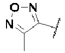 | 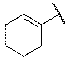 | |
| 4-193 | NO₂ | SO₂CH₃ | 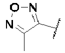 | 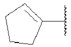 | |
| 4-194 | NO₂ | Cl | 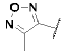 | 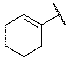 | |
| 4-195 | Cl | Cl | 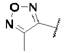 | 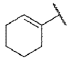 | |
| 4-196 | Cl | Cl | 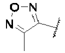 | 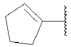 | |
| 4-197 | Cl | SO₂CH₃ | 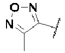 | 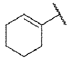 | |
| 4-198 | Cl | SO₂CH₃ | 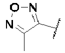 | 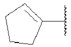 | |
| 4-199 | CH₃ | SO₂CH₃ | 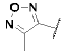 | 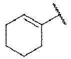 | |
| 4-200 | CN | SO₂CH₃ | 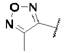 | 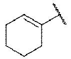 | |
| 4-201 | CF₃ | SO₂CH₃ | 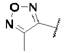 | 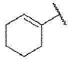 | |
| 4-202 | 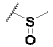 | SO₂CH₃ | 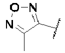 | 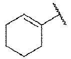 | |
| 4-203 | 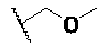 | SO₂CH₃ | 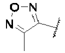 | 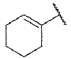 | |
| 4-204 | 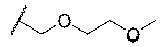 | SO₂CH₃ | 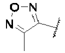 | 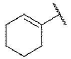 | |
| 4-205 | 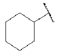 | SO₂CH₃ | 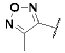 | 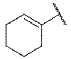 | |
| 4-206 | 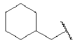 | SO₂CH₃ | 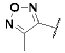 | 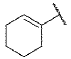 | |
| 4-207 | 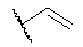 | SO₂CH₃ | 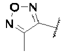 | 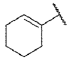 | |
| 4-208 | 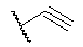 | SO₂CH₃ | 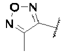 | 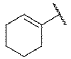 | |
| 4-209 | SO₂CH=CH₂ | SO₂CH₃ | 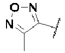 | 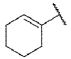 | |
| 4-210 | 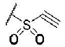 | SO₂CH₃ | 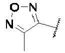 | 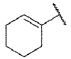 | |
| 4-211 | 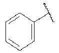 | SO₂CH₃ | 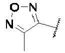 | 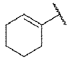 | |
| 4-212 | 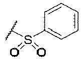 | SO₂CH₃ | 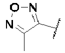 | 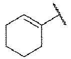 | |
| 4-213 | 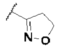 | SO₂CH₃ | 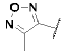 | 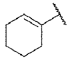 | |
| 4-214 | 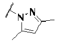 | SO₂CH₃ | 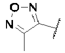 | 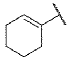 | |
| 4-215 | 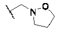 | SO₂CH₃ | 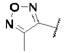 | 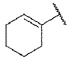 | |
| 4-216 | 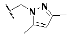 | SO₂CH₃ | 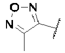 | 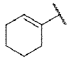 | |
| 4-217 | 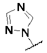 | CF₃ | 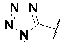 | 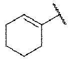 | |
| 4-218 | 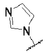 | CF₃ | 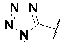 | 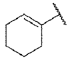 | |
| 4-219 | 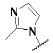 | CF₃ | 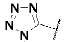 | 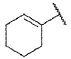 | |
| 4-220 | 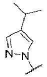 | CF₃ | 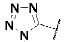 | 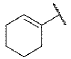 | |
| 4-221 | 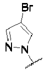 | CF₃ | 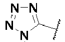 | 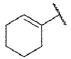 | |
| 4-222 | 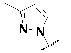 | CF₃ | 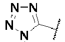 | 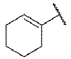 | |
| 4-223 | 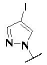 | CF₃ | 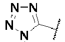 | 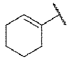 | |
| 4-224 | 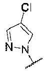 | CF₃ | 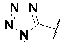 | 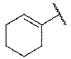 | |
| 4-225 | 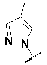 | CF₃ | 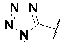 | 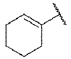 | |
| 4-226 | 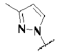 | CF₃ | 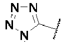 | 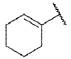 | |
| 4-227 | 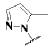 | CF₃ | 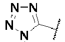 | 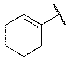 | |
| 4-228 | 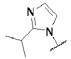 | CF₃ | 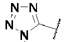 | 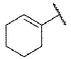 | |
| 4-229 | 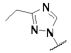 | CF₃ | 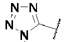 | 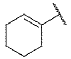 | |
| 4-230 | 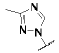 | CF₃ | 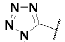 | 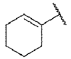 | |
| 4-231 | 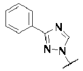 | CF₃ | 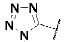 | 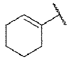 | |
| 4-232 | 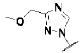 | CF₃ | 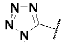 | 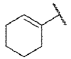 | |
| 4-233 | 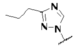 | CF₃ | 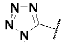 | 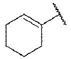 | |
| 4-234 | 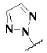 | CF₃ | 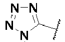 | 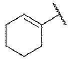 | |
| 4-235 | 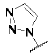 | CF₃ | 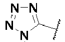 | 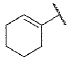 | |
| 4-236 | 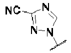 | CF₃ | 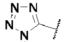 | 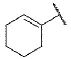 | |
| 4-237 | 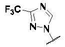 | CF₃ | 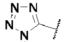 | 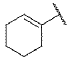 | |
| 4-238 | 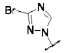 | CF₃ | 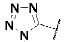 | 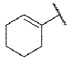 | |
| 4-239 | 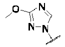 | CF₃ | 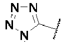 | 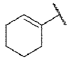 | |
| 4-240 | 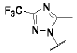 | CF₃ | 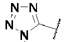 | 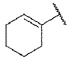 | |
| 4-241 | 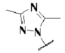 | CF₃ | 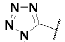 | 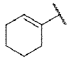 | |
| 4-242 | 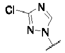 | CF₃ | 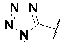 | 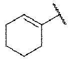 | |
| 4-243 | 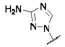 | CF₃ | 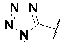 | 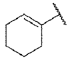 | |
| 4-244 | 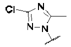 | CF₃ | 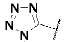 | 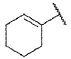 | |
| 4-245 | 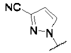 | CF₃ | 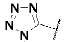 | 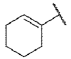 | |
| 4-246 | 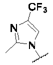 | CF₃ | 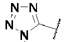 | 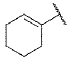 | |
| 4-247 | 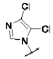 | CF₃ | 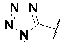 | 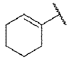 | |
| 4-248 | Cl | CF₃ | 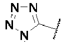 | 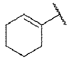 | white oil |
| 4-249 | Cl | CF₃ | 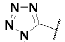 | 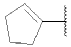 | white oil |

¹H NMR data of part of compounds is as follows:
Compound 1-1 (600MHz, DMSO-*d*₆): 8.40 (s, 1H), 8.28 (d, 1H), 8.18 (d, 1H), 7.79 (d, 1H), 7.63 (d, 2H), 7.40-7.47 (m, 3H), 6.56 (s, 1H), 4.17 (s, 3H), 3.41 (s, 3H).
Compound 1-8 (600MHz, DMSO-*d*₆): 8.39 (s, 1H), 8.31 (s, 1H), 8.27 (d, 1H), 8.16 (d, 1H), 7.70 (d, 1H), 7.34 (s, 1H), 7.17 (d, 1H), 6.96 (d, 1H), 6.09 (s, 2H), 4.16(s, 3H), 3.39(s, 3H).
Compound 1-12 (600MHz, DMSO-*d*₆): 8.78 (s, 1H), 8.52 (s, 1H), 8.27 (s, 1H), 7.79 (d, 1H), 7.65 (s, 2H), 7.42-7.45 (m, 3H), 6.56 (d, 1H), 4.22 (s, 3H), 3.46 (s, 3H).
Compound 1-13 (600MHz, DMSO-*d*₆): 8.76 (s, 1H), 8.50 (d, 1H), 8.24 (d, 1H), 7.70 (d, 1H), 7.37 (s, 1H), 7.18 (d, 1H), 6.96 (d, 1H), 6.07-6.09 (m, 3H), 4.20 (s, 3H), 3.46 (s, 3H).
Compound 1-14 (600MHz, DMSO-*d*₆): 8.43 (d, 1H), 8.07 (dd, 1H), 7.98 (d, 1H), 7.79 (d, 1H), 7.65 (d, 2H), 7.41-7.43(m, 3H), 6.65 (d, 1H), 4.17 (s, 3H).
Compound 1-15 (600MHz, DMSO-*d*₆): 7.81-7.83 (m, 3H), 7.67 (d, 2H), 7.59 (d, 1H), 7.43-7.47 (m, 3H), 6.90 (d, 1H), 4.09 (s, 3H).
Compound 1-16 (600MHz, DMSO-*d*₆): 7.80-7.82 (m, 2H), 7.72-7.75 (m, 1H), 7.58 (dd, 1H), 7.34 (s, 1H), 7.22 (d, 1H), 6.98 (d, 1H), 6.65 (d, 1H), 6.10 (s, 2H), 4.08 (s, 3H).
Compound 1-17 (600MHz, DMSO-*d*₆): 8.14 (s, 1H), 8.10 (d, 1H), 8.02 (d, 1H), 7.83 (d, 1H), 7.67 (d, 2H), 7.42-7.49 (m, 3H), 6.82 (s, 1H), 4.14 (s, 3H), 3.35(s, 3H).
Compound 1-18 (600MHz, DMSO-*d*₆): 8.14 (s, 1H), 8.08 (d, 1H), 8.01 (dd, 1H), 7.74 (d, 1H), 7.37 (s, 1H), 7.22 (d, 1H), 6.98 (d, 1H), 6.56 (d, 1H), 6.10 (s, 2H), 4.12 (s, 3H), 3.36(s, 3H).
Compound 1-19 (600MHz, DMSO-*d*₆): 8.03 (d, 1H), 7.89 (d, 1H), 7.82 (d, 1H), 7.66 (d, 2H), 7.39-7.49 (m, 3H), 6.79 (d, 1H), 4.14 (s, 3H), 3.32(s, 3H), 2.72(s, 3H).
Compound 1-20 (600MHz, DMSO-*d*₆): 8.02 (d, 1H), 7.88 (d, 1H), 7.73 (d, 1H), 7.36 (s, 1H), 7.21 (d, 1H), 6.98 (d, 1H), 6.54 (d, 1H), 6.10 (s, 2H), 4.13 (s, 3H), 3.34 (s, 3H), 2.72(s, 3H).
Compound 1-21 (600MHz, DMSO-*d*₆): 8.02 (d, 1H), 7.87 (d, 1H), 7.78 (d, 1H), 7.63 (d, 2H), 6.98 (d, 2H), 6.58 (d, 1H), 4.13 (s, 3H), 3.80 (s, 3H), 3.33 (s, 3H), 2.72 (s, 3H).
Compound 1-22 (600MHz, DMSO-*d*₆): 8.03 (d, 1H), 7.86-7.91 (m, 2H), 7.71-7.76 (m, 6H), 7.49 (t, 2H), 7.41 (t, 1H), 6.82 (d, 1H), 4.15 (s, 3H), 3.34 (s, 3H), 2.73(s, 3H).
Compound 1-24 (600MHz, DMSO-*d*₆): 8.10 (d, 1H), 8.06 (d, 1H), 7.82 (d, 1H), 7.66 (d, 2H), 7.42-7.49 (m, 3H), 6.76 (d, 1H), 4.95 (s, 2H), 4.15 (s, 3H), 3.39(s, 3H), 3.35(s, 3H).
Compound 1-26 (600MHz, CDCl₃): 8.18 (d, 1H), 7.84 (d, 1H), 7.66 (d, 1H), 7.42 (d, 2H), 6.89 (d, 2H), 6.42 (d, 1H), 5.07 (s, 2H), 4.06 (s, 3H), 3.85 (s, 3H), 3.49 (s, 3H), 3.25 (s, 3H).
Compound 1-28 (600MHz, CDCl₃): 8.18 (d, 1H), 7.88 (d, 1H), 7.64-7.67 (m, 3H), 7.58-7.61 (m, 2H), 6.79 (d, 1H), 5.05 (s, 2H), 4.05 (s, 3H), 3.47 (s, 3H), 3.23 (s,3H).
Compound 1-30 (600MHz, DMSO-*d*₆): 8.11 (d, 2H), 7.87 (d, 1H), 7.72-7.78 (m, 7H), 7.49 (t, 2H), 7.41 (t, 1H), 4.96 (s, 2H), 4.17 (s, 3H), 3.40 (s, 3H), 3.38 (s, 3H).
Compound 1-31 (600MHz, CDCl₃): 8.17 (d, 1H), 7.77 (d, 1H), 7.65 (d, 1H), 7.00 (dd, 1H), 6.91 (d, 1H), 6.81 (d, 1H), 6.40 (d, 1H), 6.03 (s, 2H), 5.06 (s, 2H), 4.05 (s, 3H), 3.48 (s, 3H), 3.24 (s, 3H).
Compound 1-33 (600MHz, CDCl₃): 8.75 (d, 1H), 8.20 (d, 1H), 8.13 (d, 1H), 7.96 (d, 1H), 7.88 (d, 1H), 7.68-7.70 (m, 2H), 7.58-7.60 (m, 1H), 7.54-7.56 (m, 1H), 7.48 (t, 1H), 6.71 (d, 1H), 5.08 (s, 2H), 4.09 (s, 3H), 3.49 (s, 3H), 3.25 (s, 3H).
Compound 1-41 (600MHz, DMSO-d6): 8.06-8.11 (m, 2H), 7.82 (d, 1H), 7.65 (d, 2H), 7.43-7.48 (m, 4H), 5.06 (s, 2H), 4.15 (s, 3H), 3.69-3.72 (m, 1H), 3.50-3.62 (m, 4H), 3.38 (s, 3H), 1.75-1.78 (m, 3H), 1.49-1.54 (m, 1H).
Compound 1-53 (600MHz, DMSO-*d*₆): 8.09 (s, 2H), 7.82 (d, 1H), 7.66 (d, 2H), 7.43-7.47 (m, 3H), 6.78(d, 1H), 5.05(s, 2H), 4.16 (s, 3H), 3.68(s, 2H), 3.49(s, 2H), 3.37(s, 3H), 3.23(s, 3H).
Compound 1-56 (600MHz, CDCl₃): 7.98 (d, 1H), 7.87 (d, 1H), 7.43-7.48 (m, 4H), 7.36-7.41 (m, 2H), 6.61 (d, 1H), 4.06 (s, 3H), 4.05 (s, 3H), 3.24 (s, 3H).
Compound 1-57 (600MHz, CDCl₃): 7.98 (d, 1H), 7.87 (d, 1H), 7.36-7.49 (m, 6H), 6.62 (d, 1H), 4.27 (q, 2H), 4.05 (s, 3H), 3.25 (s, 3H), 1.49 (t, 3H).
Compound 1-90 (600MHz, DMSO-*d*₆): 8.15 (d, 1H), 8.07 (d, 1H), 7.85 (d, 1H), 7.67 (d, 2H), 7.43-7.48 (m, 3H), 6.76 (d, 1H), 4.16 (s, 3H), 3.48(s, 3H).
Compound 1-91 (600MHz, CDCl₃): 7.79-7.84 (m, 2H), 7.67-7.69 (m, 3H), 7.44-7.49 (m, 3H), 7.08 (d, 1H), 4.10 (s, 3H), 3.22 (s, 3H), 2.60 (s, 3H), 2.40 (s, 3H).
compound 1-95 (600MHz, CDCl₃): 7.92 (d, 1H), 7.81 (d, 1H), 7.46-7.53 (m, 5H), 7.19 (d, 1H), 6.87 (d, 1H), 4.01 (s, 3H), 3.96 (s, 3H), 3.22 (s, 3H), 2.53 (s, 3H).
Compound 1-96 (600MHz, DMSO-*d*₆): 7.81 (d, 1H), 7.73 (d, 1H), 7.63-7.68 (m, 3H), 7.44-7.49 (m, 3H), 7.05 (d, 1H), 4.10 (s, 3H), 4.01 (q, 2H), 3.29 (s, 3H), 2.41 (s, 3H), 1.41 (t, 3H).
Compound 1-97 (600MHz, DMSO-*d*₆): 7.81 (d, 1H), 7.75 (d, 1H), 7.67-7.68 (m, 3H), 7.44-7.49 (m, 3H), 7.07 (d, 1H), 4.06-4.11 (m, 5H), 3.74 (t, 2H), 3.36 (s, 3H), 3.33 (s, 3H), 2.44 (s, 3H).
Compound 1-98 (600MHz, CDCl₃): 7.88 (d, 1H), 7.85 (d, 1H), 7.79 (d, 1H), 7.66-7.68 (m, 2H), 7.43-7.49 (m, 3H), 7.03 (d, 1H), 4.86 (s, 2H), 4.11 (s, 3H), 3.38 (s, 3H), 3.25 (s, 3H), 2.51 (s, 3H).
Compound 1-100 (600MHz, CDCl₃): 7.91 (d, 1H), 7.82 (d, 1H), 7.40-7.52 (m, 5H), 7.20 (d, 1H), 6.86 (d, 1H), 4.37-4.41(m, 1H), 4.03-4.09(m, 2H), 4.01 (s, 3H), 3.95-3.98(m, 1H), 3.86-3.90 (m, 1H), 3.28 (s, 3H), 2.56 (s, 3H), 2.06-2.12 (m, 1H), 1.93-1.99 (m, 2H), 1.67-1.73 (m, 1H).
Compound 1-102 (600MHz, DMSO-*d*₆): 7.79-7.81 (m, 3H), 7.67 (d, 2H), 7.44-7.50 (m, 3H), 7.03 (d, 1H), 4.64 (q, 2H), 4.12 (s, 3H), 3.31 (s, 3H), 2.45 (s, 3H).
Compound 1-154 (600MHz, DMSO-*d*₆): 8.25 (s, 1H), 8.22 (d, 1H), 8.05 (d, 1H), 7.93 (d, 1H), 7.75-7.77 (m, 2H), 7.73 (s, 1H), 7.46-7.50 (m, 3H), 3.63 (s, 3H), 2.66 (s, 3H).
Compound 1-187 (600MHz, CDCl₃): 8.29 (s, 1H), 7.91 (d, 1H), 7.70-7.74 (m, 2H), 7.30-7.37 (m, 5H), 6.44 (d, 1H), 4.39 (s, 3H), 3.23 (s, 3H).
Compound 1-296 (600MHz, CDCl₃): 7.91 (s, 1H), 7.80 (d, 1H), 7.51-7.53 (m, 2H), 7.46 (t, 1H), 7.40-7.43 (m, 2H), 7.16 (d, 1H), 6.89 (d, 1H), 4.00-4.02 (m, 5H), 3.23 (s, 3H), 2.51 (s, 3H), 1.88-1.94 (m, 2H), 1.09 (t, 3H).
Compound 1-297 (600MHz, CDCl₃): 7.92 (d, 1H), 7.84 (d, 1H), 7.53-7.55 (m, 2H), 7.45-7.48 (m, 1H), 7.40-7.43 (m, 2H), 7.11 (d, 1H), 6.96 (d, 1H), 4.83-4.87 (m, 1H), 4.00 (s, 3H), 3.20 (s, 3H), 2.51 (s, 3H), 1.33 (d, 6H).
Compound 1-298 (600MHz, CDCl₃): 8.17 (d, 1H), 7.84 (d, 1H), 7.65 (d, 1H), 7.35 (d, 2H), 7.18 (d, 2H), 6.53 (d, 1H), 5.06 (s, 2H), 4.05 (s, 3H), 3.48 (s, 3H), 3.24 (s, 3H), 2.38 (s, 3H).
Compound 1-299 (600MHz, CDCl₃): 8.18 (d, 1H), 7.84 (d, 1H), 7.66 (d, 1H), 7.47-7.49 (m, 2H), 7.09 (t, 2H), 6.58 (d, 1H), 5.06 (s, 2H), 4.06 (s, 3H), 3.48 (s, 3H), 3.24 (s, 3H).
Compound 1-300 (600MHz, CDCl₃): 8.17-8.19 (m, 2H), 7.67 (d, 1H), 7.42 (d, 1H), 7.31-7.34 (m, 1H), 7.18-7.26 (m, 2H), 6.51 (d, 1H), 5.07 (s, 2H), 4.07 (s, 3H), 3.49 (s, 3H), 3.24 (s, 3H), 2.41 (s ,3H).
Compound 1-301 (600MHz, CDCl₃): 8.17 (d, 1H), 8.04 (d, 1H), 7.65 (d, 1H), 7.34-7.40 (m, 2H), 6.88-6.94 (m, 2H), 6.66 (d, 1H), 5.07 (s, 2H), 4.06 (s, 3H), 3.81 (s, 3H), 3.48 (s, 3H), 3.24 (s ,3H).
Compound 1-302 (600MHz, CDCl₃): 7.88 (d, 1H), 7.80 (d, 1H), 7.50-7.51 (m, 2H), 7.44-7.46 (m, 1H), 7.39-7.41 (m, 2H), 7.19 (d, 1H), 6.86 (d, 1H), 4.21 (t, 2H), 3.99 (s, 3H), 3.82 (t, 2H), 3.61 (q, 2H), 3.26 (s ,3H), 2.55 (s, 3H), 1.26 (t, 3H).
Compound 1-303 (600MHz, CDCl₃): 8.18 (d, 1H), 7.97 (d, 1H), 7.66 (d, 1H), 7.39-7.46 (m, 2H), 7.14-7.19 (m, 1H), 7.06-7.12 (m, 1H), 6.70 (d, 1H), 5.06 (s, 2H), 4.05 (s, 3H), 3.47 (s, 3H), 3.24 (s, 3H).
Compound 1-304 (600MHz, CDCl₃): 8.19-8.23 (m, 1H), 8.18 (d, 1H), 7.71 (d, 1H), 7.67 (d, 1H), 7.51-7.61 (m, 3H), 6.67 (d, 1H), 5.06 (s, 2H), 4.05 (s, 3H), 3.47 (s, 3H), 3.24 (s, 3H).
Compound 1-305 (600MHz, CDCl₃): 8.27 (d, 1H), 8.17 (d, 1H), 7.66 (d, 1H), 7.50 (d, 1H), 7.42 (d, 1H), 7.33-7.38 (m, 1H), 7.26-7.30 (m, 1H), 6.67 (d, 1H), 5.06 (s, 2H), 4.05 (s, 3H), 3.47 (s, 3H), 3.24 (s, 3H).
Compound 1-306 (600MHz, CDCl₃): 8.24 (d, 1H), 8.18 (d, 1H), 7.66 (d, 1H), 7.62 (d, 1H), 7.49 (d, 1H), 7.30-7.34 (m, 1H), 7.26-7.30 (m, 1H), 6.63 (d, 1H), 5.06 (s, 2H), 4.05 (s, 3H), 3.47 (s, 3H), 3.24 (s, 3H).
Compound 1-307 (600MHz, CDCl₃): 8.18 (d, 1H), 7.84 (d, 1H), 7.67 (d, 1H), 7.26-7.29 (m, 4H), 6.57 (d, 1H), 5.06 (s, 2H), 4.05 (s, 3H), 3.47 (s, 3H), 3.24 (s, 3H), 2.35 (s, 3H).
Compound 1-308 (600MHz, CDCl₃): 8.17 (d, 1H), 7.79 (d, 1H), 7.66 (d, 1H), 7.60 (d, 1H), 7.57 (d, 1H), 7.38-7.41 (m, 1H), 7.26-7.29 (m, 1H), 6.68 (d, 1H), 5.06 (s, 2H), 4.05 (s, 3H), 3.47 (s, 3H), 3.24 (s, 3H).
Compound 1-309 (600MHz, CDCl₃): 8.17 (d, 1H), 7.79 (d, 1H), 7.67 (d, 1H), 7.38-7.46 (m, 2H), 7.30-7.36 (m, 2H), 6.68 (d, 1H), 5.06 (s, 2H), 4.05 (s, 3H), 3.47 (s, 3H), 3.24 (s, 3H).
Compound 1-310 (600MHz, CDCl₃): 8.17 (d, 1H), 7.89 (d, 1H), 7.65-7.72(m, 4H), 7.51-7.57 (m, 1H), 6.80 (d, 1H), 5.06 (s, 2H), 4.05 (s, 3H), 3.46 (s, 3H), 3.23 (s, 3H).
Compound 1-311 (600MHz, CDCl₃): 8.18 (d, 1H), 7.82 (d, 1H), 7.66 (d, 1H), 7.34-7.40 (m, 1H), 7.26-7.28 (m, 1H), 7.12-7.18 (m, 2H), 6.65-6.69 (d, 1H), 5.06 (s, 2H), 4.05 (s, 3H), 3.46 (s, 3H), 3.23 (s, 3H).
Compound 1-312 (600MHz, CDCl₃): 7.91 (d, 1H), 7.81 (d, 1H), 7.51-7.52 (m, 2H), 7.46-7.48 (m, 1H), 7.40-7.45 (m, 2H), 7.17 (d, 1H), 6.88 (d, 1H), 4.15 (t, 2H), 4.00 (s, 3H), 3.66 (t, 2H), 3.38 (s, 3H), 3.22 (s, 3H), 2.51 (s, 3H), 2.12-2.16 (m, 2H).
Compound 1-313 (600MHz, CDCl₃): 7.92 (d, 1H), 7.90 (d, 1H), 7.79-7.81 (m, 2H), 7.51-7.52 (m, 1H), 7.41-7.46 (m, 2H), 7.16 (d, 1H), 6.88 (d, 1H), 4.07 (t, 2H), 4.00 (s, 3H), 3.47 (t, 2H), 3.36 (s, 3H), 3.22 (s, 3H), 2.51 (s, 3H), 1.93-1.96 (m, 2H), 1.78-1.80 (m, 2H).
Compound 1-314 (600MHz, CDCl₃): 8.09 (d, 1H), 8.06 (d, 1H), 7.83 (d, 1H), 7.65-7.66 (m, 2H), 7.41-7.49 (m, 3H), 6.77 (d, 1H), 5.06 (s, 2H), 4.15 (s, 3H), 3.68 (t, 2H), 3.52 (t, 2H), 3.42 (q, 2H), 3.39 (s, 3H), 1.07 (t, 3H).
Compound 1-315 (600MHz, CDCl₃): 8.00 (d, 1H), 7.87 (d, 1H), 7.43-7.50 (m, 4H), 7.36-7.42 (m, 2H), 6.63 (d, 1H), 4.33-4.42 (m, 2H), 4.04 (s, 3H), 3.78-3.84 (m, 2H), 3.45 (s, 3H), 3.29 (s, 3H).
Compound 1-316 (600MHz, CDCl₃): 8.01 (d, 1H), 7.87 (d, 1H), 7.44-7.48 (m, 4H), 7.38-7.41 (m, 2H), 6.63 (d, 1H), 4.39 (t, 2H), 4.05 (s, 3H), 3.86 (t, 2H), 3.62 (q, 2H), 3.31 (s, 3H), 1.25 (t, 3H).
Compound 1-317 (600MHz, CDCl₃): 7.99 (d, 1H), 7.87 (d, 1H), 7.42-7.47 (m, 4H), 7.37-7.41 (m, 2H), 6.62 (d, 1H), 4.31 (t, 2H), 4.06 (s, 3H), 3.61 (t, 2H), 3.36 (s, 3H), 3.25 (s, 3H), 2.12-2.17 (m, 2H).
Compound 1-318 (600MHz, CDCl₃): 7.98 (d, 1H), 7.87 (d, 1H), 7.37-7.48 (m, 6H), 6.62 (d, 1H), 4.17 (t, 2H), 4.05 (s, 3H), 3.24 (s, 3H), 1.91 (q, 2H), 1.06 (t, 3H).
Compound 1-319 (600MHz, CDCl₃): 7.98 (d, 1H), 7.87 (d, 1H), 7.37-7.48 (m, 6H), 6.62 (d, 1H), 4.05 (d, 2H), 4.01 (s, 3H), 3.29 (s, 3H), 1.42 (ddd, 1H), 0.60-0.70 (m, 2H), 0.43 (dt, 2H).
Compound 2-7 (600MHz, CDCl₃): 7.86 (d, 1H), 7.27 (d, 1H), 6.76-6.78 (m, 1H), 4.11 (q, 2H), 3.98 (s, 3H), 3.24 (s, 3H), 2.44 (s, 3H), 2.09-2.13 (m, 2H), 1.99-2.06 (m, 2H), 1.49 (t, 3H), 1.43-1.47 (m, 4H).
Compound 2-9 (600MHz, CDCl₃): 7.84 (d, 1H), 7.25 (d, 1H), 6.61-6.63 (m, 1H), 4.11 (q, 2H), 3.97 (s, 3H), 3.25 (s, 3H), 2.46-2.48 (m, 2H), 2.45 (s, 3H), 2.34-2.40 (m, 2H), 1.83-1.88 (m, 2H), 1.48 (t, 3H).
Compound 2-13 (600MHz, CDCl₃): 7.90 (d, 1H), 7.37 (d, 1H), 6.76-6.77 (m, 1H), 4.48 (q, 2H), 3.98 (s, 3H), 3.26 (s, 3H), 2.49 (s, 3H), 2.12-2.16 (m, 2H), 2.05-2.09 (m, 2H), 1.47-1.55 (m, 4H).
Compound 2-15 (600MHz, CDCl₃): 7.89 (d, 1H), 7.38 (d, 1H), 6.61-6.63 (m, 1H), 4.49 (q, 2H), 3.98 (s, 3H), 3.26 (s, 3H), 2.50 (s, 3H), 2.47-2.49 (m, 2H), 2.37-2.41 (m, 2H), 1.86-1.91 (m, 2H).
Compound 2-19 (600MHz, CDCl₃): 7.88 (d, 1H), 7.29 (d, 1H), 6.78-6.80 (m, 1H), 4.24 (t, 2H), 3.99 (s, 3H), 3.81 (t, 2H), 3.48 (s, 3H), 3.29 (s, 3H), 2.50 (s, 3H), 2.01-2.16 (m, 4H), 1.33-1.53 (m, 4H).
Compound 2-21 (600MHz, CDCl₃): 7.85 (d, 1H), 7.30 (d, 1H), 6.59-6.64 (m,1H), 4.19-4.25 (m, 2H), 3.97 (s, 3H), 3.78-3.83 (m, 2H), 3.47 (s, 3H), 3.28 (s, 3H), 2.48 (s, 3H), 2.43-2.48 (m, 2H), 2.31-2.40 (m, 2H), 1.80-1.90 (m, 2H).
Compound 2-37 (600MHz, CDCl₃): 8.06 (d, 1H), 7.61 (d, 1H), 6.79-6.82 (m, 1H), 4.61 (t, 2H), 4.01 (s, 3H), 3.36 (brs, 2H), 3.19 (s, 3H), 2.44 (s, 3H), 2.13-2.15 (m, 2H), 2.02-2.04 (m, 2H), 1.48-1.54 (m, 4H).
Compound 2-43 (600MHz, CDCl₃): 7.98 (d, 1H), 7.37 (d, 1H), 6.77-6.78 (m, 1H), 3.99 (s, 3H), 3.10 (s, 3H), 2.69 (s, 3H), 2.44 (s, 3H), 2.12-2.13 (m, 2H), 2.00-2.01 (m, 2H), 1.46-1.47 (m, 4H).
Compound 2-55 (600MHz, CDCl₃): 7.99 (d, 1H), 7.43 (d, 1H), 6.74 (d, 1H), 4.07 (s, 3H), 4.03 (s, 3H), 3.25 (s, 3H), 2.10-2.15 (m, 2H), 1.96-2.00 (m, 2H), 1.47 (q, 4H).
Compound 2-61 (600MHz, CDCl₃): 7.96 (d, 1H), 7.40 (d, 1H), 6.72 (d, 1H), 4.27 (q, 2H), 4.01 (s, 3H), 3.24 (s, 3H), 2.07-2.12 (m, 2H), 1.96-2.00 (m, 2H), 1.48 (t, 3H), 1.44 (qd, 4H).
Compound 2-97 (600MHz, CDCl₃): 8.13 (d, 1H), 7.55 (d, 1H), 6.74 (s, 1H), 4.06 (s, 3H), 3.15 (s, 3H), 2.81 (s, 3H), 2.11-2.12 (m, 2H), 2.01-2.02 (m, 2H), 1.46-1.48 (m, 4H).
Compound 2-103 (600MHz, CDCl₃): 8.18 (d, 1H), 7.68 (d, 1H), 6.75 (s, 1H), 5.08 (s, 2H), 4.06 (s, 3H), 3.51 (s, 3H), 3.26 (s, 3H), 2.11-2.12 (m, 2H), 1.99-2.00 (m, 2H), 1.44-1.46 (m, 4H).
Compound 2-104 (600MHz, CDCl₃): 8.16 (d, 1H), 7.58 (d, 1H), 5.61-5.69 (m, 2H), 5.05 (s, 2H), 4.03 (s, 3H), 3.48 (s, 3H), 3.24 (s, 3H), 2.84-2.89 (m, 1H), 1.99-2.32 (m, 5H), 1.66-1.73 (m, 1H).
Compound 2-105 (600MHz, CDCl₃): 8.20 (d, 1H), 7.68 (d, 1H), 6.51-6.54 (m, 1H), 5.08 (s, 2H), 4.06 (s, 3H), 3.50 (s, 3H), 3.26 (s, 3H), 2.43-2.46 (m, 2H), 2.29-2.32 (m, 2H), 1.83-1.88 (m, 2H).
Compound 2-106 (600MHz, CDCl₃): 8.17 (d, 1H), 7.58 (d, 1H), 5.61 (s, 2H), 5.06 (s, 2H), 4.05 (s, 3H), 3.46-3.51 (m, 4H), 3.25 (s, 3H), 2.69-2.72 (m, 2H), 2.52-2.57 (m, 2H).
Compound 2-235 (600MHz, DMSO-*d*₆): 8.09 (d, 1H), 7.93 (d, 1H), 6.82-6.80 (m,1H), 4.95 (s,2H), 4.45 (s, 3H), 3.40 (s, 3H), 3.36 (s, 3H), 2.10-2.06 (m, 2H), 1.92-1.86 (m, 2H), 1.36-1.28 (m, 4H).
Compound 2-237 (600MHz, DMSO-*d*₆): 8.07 (d, 1H), 7.94 (d, 1H), 6.66-6.63 (m,1H), 4.95 (s, 2H), 4.44 (s, 3H), 3.40 (s, 3H), 3.36 (s, 3H), 2.40-2.36 (m, 2H), 2.25-2.21 (m, 2H), 1.72-1.67 (m, 2H).
Compound 2-265 (600MHz, DMSO-*d*₆): 8.38 (s, 1H), 8.32 (d, 1H), 8.27 (d, 1H), 6.72(s, 1H), 4.15 (s, 3H), 3.40 (s, 3H), 1.92-1.99 (m, 4H), 1.31-1.35 (m, 4H).
Compound 2-266 (600MHz, DMSO-*d*₆): 8.38 (s, 1H), 8.29 (d, 1H), 8.25 (d, 1H), 6.48(s, 1H), 4.16 (s, 3H), 3.38 (s, 3H), 2.33-2.35 (m, 2H), 2.13-2.21 (m, 2H), 1.69-1.74 (m, 2H).
Compound 2-267 (600MHz, CDCl₃): 8.16 (d, 1H), 7.76 (dd, 1H), 7.56 (d, 1H), 6.45 (t, 1H), 4.18 (s, 3H), 1.99-2.02 (m, 4H), 1.47-1.50 (m, 2H), 1.41-1.44 (m, 2H).
Compound 2-268 (600MHz, CDCl₃): 7.48 (d, 1H), 7.43 (d, 1H), 7.36 (dd, 1H), 6.73 (t, 1H), 3.98 (s, 3H), 2.09-2.10 (m, 2H), 2.01-2.02 (m, 2H), 1.43-1.47 (m, 4H).
Compound 2-269 (600MHz, CDCl₃): 8.02 (s, 1H), 7.97 (d, 1H), 7.77 (d, 1H), 6.73 (s, 1H), 4.05 (s, 3H), 3.12 (s, 3H), 2.12-2.13 (m, 2H), 2.03-2.04 (m, 2H), 1.49-1.50 (m, 4H).
Compound 2-270 (600MHz, CDCl₃): 8.14 (d, 1H), 7.63 (d, 1H), 6.71 (t, 1H), 5.13 (s, 2H), 4.02 (s, 3H), 3.75 (t, 2H), 3.53 (t, 2H), 3.30 (s, 3H), 3.27 (s, 3H), 2.06-2.07 (m, 2H), 1.94-1.96 (m, 2H), 1.39-1.40 (m, 4H).
Compound 2-271 (600MHz, CDCl₃): 8.27 (d, 1H), 8.24 (dd, 1H), 7.74 (d, 1H), 6.70 (t, 1H), 4.03 (s, 3H), 2.11-2.12 (m, 2H), 2.01-2.02 (m, 2H), 1.45-1.51 (m, 4H).
Compound 2-274 (600MHz, CDCl₃): 7.86 (d, 1H), 7.28 (d, 1H), 6.74-6.79 (m, 1H), 4.19-4.25 (m, 2H), 3.98 (s, 3H), 3.80-3.85 (m, 2H), 3.56-3.65 (m, 2H), 3.28 (s, 3H), 2.48 (s, 3H), 2.08-2.16 (m, 2H), 1.98-2.07 (m, 2H), 1.41-1.49 (m, 4H), 1.17-1.26 (m, 3H).
Compound 2-275 (600MHz, CDCl₃): 7.85 (d, 1H), 7.29 (d, 1H), 6.60-6.64 (m, 1H), 4.20-4.25 (m, 2H), 3.97 (s, 3H), 3.8-3.85 (m, 2H), 3.57-3.65 (m, 2H), 3.29 (s, 3H), 2.49 (s, 3H), 2.44-2.48 (m, 2H), 2.35-2.40 (m, 2H), 1.80-1.90 (m, 2H), 1.18-1.28 (m, 3H).
Compound 2-276 (600MHz, CDCl₃): 8.06 (s, 1H), 7.68-7.69 (m, 1H), 7.63-7.64 (m, 1H), 6.83 (s, 1H), 4.07 (s, 3H), 3.24 (s, 3H), 2.04-2.13 (m ,4H), 1.41-1.51 (m, 4H).
Compound 2-277 (600MHz, CDCl₃): 7.82 (d, 1H), 7.25 (d, 1H), 6.61-6.64 (m, 1H), 4.00-4.04 (m, 2H), 3.97 (s, 3H), 3.20 (s, 3H), 2.46-2.50 (m, 2H), 2.45 (s, 3H), 1.84-1.92 (m, 4H), 1.59-1.63 (m, 2H), 1.07 (t, 3H).
Compound 2-278 (600MHz, CDCl₃): 7.84 (d, 1H), 7.25 (d, 1H), 6.76-6.77 (m, 1H), 4.01-4.03 (m, 2H), 3.98 (s, 3H), 3.24 (s, 3H), 2.44 (s, 3H), 2.10-2.16 (m, 2H), 2.00-2.06 (m, 2H), 1.84-1.94 (m, 2H), 1.43-1.51 (m, 4H), 1.08 (t, 3H).
Compound 2-279 (600MHz, CDCl₃): 8.11 (d, 1H), 8.08 (d, 1H), 6.86-6.87 (m, 1H), 5.00 (s, 2H), 4.12 (s, 3H), 3.54 (t, 2H), 3.89 (s, 3H), 2.03-2.04 (m, 2H), 1.89-1.90 (m, 2H), 1.56-1.57 (m, 2H), 1.28-1.30 (m, 4H), 0.88 (t, 3H).
Compound 2-280 (600MHz, CDCl₃): 8.01 (d, 1H), 7.44 (d, 1H), 6.71-6.73 (m, 1H), 4.39 (t, 2H), 4.03 (s, 3H), 3.87 (t, 2H), 3.62 (q, 2H), 3.31 (s, 3H), 2.12-2.14(m, 2H), 2.02-2.04 (m, 2H), 1.49-1.52 (m, 4H), 1.25 (t, 3H).
Compound 2-281 (600MHz, CDCl₃): 7.99 (d, 1H), 7.42 (d, 1H), 6.73-6.75 (m, 1H), 4.33 (t, 2H), 4.03 (s, 3H), 3.61 (t, 2H), 3.37 (s, 3H), 3.26 (s, 3H), 2.15-2.19 (m, 2H), 2.13-2.15 (m, 2H), 2.02 -2.04 (m, 2H), 1.48-1.52 (m, 4H).
Compound 2-282 (600MHz, CDCl₃): 7.94 (d, 1H), 7.39 (d, 1H), 6.70 (q, 1H), 4.05 (qt, 2H), 3.99 (s, 3H), 3.22 (s, 3H), 2.04-2.11 (m, 2H), 1.96-2.00 (m, 2H), 1.81-1.93 (m, 2H), 1.44 (qd, 4H), 1.03 (tt, 3H).
Compound 2-283 (600MHz, CDCl₃): 7.97 (d, 1H), 7.40 (d, 1H), 6.72 (tt, 1H), 4.05 (d, 2H), 4.01 (s, 3H), 3.29 (s, 3H), 2.08-2.13 (m, 2H), 2.00 (t, 2H), 1.38-1.49 (m, 5H), 0.61-0.68 (m, 2H), 0.43 (dt, 2H).
Compound 3-217 (600MHz, CDCl₃): 8.09 (d, 1H), 7.91 (d, 1H), 7.77 (d, 1H), 7.43-7.50 (m, 3H), 7.35-7.42 (m, 2H), 6.51 (d, 1H), 4.07 (s, 3H).
Compound 3-218 (600MHz, CDCl₃): 8.65 (d, 1H), 7.96-8.01 (m, 1H), 7.92 (d, 1H), 7.37-7.53 (m, 6H), 6.61 (d, 1H), 3.98 (s, 3H).
Compound 3-219 (600MHz, CDCl₃): 8.23-8.27 (m, 1H), 7.92 (d, 1H), 7.84 (d, 1H), 7.49-7.53 (m, 2H), 7.37-7.47 (m, 3H), 6.76 (d, 1H), 4.03 (s, 3H), 2.56 (s, 3H).
Compound 3-220 (600MHz, CDCl₃): 8.86 (d, 1H), 8.76-8.79 (m, 1H), 8.04 (d, 1H), 7.92 (d, 1H), 7.48-7.53 (m, 2H), 7.37-7.47 (m, 4H), 6.71 (d, 1H), 4.00 (s, 3H).
Compound 4-248 (600MHz, CDCl₃): 8.10 (d, 1H), 7.79 (d, 1H), 6.62-6.65 (m, 1H), 4.06 (s, 3H), 2.10-2.15 (m, 2H), 2.02-2.08 (m, 2H), 1.58-1.70 (m, 2H), 1.49-1.52 (m, 2H).
Compound 4-249 (600MHz, CDCl₃): 8.11 (d, 1H), 7.80 (d, 1H), 6.50-6.52 (m, 1H), 4.06 (s, 3H), 2.42-2.49 (m, 2H), 2.24-2.32 (m, 2H), 1.84-1.93 (m, 2H).

### Biometric Test Examples

### Embodiment 4 Determination of herbicidal activity

Seeds of broadleaf weeds (zinnia and piemarker) or grassy weeds (green bristlegrass and barnyard grass) are respectively sown in a paper cup having a diameter of 7 cm and containing nutrient soil; after sowing, the seeds are covered with 1 cm of soil; the soil is pressed and watered, and then the seeds are cultivated in a greenhouse according to a conventional method; and stems and leaves are sprayed after 2-3 leaf stage of the weeds.

After the original medicinal acetone was dissolved, the test requires to use 1 ‰ of Tween 80 to stand in running water to prepare the solution to be tested with a required concentration. According to the design dose of the test, spray treatment was carried out on a track-type crop sprayer (designed and produced by British Engineer Research Ltd.) (spray pressure is 1.95 kg/cm², spray volume is 500 L/hm² and track speed is 1.48 km/h). The test was repeated for three times. The test material was treated and then placed in an operation hall. The medicinal liquid was naturally dried in the shade, and then was placed in a greenhouse and managed according to the conventional method. The response of the weeds to the drug was observed and recorded. After treatment, the control effects of the test drug on the weeds were visually inspected regularly, expressed by 0-100%. "0" represents no control effect and "100%" represents complete killing.

The test results show that the compounds of the formula I generally have high control effects on various weeds. Part of the test compounds, such as compounds 1-1, 1-8, 1-15, 1-17, 1-19, 1-24, 1-41, 1-53, 1-95, 1-100, 1-296, 2-7, 2-9, 2-13, 2-15, 2-19, 2-43, 2-97, 2-103, 2-104, 2-105, 2-106, 2-115, 2-265, 2-266, 2-267, 2-268, 2-269 and 2-270, have good control effects on zinnia, piemarker, green bristlegrass or barnyard grass at the application dose of 600 g a.i./hm², and the control effects are greater than or equal to 90%.

According to the above test method, part of the compounds of the formula I and KC are selected for activity test of controlling the zinnia. The results are shown in Table 5.

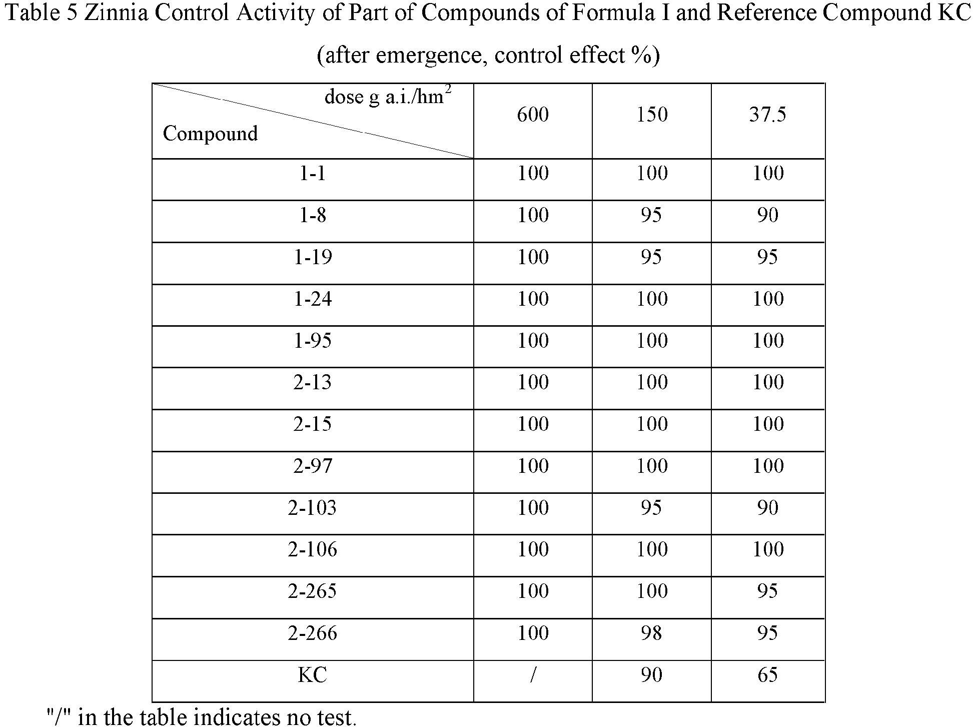

According to the above test method, part of the compounds of the formula I and KC are selected for activity test of controlling the piemarker. The results are shown in Table 6.

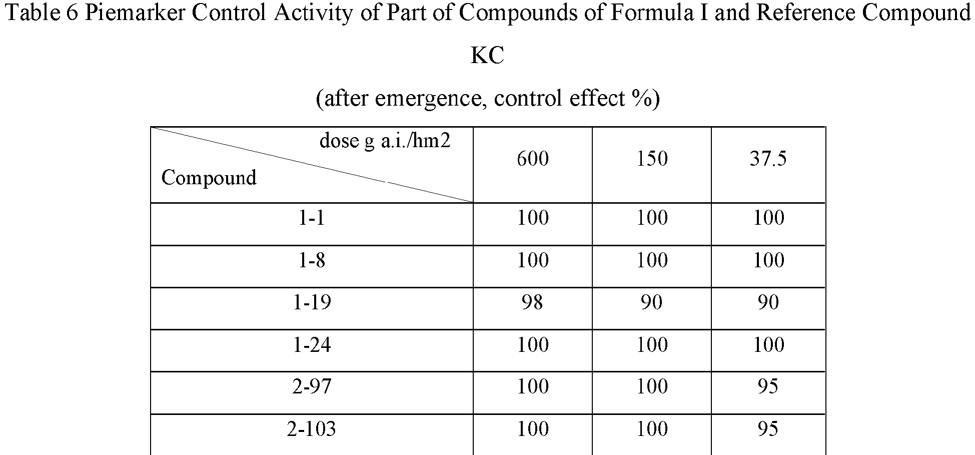

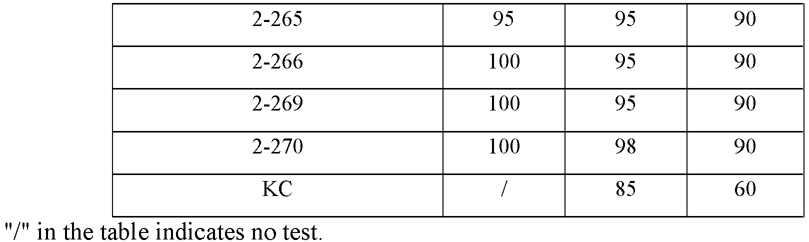

At the same time, part of compounds of the formula I are further subjected to the activity test of controlling piemarker in a smaller dose. Namely, under the dose of 18.75 g a.i./hm², the compounds have obvious effects, wherein the control effects of 1-1, 1-8, 1-19, 1-24, 2-103, 2-265 and 2-266 are greater than or equal to 70%. The control effects of 2-265 and 2-266 can reach 90%.

According to the above test method, part of the compounds of the formula I and KC are selected for the activity test of controlling the green bristlegrass. The results are shown in Table 7.

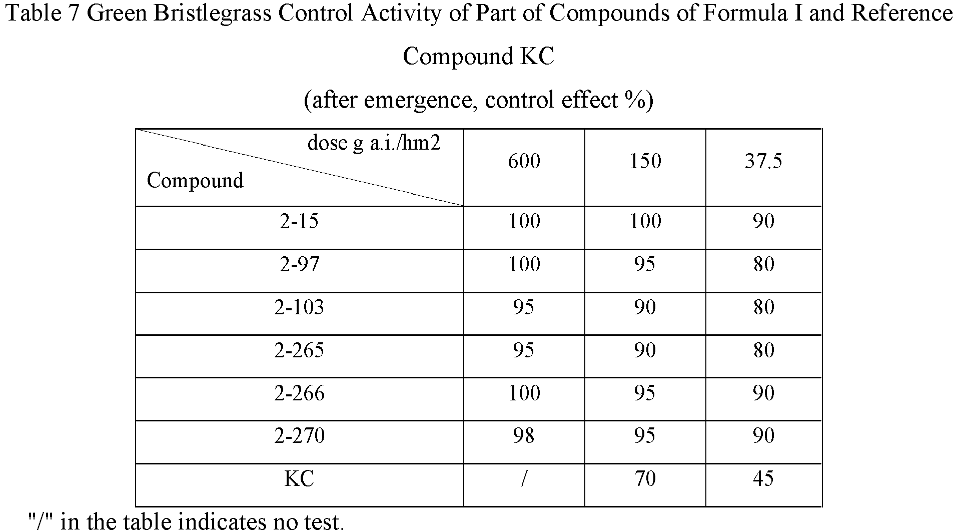

According to the above test method, part of the compounds of the formula I and KC are selected for the activity test of controlling the barnyard grass. The results are shown in Table 8.

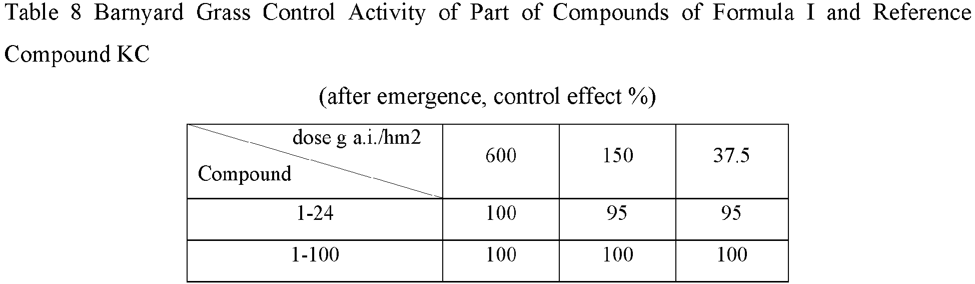

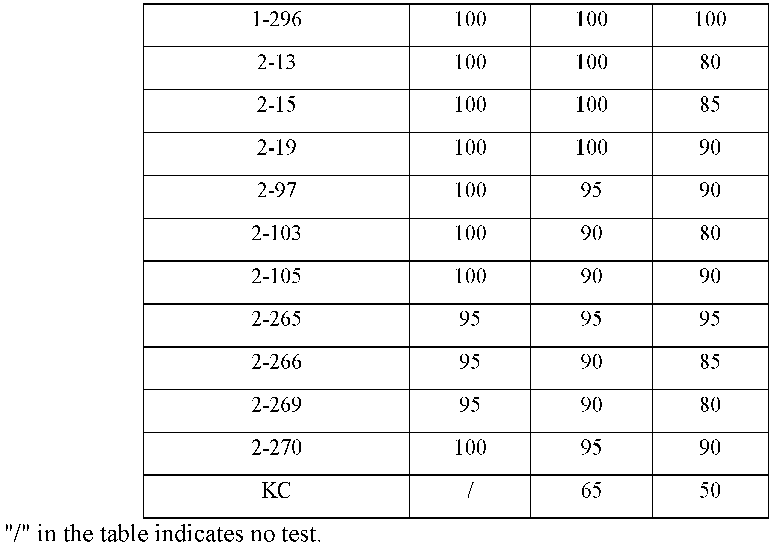

To sum up, the alkene-containing amide compound of the present invention has excellent herbicidal activity, also has high herbicidal activity at a lower dosage, and can be used for agriculturally controlling various weeds.

## Claims

1. An alkene-containing amide compound, **characterized in that** the compound is shown in formula I: 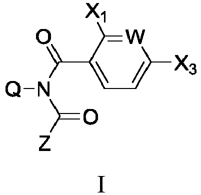 in the formula:
X₁ and X₃ are independently selected from hydrogen, cyano, nitro, halogen, C₁-C₆ alkylsulfonyl, C₁-C₆ haloalkylsulfonyl, C₁-C₆ alkylsulfinyl, C₁-C₆ haloalkylsulfinyl, C₁-C₆ alkylthio, C₁-C₆ haloalkylthio, C₁-C₆ alkyl, C₁-C₆ haloalkyl, C₁-C₆ alkoxy, C₁-C₆ haloalkoxy, C₁-C₆ alkoxy C₁-C₃ alkyl, C₁-C₆ alkoxy C₁-C₃ alkoxy, C₁-C₆ alkoxy C₁-C₃ alkoxy C₁-C₃ alkyl, C₃-C₆ cycloalkyl, C₃-C₆ cycloalkyl C₁-C₃ alkyl, C₃-C₆ cycloalkyl C₁-C₃ alkoxy, C₃-C₆ cycloalkyloxy, C₂-C₆ alkenyl, C₂-C₆ alkynyl, phenyl, C₂-C₆ alkenylsulfonyl, C₂-C₆ alkynylsulfonyl, phenylsulfonyl, C₂-C₆ alkenyloxy, C₂-C₆ alkynyloxy, phenyloxy, C₂-C₆ alkenylthio, C₂-C₆ alkynylthio, phenylthio, 5-7 membered aliphatic heterocycle containing 1-4 heteroatoms, 5-7 membered aromatic heterocycle containing 1-4 heteroatoms, 5-7 membered aliphatic heterocyclic C₁-C₆ alkyl containing 1-4 heteroatoms or 5-7 membered aromatic heterocyclic C₁-C₆ alkyl containing 1-4 heteroatoms; hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy or C₃-C₆ cycloalkyl;
W is selected from N or CX₂;
X₂ is selected from hydrogen, cyano, nitro, halogen, C₁-C₆ alkyl, C₁-C₆ haloalkyl, C₃-C₆ cycloalkyl, C₃-C₆ cycloalkyl C₁-C₆ alkyl, C₃-C₆ halocycloalkyl C₁-C₆ alkyl, Y₁ oxy, Y₁ thio, Y₁Y₂ amino, Y₁ sulfinyl, Y₁ sulfonyl, Y₁ oxy C₁-C₆ alkyl, Y₁ thio C₁-C₆ alkyl, Y₁Y₂ amino C₁-C₆ alkyl, Y₁ sulfinyl C₁-C₆ alkyl, Y₁ sulfonyl C₁-C₆ alkyl, C(O)Y₁, C(O)OY₁, OC(O)OY₁, N(Y₁)C(O)OY₂ , C(O)N(Y₁)Y₂, N(Y₁)C(O)N(Y₁)Y₂, OC(O)N(Y₁)Y₂, C(O)N(Y₁)OY₂, N(Y₁)S(O)₂Y₂, N(Y₁)C(O)Y₂, OS(O)₂Y₁, CH=NOY₁, C₁-C₆ alkyl-CH=NOY₁, C₁-C₆ alkyl-O-N=C(Y₁)Y₂, phenyl, 5-7 membered aliphatic heterocycle containing 1-4 heteroatoms, 5-7 membered aromatic heterocycle containing 1-4 heteroatoms, 5-7 membered aliphatic heterocyclic C₁-C₆ alkyl containing 1-4 heteroatoms or 5-7 membered aromatic heterocyclic C₁-C₆ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy, C₃-C₆ cycloalkyl, C₃-C₆ cycloalkoxy, C₂-C₆ alkenyl, C₂-C₆ alkynyl, phenyl or halophenyl;
Y₁ and Y₂ are independently selected from C₁-C₆ alkyl, C₁-C₆ haloalkyl, C₃-C₆ cycloalkyl, C₃-C₆ cycloalkyl C₁-C₆ alkyl, C₁-C₆ alkoxy C₁-C₆ alkyl, phenyl, 5-7 membered aliphatic heterocycle containing 1-4 heteroatoms, 5-7 membered aromatic heterocycle containing 1-4 heteroatoms, 5-7 membered aliphatic heterocyclic C₁-C₆ alkyl containing 1-4 heteroatoms or 5-7 membered aromatic heterocyclic C₁-C₆ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy, C₃-C₆ cycloalkyl, C₃-C₆ cycloalkoxy, C₂-C₆ alkenyl, C₂-C₆ alkynyl, phenyl or halophenyl;
Z is selected from Z₁ or Z₂ group; 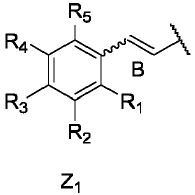
Z₂ is selected from C₃-C₈ cycloalkenyl; the hydrogen on the ring can be substituted by the following substituents; the following substituents are selected from nitro, halogen, C₁-C₆ alkyl, C₁-C₆ haloalkyl, C₁-C₆ alkoxy, C₁-C₆ haloalkoxy, C₁-C₆ alkenyl or C₃-C₆ cycloalkyl;
Q is selected from Q₁, Q₂, Q₃, Q₄, Q₅ or Q₆ group; 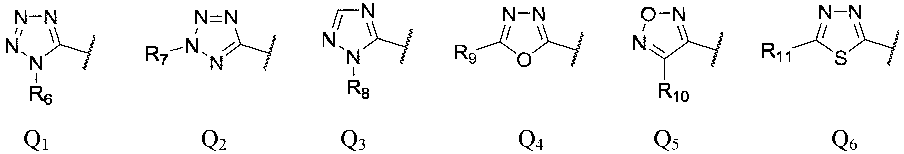
R₁ to R₅ are independently selected from hydrogen, hydroxyl, cyano, nitro, halogen, phenyl, C₁-C₆ alkyl, C₁-C₆ haloalkyl, C₂-C₆ alkenyl, C₂-C₆ haloalkenyl, C₂ -C₆ alkynyl, C₂-C₆ haloalkynyl, C₁-C₆ alkoxy, C₁-C₆ haloalkoxy, C₁-C₆ alkylthio or benzyloxy, wherein
R₁ and R₂ form a benzene ring, a 5-7 membered aliphatic heterocycle containing 1-4 heteroatoms or a 5-7 membered aromatic heterocycle containing 1-4 heteroatoms together with the carbon atoms on the connected benzene ring;
R₂ and R₃ can form a benzene ring, a 5-7 membered aliphatic heterocycle containing 1-4 heteroatoms or a 5-7 membered aromatic heterocycle containing 1-4 heteroatoms together with the carbon atoms on the connected benzene ring;
R₆, R₇ and R₈ are independently selected from hydrogen, C₁-C₆ alkyl, C₁-C₆ haloalkyl, C₃-C₆ cycloalkyl, C₃-C₆ cycloalkyl C₁-C₆ alkyl, C₂-C₆ alkenyl, C₂-C₆ alkynyl or phenyl;
R₉, R₁₀, and R₁₁ are independently selected from hydrogen, halogen, cyano, nitro, C₁-C₆ alkylsulfonyl, C₁-C₆ haloalkylsulfonyl, C₁-C₆ alkylsulfinyl, C₁-C₆ haloalkylsulfinyl, C₁-C₆ alkylthio, C₁-C₆ haloalkylthio, C₁-C₆ alkyl, C₁-C₆ haloalkyl, C₁-C₆ alkoxy, C₁-C₆ haloalkoxy, C₁-C₃ alkoxy C₁-C₃ alkyl, C₁-C₆ alkoxy C₁-C₃ alkoxy, C₁-C₆ alkoxy C₁-C₃ alkoxy C₁-C₃ alkyl, C₃-C₆ cycloalkyl, C₃-C₆ cycloalkyl C₁-C₃ alkyl, C₃-C₆ cycloalkyl C₁-C₃ alkoxy or C₃-C₆ cycloalkyloxy;
a stereoisomer of the compound of the above formula I; or, the compound of the formula I and agriculturally acceptable salt of the isomer.

2. The compound according to claim 1, **characterized in that** in the formula I:
X₁ and X₃ are independently selected from hydrogen, cyano, nitro, halogen, C₁-C₆ alkylsulfonyl, C₁-C₆ alkylsulfinyl, C₁-C₆ alkylthio, C₁-C₆ haloalkylthio, C₁-C₆ alkyl, C₁-C₆ haloalkyl, C₁-C₆ alkoxy, C₁-C₆ alkoxy C₁-C₃ alkyl, C₁-C₆ alkoxy C₁-C₃ alkoxy, C₁-C₆ alkoxy C₁-C₃ alkoxy C₁-C₃ alkyl, C₃-C₆ cycloalkyl, C₃-C₆ cycloalkyl C₁-C₃ alkyl, C₃-C₆ cycloalkyl, C₁-C₃ alkoxy, C₃-C₆ cycloalkyloxy, C₂-C₆ alkenyloxy, C₂-C₆ alkenyl, C₂-C₆ alkynyl, phenyl, phenylsulfonyl, phenyloxy, a 5-7 membered aliphatic heterocycle containing 1-4 heteroatoms and a 5-7 membered aromatic heterocycle containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy or C₃-C₆ cycloalkyl;
W is selected from N or CX₂;
X₂ is selected from hydrogen, cyano, nitro, halogen, C₁-C₆ alkyl, C₁-C₆ haloalkyl, C₃-C₆ cycloalkyl, C₃-C₆ cycloalkyl C₁-C₆ alkyl, C₃-C₆ halocycloalkyl C₁-C₆ alkyl, Y₁ oxy, Y₁ thio, Y₁Y₂ amino, Y₁ sulfinyl, Y₁ sulfonyl, Y₁ oxy C₁-C₆ alkyl, Y₁ thio C₁-C₆ alkyl, Y₁Y₂ amino C₁-C₆ alkyl, Y₁ sulfinyl C₁-C₆ alkyl, Y₁ sulfonyl C₁-C₆ alkyl, C(O)Y₁, C(O)OY₁, OC(O)OY₁, N(Y₁)C(O)OY₂ , C(O)N(Y₁)Y₂, N(Y₁)C(O)N(Y₁)Y₂, OC(O)N(Y₁)Y₂, C(O)N(Y₁)OY₂, N(Y₁)S(O)₂Y₂, N(Y₁)C(O)Y₂, OS(O)₂Y₁, CH=NOY₁, C₁-C₆ alkyl-CH=NOY₁, C₁-C₆ alkyl-O-N=C(Y₁)Y₂, phenyl, 5-7 membered aliphatic heterocycle containing 1-4 heteroatoms, 5-7 membered aromatic heterocycle containing 1-4 heteroatoms, 5-7 membered aliphatic heterocyclic C₁-C₆ alkyl containing 1-4 heteroatoms or 5-7 membered aromatic heterocyclic C₁-C₆ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy, C₃-C₆ cycloalkyl or C₃-C₆ cycloalkoxy;
Y₁ and Y₂ are independently selected from C₁-C₆ alkyl, C₁-C₆ haloalkyl, C₃-C₆ cycloalkyl, C₃-C₆ cycloalkyl C₁-C₆ alkyl, C₁-C₆ alkoxy C₁-C₆ alkyl, phenyl, a 5-7 membered aliphatic heterocycle containing 1-4 heteroatoms, a 5-7 membered aromatic heterocycle containing 1-4 heteroatoms, a 5-7 membered aliphatic heterocyclic C₁-C₆ alkyl containing 1-4 heteroatoms or a 5-7 membered aromatic heterocyclic C₁-C₆ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy, C₃-C₆ cycloalkyl or C₃-C₆ cycloalkoxy;
Z is selected from Z₁ or Z₂ group; 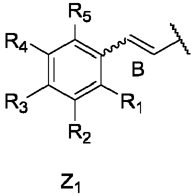
Z₂ is selected from C₃-C₈ cycloalkenyl; the hydrogen on the ring can be substituted by the following substituents; the following substituents are selected from nitro, halogen, C₁-C₆ alkyl, C₁-C₆ haloalkyl, C₁-C₆ alkoxy, C₁-C₆ haloalkoxy, C₁-C₆ alkenyl or C₃-C₆ cycloalkyl;
Q is selected from Q₁, Q₂, Q₃, Q₄ or Q₅ group; 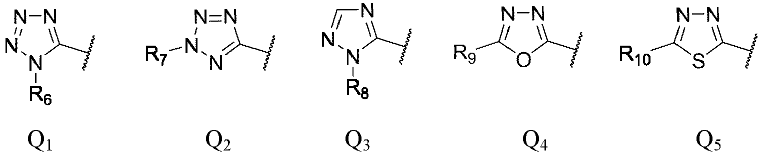
R₁ to R₅ are independently selected from hydrogen, hydroxyl, cyano, nitro, halogen, phenyl, C₁-C₆ alkyl, C₂-C₆ alkenyl, C₂-C₆ alkynyl, C₁-C₆ alkoxy, C₁-C₆ alkylthio or benzyloxy,
wherein R₁ and R₂ form a benzene ring, a 5-7 membered aliphatic heterocycle containing 1-4 heteroatoms or a 5-7 membered aromatic heterocycle containing 1-3 heteroatoms together with the carbon atoms on the connected benzene ring;
R₂ and R₃ can form a benzene ring, a 5-7 membered aliphatic heterocycle containing 1-4 heteroatoms or a 5-7 membered aromatic heterocycle containing 1-3 heteroatoms together with the carbon atoms on the connected benzene ring;
R₆, R₇ and R₈ are independently selected from hydrogen, C₁-C₆ alkyl, C₃-C₆ cycloalkyl, C₂-C₆ alkenyl, C₂-C₆ alkynyl or phenyl;
R₉ and R₁₀ are independently selected from hydrogen, halogen, C₁-C₆ alkylthio, C₁-C₆ alkyl, C₁-C₆ alkoxy, C₁-C₃ alkoxy C₁-C₃ alkyl, C₁-C₆ alkoxy C₁-C₃ alkoxy, C₁-C₆ alkoxy C₁-C₃ alkoxy C₁-C₃ alkyl or C₃-C₆ cycloalkyl;
the Z of the above formula I is selected from the stereoisomer of the compound shown by Z₁.

3. The compound according to claim 2, **characterized in that** in the formula I:
X₁ and X₃ are independently selected from hydrogen, cyano, nitro, halogen, C₁-C₃ alkylsulfonyl, C₁-C₃ alkylsulfinyl, C₁-C₃ alkylthio, C₁-C₃ haloalkylthio, C₁-C₃ alkyl, C₁-C₃ haloalkyl, C₁-C₃ alkoxy, C₁-C₃ alkoxy C₁-C₃ alkyl, C₁-C₃ alkoxy C₁-C₃ alkoxy, C₁-C₃ alkoxy C₁-C₃ alkoxy C₁-C₃ alkyl, C₃-C₆ cycloalkyl or C₃-C₆ cycloalkyloxy;
W is selected from N or CX₂;
X₂ is selected from hydrogen, cyano, nitro, halogen, C₁-C₃ alkyl, C₁-C₃ haloalkyl, C₃-C₆ cycloalkyl, C₃-C₆ cycloalkyl C₁-C₃ alkyl, C₃-C₆ halocycloalkyl C₁-C₃ alkyl, Y₁ oxy, Y₁ thio, Y₁Y₂ amino, Y₁ sulfinyl, Y₁ sulfonyl, Y₁ oxy C₁-C₃ alkyl, Y₁ thio C₁-C₃ alkyl, Y₁Y₂ amino C₁-C₃ alkyl, Y₁ sulfinyl C₁-C₃ alkyl, Y₁ sulfonyl C₁-C₃ alkyl, C(O)Y₁, C(O)OY₁, OC(O)OY₁, N(Y₁)C(O)OY₂ , C(O)N(Y₁)Y₂, N(Y₁)C(O)N(Y₁)Y₂, OC(O)N(Y₁)Y₂, C(O)N(Y₁)OY₂, N(Y₁)S(O)₂Y₂, N(Y₁)C(O)Y₂, OS(O)₂Y₁, CH=NOY₁, C₁-C₆ alkyl-CH=NOY₁, C₁-C₆ alkyl-O-N=C(Y₁)Y₂, phenyl, 5-7 membered aliphatic heterocycle containing 1-4 heteroatoms, 5-7 membered aromatic heterocycle containing 1-4 heteroatoms, 5-7 membered aliphatic heterocyclic C₁-C₃ alkyl containing 1-4 heteroatoms or 5-7 membered aromatic heterocyclic C₁-C₃ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₃ alkyl, C₁-C₃ haloalkyl, C₁-C₃ alkoxy, C₃-C₆ cycloalkyl or C₃-C₆ cycloalkoxy;
Y₁ and Y₂ are independently selected from C₁-C₆ alkyl, C₁-C₆ haloalkyl, C₃-C₆ cycloalkyl, C₃-C₆ cycloalkyl C₁-C₆ alkyl, C₁-C₆ alkoxy C₁-C₆ alkyl, phenyl, a 5-7 membered aliphatic heterocycle containing 1-4 heteroatoms, a 5-7 membered aromatic heterocycle containing 1-4 heteroatoms, a 5-7 membered aliphatic heterocyclic C₁-C₆ alkyl containing 1-4 heteroatoms or a 5-7 membered aromatic heterocyclic C₁-C₆ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy, C₃-C₆ cycloalkyl or C₃-C₆ cycloalkoxy;
Z is selected from Z₁ or Z₂ group; 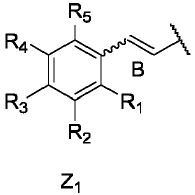
Z₂ is selected from C₅-C₆ cycloalkenyl; the hydrogen on the ring may be substituted by the following substituents which are selected from C₁-C₆ alkyl, C₁-C₆ alkoxy or C₁-C₆ alkenyl;
Q is selected from Q₁, Q₂, Q₃ or Q₄ group; 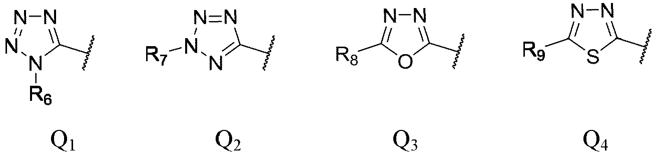
R₁ to R₅ are independently selected from hydrogen, hydroxyl, cyano, nitro, halogen, phenyl, C₁-C₆ alkyl, C₂-C₆ alkenyl, C₂-C₆ alkynyl, C₁-C₆ alkoxy or benzyloxy, wherein
R₁ and R₂ form a benzene ring or a 5-7 membered aliphatic heterocycle containing 1-4 heteroatoms together with the carbon atoms on the connected benzene ring;
R₂ and R₃ can form a benzene ring or a 5-7 membered aliphatic heterocycle containing 1-4 heteroatoms together with the carbon atoms on the connected benzene ring;
R₆ and R₇ are independently selected from hydrogen, C₁-C₆ alkyl, C₂-C₆ alkenyl or phenyl;
R₈ and R₉ are independently selected from hydrogen, halogen, C₁-C₆ alkyl, C₁-C₆ alkoxy, C₁-C₃ alkoxy C₁-C₃ alkyl, C₁-C₆ alkoxy C₁-C₃ alkoxy or C₃-C₆ cycloalkyl;
the Z of the above formula I is selected from the stereoisomer of the compound shown by Z₁.

4. The compound according to claim 3, **characterized in that** in the formula I:
X₁ and X₃ are independently selected from hydrogen, cyano, nitro, halogen, C₁-C₃ alkylsulfonyl, C₁-C₃ alkyl and C₁-C₃ haloalkyl;
W is selected from N or CX₂;
X₂ is selected from hydrogen, halogen, C₁-C₃ alkyl, C₁-C₃ haloalkyl, C₃-C₆ cycloalkyl, C₃-C₆ cycloalkyl C₁-C₃ alkyl, C₃-C₆ halocycloalkyl C₁-C₃ alkyl, Y₁ oxy, Y₁ thio, Y₁Y₂ amino, Y₁ sulfonyl, Y₁ oxy C₁-C₃ alkyl, Y₁ thio C₁-C₃ alkyl, Y₁Y₂ amino C₁-C₃ alkyl, Y₁ sulfonyl C₁-C₃ alkyl, C(O)Y₁, C(O)OY₁, OC(O)OY₁, N(Y₁)C(O)OY₂ , C(O)N(Y₁)Y₂, N(Y₁)C(O)N(Y₁)Y₂, OC(O)N(Y₁)Y₂, C(O)N(Y₁)OY₂, N(Y₁)S(O)₂Y₂, N(Y₁)C(O)Y₂, OS(O)₂Y₁, CH=NOY₁, C₁-C₆ alkyl-CH=NOY₁, C₁-C₆ alkyl-O-N=C(Y₁)Y₂, phenyl, 5-7 membered aliphatic heterocycle containing 1-4 heteroatoms, 5-7 membered aromatic heterocycle containing 1-4 heteroatoms, 5-7 membered aliphatic heterocyclic C₁-C₃ alkyl containing 1-4 heteroatoms or 5-7 membered aromatic heterocyclic C₁-C₃ alkyl containing 1-4 heteroatoms; the hydrogen on the phenyl, the aliphatic heterocycleand the aromatic heterocycle mentioned above may be substituted by one or more of the following substituents which are selected from nitro, halogen, C₁-C₃ alkyl, C₁-C₃ haloalkyl, C₁-C₃ alkoxy, C₃-C₆ cycloalkyl or C₃-C₆ cycloalkoxy;
Y₁ and Y₂ are independently selected from C₁-C₆ alkyl, C₁-C₆ haloalkyl, C₃-C₆ cycloalkyl, C₃-C₆ cycloalkyl C₁-C₆ alkyl, C₁-C₆ alkoxy C₁-C₆ alkyl, phenyl, a 5-7 membered aliphatic heterocycle containing 1-4 heteroatoms, a 5-7 membered aromatic heterocycle containing 1-4 heteroatoms, a 5-7 membered aliphatic heterocyclic C₁-C₆ alkyl containing 1-4 heteroatoms or a 5-7 membered aromatic heterocyclic C₁-C₆ alkyl containing 1-4 heteroatoms; the hydrogen on the phenyl, the aliphatic heterocycle and the aromatic heterocycle mentioned above may be substituted by one or more of the following substituents which are selected from nitro, halogen, C₁-C₆ alkyl, C₁-C₆ haloalkyl, C₁-C₆ alkoxy, C₁-C₆ haloalkoxy, C₃-C₆ cycloalkyl or C₃-C₆ cycloalkoxy;
Z is selected from Z₁ or Z₂ group; 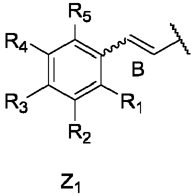
Z₂ is selected from G₁, G₂, G₃, G₄, G₅ or G₆ group; 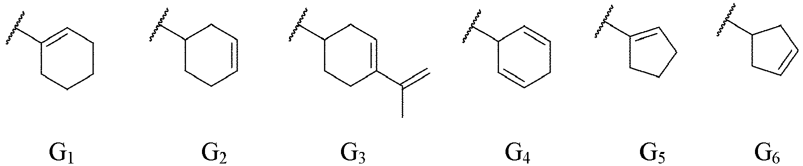
Q is selected from Q₁, Q₂, Q₃ or Q₄ group; 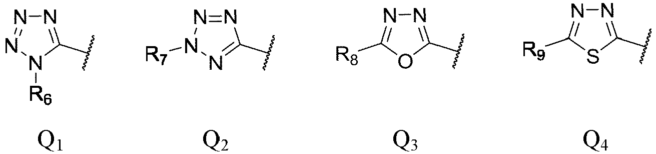
R₁ to R₅ are independently selected from hydrogen, hydroxyl, cyano, nitro, halogen, phenyl, methyl, ethyl, propyl, vinyl, propenyl, ethynyl, propynyl, methoxy, ethoxyl, benzyloxy, trifluoromethyl or trifluoromethoxy;
R₁ and R₂ can form a benzene ring together with the carbon atoms on the connected benzene ring;
R₂ and R₃ can form a benzene ring, 1,3-dioxane ring or 1,4-dioxane ring together with the carbon atoms on the connected benzene ring;
R₆ and R₇ are independently selected from hydrogen, methyl or ethyl;
R₈ and R₉ are independently selected from hydrogen, chlorine or methyl;
the Z of the above formula I is selected from a trans-stereoisomer of the compound shown by Z₁.

5. An application of the compound of the formula I of claim 1, **characterized in** the application of the compound of the formula I and the stereoisomer thereof or the compound of the formula I and the agriculturally acceptable salt of the isomer in control for weeds.

6. A herbicidal composition, **characterized in that** the herbicidal composition comprises an active ingredient and an agriculturally acceptable carrier; the active ingredient comprises the compound of the formula I and the stereoisomer thereof; or, the compound of the formula I and agriculturally acceptable salt of the isomer; the weight percentage of the active ingredient in the composition is 1-99%.

7. A method for controlling weeds by a herbicidal composition of claim 6, **characterized in that** a herbicidally effective dose of the herbicidal composition of claim 5 is applied to a weed or a growth medium or site of the weed.
